# EUROPÄISCHE PATENTSCHRIFT

(11) **EP 0 433 855 B1**
(45) Veröffentlichungstag und Bekanntmachung des Hinweises auf die Patenterteilung: **07.03.2001**
(21) Anmeldenummer: 90123836.0
(22) Anmeldetag: 11.12.1990
(51) Int. Cl.: C07H 15/26, C12Q 1/26, C12Q 1/40, C12Q 1/54, C07F 9/6561, C07D 471/04, C07D 487/04, C07D 513/04, C12Q 1/34

(54) **N- und O-substituierte Aminophenolderivate, Zwischenprodukte zu deren Herstellung, deren Verwendung als Hydrolasesubstrate, ein entsprechendes Bestimmungsverfahren und hierfür geeignetes diagnostisches Mittel**
N- and O-substituted aminophenol derivatives, intermediates for their preparation, their use as hydrolase substrates, a corresponding assay and diagnostic agents
Ammophenols O- et N-substitués, intermédaires pour leur préparation, leur utilisation comme substrats pour hydrolases, procédé d'analyse correspondant et agents diagnostiques

(30) Priorität: 21.12.1989 DE 3942355
(43) Veröffentlichungstag der Anmeldung: 26.06.1991
(73) Patentinhaber: Roche Diagnostics GmbH, 68298 Mannheim (DE)
(72) Erfinder: Mangold, Dieter, Dipl.-Chem. Dr. rer. nat., W-6701 Maxdorf (DE); Zimmermann, Gerd, Dr. rer. nat., W-6800 Mannheim 41 (DE)

(56) Entgegenhaltungen:
- EP-A- 0 034 323
- EP-A- 0 268 167
- EP-A- 0 433 853
- WO-A-89/02473
- FR-A- 1 448 348
- US-A- 3 875 013

## Beschreibung

Die Erfindung betrifft neue N- und O-substituierte Aminophenolderivate und ein Verfahren zur Herstellung der neuen Substanzen.

Außerdem betrifft die Erfindung Zwischenprodukte zur Herstellung der erfindungsgemäßen N- und O-substituierten Aminophenolderivate sowie Verfahren zur Herstellung der Zwischenprodukte.

Weiter betrifft die Erfindung die Verwendung von N- und O-substituierten Aminophenolderivaten als Hydrolasesubstrate.

Im übrigen betrifft die Erfindung ein Verfahren zur kolorimetrischen Bestimmung einer Hydrolase durch Umsetzung eines chromogenen Enzymsubstrats mit dem Enzym, Oxidation eines durch das Enzym aus dem Substrat freigesetzten Leukofarbstoffs und Bestimmung des resultierenden Farbstoffs als Maß für die Menge an Enzym.

Die Erfindung betrifft auch ein diagnostisches Mittel zur Bestimmung einer Hydrolase enthaltend ein chromogenes Enzymsubstrat und eine geeignete Puffersubstanz.

Schließlich betrifft die Erfindung die Verwendung eines N- und O-substituierten Aminophenolderivats zur Herstellung eines diagnostischen Mittels zur Bestimmung einer Hydrolase.

Unter Hydrolasen werden allgemein Enzyme verstanden, welche Bindungen unter Wasserverbrauch, hydrolytisch, spalten. In der klinischen Chemie und in der Diagnostik hat in den letzten Jahren vor allem die Bestimmung der Aktivität solcher Hydrolasen an Bedeutung gewonnen, welche glycosidische und Etherbindungen spalten. Zu nennen sind hier außerdem beispielhaft Esterasen, wie z. B. die in Leukozyten vorkommenden Carboxylester spaltenden Enzyme oder Phosphatasen, wie z. B. alkalische oder saure Phosphatasen, die Phosphorsäureester hydrolysieren. Für die Diagnostik von Erkrankungen der Niere und des Urogenitaltraktes hat es sich als nützlich erwiesen, Leukozyten anhand der ihnen innewohnenden esterolytischen Aktivität in Urin nachzuweisen. Hierfür geeignete Aminosäure- und Peptidester von Leuko-Indoanilinen sind beispielsweise aus EP-A 0 034 323 bekannt.Die Aktivitätsbestimmung der sauren Phosphatase ist ein wertvolles Mittel zur Frühdiagnose von Prostata-Karzinomen. Alkalische Phosphatase kann als Markerenzym für Enzymimmunoassays eingesetzt werden.

EP-A 0 268 167 beschreibt ein analytisches Element für die Bestimmung von Wasserstoffpercxid enthaltend ein aromatisches primäres Amin, das nach Oxidation mit einer heterocyclischen Verbindung zu einem Farbstoff reagiert. Unter der Vielzahl an genannten Verbindungen sind keine Aminophenolderivate aufgeführt.

Glykosidasen, wie z. B. Galactosidasen, Glucosidasen, Mannosidasen, Amylase oder N-Acetyl-β-D-Glucosaminidase spalten glykosidische Bindungen. Sie erfüllen im menschlichen und tierischen Organismus eine vielfache physiologische Funktion. So spielt beispielsweise die β-D-Galactosidase eine gewichtige Rolle im Kohlenhydratstoffwechsel, da durch sie die Hydrolyse der Lactose erfolgt. Darüber hinaus stellt die β-D-Galactosidase das Schlüsselenzym beim Abbau von Glycolipiden, Mucopolysacchariden und Glycoproteinen dar. Als weitere physiologisch bedeutsame Glycosidasen sind zu nennen: die α-D-Galactosidase, die α-D- und β-D-Glucosidase sowie die α-D-Mannosidase.

Über ihren physiologischen Stellenwert hinaus haben die Glycosidasen in den letzten Jahren im diagnostischen sowie im biotechnologischen Bereich an Bedeutung gewonnen. So werden beispielsweise diese Enzyme in zunehmendem Maße als Indikator-Enzym für Enzymimmunoassays eingesetzt. Besonders bevorzugt wird in diesem Zusammenhang die β-D-Galactosidase.

Das Vorhandensein des Enzyms N-Acetyl-β-D-glucosaminidase (β-NAGase) in Körperflüssigkeiten ist ein wertvoller Indikator für Krankheiten oder Fehlfunktionen im Organismus. Im Harn sind z. B. erhöhte Werte bei Nierenübertragungen ein Zeichen für die Abstoßung der Spenderniere. Erhöhte Werte treten ebenso auf bei einer Vielzahl von Krankheitsbildern und von toxischen Schädigungen der Niere. Im Speichel von Frauen ist die NAGase-Aktivität ein Indikator für Fruchtbarkeit und Schwangerschaft.

Zur Bestimmung der Aktivität von Hydrolasen wird die enzymhaltige Probe mit einem geeigneten Substrat versetzt. Das Substrat wird von dem Enzym gespalten und eines der Spaltprodukte wird in geeigneter Weise nachgewiesen. Als Substrat eignen sich oft die natürlichen Substrate der nachzuweisenden Enzyme. Besonders bevorzugt werden jedoch chromogene Verbindungen verwendet, bei denen eines der Spaltprodukte einen Rest darstellt, der spektroskopisch im sichtbaren oder im UV-Bereich nachgewiesen werden kann.

Als chromogene Substrate zur Bestimmung von Ester- und glycosidischen Bindungen spaltenden Enzymen werden in EP-A-0 274 700 Dihydroresorufin-Verbindungen vorgestellt. Nach enzymatischer Spaltung der Substrate entstehen gut wasserlösliche Leukofarbstoffe, die durch Oxidationsmittel leicht zu Farbstoffen oxidiert werden können. Disubstituierte Dihydroresorufinderivate werden in einer mehrstufigen Reaktionsfolge hydrolysiert, was zu einer Komplikation bei kinetischen Messungen führen kann.

Zur Bestimmung Glycosidbindungen spaltender Enzyme sind eine Vielzahl chromogener Substrate bekannt.

So sind in Biochem. Z. 333, 209 (1960) Phenyl-β-D-galactosid sowie einige weitere am aromatischen Ring substituierte Derivate (z. B. o-Nitrophenyl- und p Nitrophenyl-β-D-galactosid) als Substrate der β-D-Galactosidase beschrieben. Die durch Hydrolyse freigesetzten Phenole werden photometrisch im UV-Bereich bzw. bei den Nitrophenolen im kurzwelligen sichtbaren Wellenlängenbereich bestimmt. Auch kann als Indikatorreaktion eine oxidative Kupplung mit Aminoantipyrin angeschlossen werden [Analytical Biochem. 40, 281 (1971)].

In WO-A 89 02473 werden Enzymsubstrate beschrieben, die die Nachteile von Nitrovinylphenylderivaten vermeiden sollen. Hierbei ist die chromogene Gruppe ein Heterocyclus, der entweder direkt oder über eine oder mehrere Gruppen ( CR=CR') an einen Arylrest gebunden ist. Pyrazoloheterocyclusreste sind nicht beschrieben.

Für histochemische Untersuchungen werden Naphthyl-β-D-galactoside verwendet, so z. B. die 1-Naphthyl-Verbindung in Histochemie 35, 199 (1973), das 6-Brom-2-naphthyl-Derivat in J. Biol. Chem. 195, 239 (1952) oder das Naphthol-β-D-galactosid (Histochemie 37, 89 (1973)). Zur Visualisierung werden dabei die entstehenden Naphthole mit verschiedenen Diazoniumsalzen zu Azo-Farbstoffen umgesetzt. Bei solchen notwendigen Kupplungsreaktionen können Probenbestandteile beispielsweise stören, wenn sie statt der Kupplungskomponente mit den durch die enzymatische Spaltung freigesetzten Substanzen reagieren.

Zum Nachweis des Enzyms N-Acetyl-β-D-glucosaminidase (NAGase) wird in US-A-3,968,011 vorgeschlagen, solche Phenolderivate einzusetzen, die bei dem für die Enzymreaktion notwendigen pH-Wert gespalten werden und deren Reaktionsprodukt bei basischem pH-Wert eine Farbe bildet, die nachgewiesen bzw. gemessen werden kann. Ein Umpufferungsschritt ist deshalb notwendig.

Ebenso werden in EP-A-0 097 506 NAGase-Substrate vorgeschlagen, die bei saurem pH in Anwesenheit des Enzyms ein Chromogen, wie p-Nitrophenol freisetzen, das nach Umpufferung auf basischen pH eine Farbe bildet, die gemessen werden kann. Bei Freisetzung von Umbelliferon kann dieses fluorometrisch detektiert werden, was jedoch sehr apparateaufwendig ist und insbesondere bei biologischen Proben auch störanfällig ist, wegen des darin enthaltenen Fluoreszenzhintergrundes.

In EP-A-0 060 793 werden Sulfophthaleinyl-N-acetyl-β-D-glucosaminide als Substrate für NAGase beschrieben. Nachteilig ist jedoch die Notwendigkeit der Messung eines Leerwertes und der zusätzliche Handlunggsschritt, der benötigt wird, um den Reaktionsansatz umzupuffern.

EP-A-0 180 961 offenbart in 2- und 4-Position durch Halogen oder Nitro substituierte N-Acetyl-β-D-glucosaminide als β-NAGase-Substrate. Das Verfahren zur Bestimmung von NAGase mittels dieser Substrate kommt zwar ohne zusätzlichen Handlungsschritt und ohne Leerwertmessung aus, die Löslichkeit des Substrats ist aber auch in Gegenwart von Netzmitteln sehr gering. Dies hat bei Messung von β-NAGase in Harn Störungen durch Harninhaltsstoffe zur Folge und der Zeitbedarf ist bei niedrigen Enzymkonzentrationen für die Testdurchführung groß.

Natrium-3,3'-dichlorophenolsulfonphthaleinyl-N-acetyl-β-D-glucosaminid, ist in EP-A 0 294 804 beschrieben. Auch hier kann direkt und ohne Abstoppen gemessen werden, die Nachweisreaktion muß aber bei pH 6,25 durchgeführt werden, da nur ab dort, nicht aber bei saurerem pH-Wert, das durch enzymatische Spaltung freigesetzte Chromogen hinreichend farbig ist. Da das Enzym in der Reaktionsrate bei pH 4,5-5 ein Maximum zeigt, führt dies zu einer verlangsamten enzymatischen Substratspaltung. Für alle bisher beschriebenen Analysenverfahren zur Bestimmung von NAGase benötigt man zudem spektrophotometrische Geräte.

Substrate, die auch eine visuelle Beurteilung gestatten, entweder direkt oder nach Alkalibehandlung, sind in DE-C 28 57 145 beschrieben. Die gekuppelten Farbstoffe haben die allgemeine Formel HX-A-Y-NO₂, wobei A für einen aromatischen Kern steht, HX für eine auxochrome Gruppe und Y für eine ungesättigte Gruppe. Diese Substrate setzen Verbindungen frei, die durch Rot- oder Blaufärbung das Enzym anzeigen. Das Verfahren hat allerdings den Nachteil, daß in Gegenwart des Enzyms nur eine Farbveränderung auftritt, da die Substrate selbst gefärbt sind. Die Substrate müssen bei einer nicht vorbehandelten oder verdünnten Probe wie z. B. Harn in hohen Konzentrationen vorgelegt werden, da andernfalls Harninhaltsstoffe die enzymatische Spaltung drastisch verlangsamen. Selbst schwach eigengefärbte Substrate ergeben dann bei den nötigen Konzentrationen stark gefärbte Teststreifen. Eine Detektion über eine Farbveränderung macht geringe Enzym-Konzentrationen nicht oder nur nach sehr langer Zeit nachweisbar.

In der japanischen Patentanmeldung JP-A-01 068389 werden N- und 0-substituierte Aminophenylderivate als NAGase-Substrate vorgeschlagen, die als N-Substituenten einen Phenol- oder Naphtholrest mit freier OH-Gruppe oder eine Chinoniminstruktur tragen. Als Bedingung wird angegeben, daß einer der beiden im Molekül vorliegenden N-Substituenten ausschließlich Elektronen anziehende Reste tragen kann, während der andere durch Elektronendonatoren substituiert ist. Diese Substanzen sind sehr schwer wasserlöslich. Hierdurch sind sie sehr störanfällig gegenüber Probenbestandteilen. Beispielsweise werden die beschriebenen Substanzen in Harn nicht durch NAGase gespalten.

Zusammenfassend stellen sich die Nachteile der aus dem Stand der Technik bekannten Hydrolase-Substrate so dar. Substrate, die eine mehrfache enzymatische Spaltung erlauben, komplizieren kinetische Messungen. Eine Lichtabsorption des durch das zu bestimmende Enzym abgespaltenen Restes im kurzwelligen sichtbaren Wellenlängenbereich (z. B. gelb gefärbte Reste) ist in vielen Proben, insbesondere solchen aus Körperflüssigkeiten, wo viele Substanzen enthalten sind, die ebenfalls im kurzwelligen Wellenlängenbereich absorbieren, nicht störungsfrei bestimmbar. Die Sichtbarmachung eines enzymatisch abgespaltenen Restes durch Kupplung mit einer weiteren Substanz kann ebenfalls durch Probenbestandteile gestört werden. Fluorometrische Bestimmungen sind apparateaufwendig und insbesondere bei biologischen Proben oft durch eine Grundfluoreszenz des Probenmaterials gestört. Mit Hydrolase-Substraten, die bei enzymatischer Spaltung lediglich ihre Farbe wechseln oder einen Farbübergang durchmachen sind Bestimmungen von niedrig konzentrierten Enzymen oft nur mit hohen Substratkonzentrationen möglich, zu deren Umsatz viel Zeit benötigt wird und die unter Umständen das zu bestimmende Enzym hemmen können. Schwerlöslichkeit des Hydrolasesubstrates führt oft zu einer geringen Empfindlichkeit der Bestimmungsmethode und erhöht den Zeitbedarf bis zu dem das gewünschte Ergebnis vorliegt. Ebenfalls benötigen Bestimmungsverfahren mit Hydrolase-Substraten, die zusätzliche Schritte, wie Leerwertmessung oder Umpufferung der Reaktionsmischung erforderlich machen, relativ viel Zeit und sind darüber hinaus in ihrer Durchführung umständlich und teilweise so kompliziert, daß nur Fachleute, aber keine Laien sie zufriedenstellend und reproduzierbar durchführen können. Bestimmungsmethoden, die zusätzliche Handlungsschritte erfordern, sind außerdem nicht oder nur sehr schwer auf Testträger, d. h. sogenannte trockenchemische Teste zu übertragen und mit solchen durchzuführen. Darüber hinaus erlauben Bestimmungsverfahren, die z. B. einen Umpufferungsschritt erfordern, keine kinetischen Bestimmungen, sondern sind reine Endpunktmethoden.

Hier will die Erfindung Abhilfe schaffen. Die Erfindung wie sie in den Ansprüchen gekennzeichnet ist, löst die Aufgabe, Hydrolase-Substrate zur Verfügung zu stellen, die Hydrolasen schnell und einfach bestimmbar machen, die sowohl kinetische Messungen als auch Endpunktbestimmungen erlauben, die auch geringe Enzymkonzentrationen empfindlich bestimmen lassen, zu einer möglichst störungsarmen Hydrolase-Bestimmung dienen können, keine aufwendigen Apparaturen notwendig machen und die auch auf Testträgern einsetzbar sind.

Gegenstand der Erfindung ist die Verwendung von N- und O-substituierten Aminophenolderivaten der allgemeinen Formel I 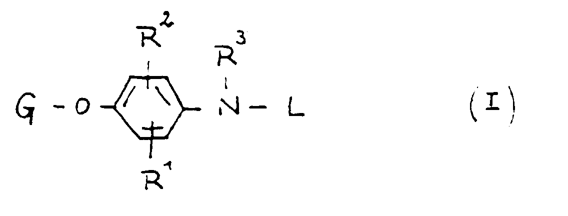 in der
- G: einen organischen oder anorganischen Säurerest oder einen Glycosidrest darstellt,
- R¹ und R²,: die gleich oder verschieden sind, Wasserstoff,
Halogen, SO₃H, PO₃H₂ oder ein Salz dieser Säuregruppe, eine Hydroxy-, Nitro-, Carboxy-, Carboxamido- oder Cyanogruppe oder eine gegebenenfalls durch einen oder mehrere Hydroxy-, Carboxy, -Halogen-, Cyano-, SO₃H- oder PO₃H₂-Reste oder ein Salz eines dieser Säurereste substituierte Alkyl-, Alkenyl-, Alkoxy-, Alkylsulfinyl-, Alkylsulfonyl-, Alkoxycarbonyl-, Alkylcarbonyl-, Aryl-oder Aralkylgruppe bedeuten oder
wenn sich beide Reste an benachbarten Kohlenstoffatomen befinden gemeinsam eine 1,4-Butadiendiylgruppe darstellen, die gegebenenfalls ein- oder mehrmals durch SO₃H, PO₃H₂ oder ein Salz dieser Säuregruppen, eine Alkyl- oder/und eine Carboxygruppe substituiert ist,
- R³: Wasserstoff, CO-COOH, SO₃H, PO₃H₂ oder ein Salz dieser Säuregruppen, eine gegebenenfalls ein- oder mehrfach durch Halogen, CO₂H, SO₃H oder/und PO₃H₂oder ein Salz dieser Säuregruppen substituierte Alkylcarbonylgruppe oder eine gegebenenfalls ein- oder mehrfach durch SO₃H, PO₃H₂ oder ein Salz dieser Säurereste substituierte Arylcarbonylgruppe ist und
- L: einen Pyrazoloheterocyclus-Rest der allgemeinen Formel III 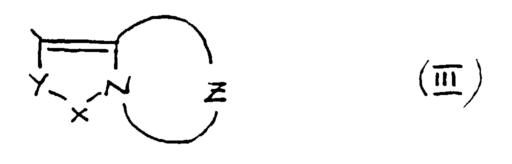
darstellt,
in der
X-Y NR⁸-CO oder N=CR⁹ bedeutet,
wobei
- R⁸: Wasserstoff oder Alkyl und
- R⁹: Alkyl, Alkenyl, Alkoxy, Alkylthio, Aryl, Aralkyl, gegebenenfalls jeweils substituiert durch Hydroxy, Dialkylphosphinyl, Carboxy, SO₃H, PO₃H₂, ein Salz eines dieser Säurereste oder/und Alkoxycarbonyl;
Amino, das gegebenenfalls durch ein oder zwei gegebenenfalls einen oder mehrere Hydroxy-, Carboxy- oder/und Alkoxycarbonylreste tragenden Alkylrest substituiert ist wobei, wenn Amino durch 2 Alkylreste substituiert ist, diese Reste auch zu einem Ring geschlossen sein können, der außer durch das N-Atom der Aminogruppe gegebenenfalls auch durch Sauerstoff, Schwefel oder ein weiteres Stickstoffatom unterbrochen sein kann oder Amino gegebenenfalls durch ein oder zwei Acylgruppen, Alkoxy- oder/und Aralkoxycarbonylgruppen, H₂N-CO, Alkyl-, Aralkyl- oder/und Arylcarbamoylgruppen substituiert ist; oder
Wasserstoff, Carboxy, Alkoxycarbonyl, Carboxamido oder Halogen ist, und
Z NR¹⁰-N=N bedeutet
wobei R¹⁰ Wasserstoff, Alkyl oder Aralkyl ist oder
Z eine ungesättigte Kette mit 3 bis 5 Gliedern aus Stickstoffatomen oder aus Kohlenstoffatomen und gegebenenfalls einem oder mehreren Stickstoff- oder Schwefelatomen darstellt, wobei Kohlenstoffatome gegebenenfalls durch Alkyl, Alkoxy, Hydroxyalkyl, Alkylthio, Hydroxy, Aralkyl, Aryl, Carboxy, Carboxamido, Alkoxycarbonyl, Cyano, Amino, das gegebenenfalls durch ein oder zwei gegebenenfalls einen oder mehrere Hydroxy-, Carboxy- oder/und Alkoxycarbonylreste tragenden Alkylrest substituiert ist, oder/und Halogen sowie Stickstoffatome, die nicht über eine Doppelbindung gebunden sind, gegebenenfalls durch Alkyl oder Aralkyl substituiert sind oder zwei benachbarte Kettensubstituenten gegebenenfalls eine Alkylengruppe bilden, die ihrerseits gegebenenfalls mit Aryl substituiert oder anelliert ist oder ein entsprechender tautomerer Rest als Hydrolase-Substrat.

Aus GB-A-2 061 537 und DE-A-22 60 202 sind Pyrazolotriazolderivate als Farbstoffbildner für die Erzeugung photographischer Bilder bekannt, die den Verbindungen der allgemeinen Formel I entsprechen, wenn dort G einen Alkancarbonsäurerest, R³ Wasserstoff, eine Arylcarbonylgruppe oder eine gegebenenfalls ein- oder mehrfach durch Halogen substituierte Alkylcarbonylgruppe und L einen Pyrazoloheterocyclus-Rest der allgemeinen Formel III mit X-Y in der Bedeutung N=CR⁹, wobei R⁹ die zuvor angegebene Bedeutung hat und zusätzlich Z einen 1,2,4-Triazolring darstellt, bei dem ein nicht über eine Doppelbindung gebundener Stickstoff mit Wasserstoff substituiert ist.

Die übrigen Verbindungen der allgemeinen Formel I sind neu.

Gegenstand der Erfindung sind deshalb auch N- und O-substituierte Aminophenolderivate der allgemeinen Formel I 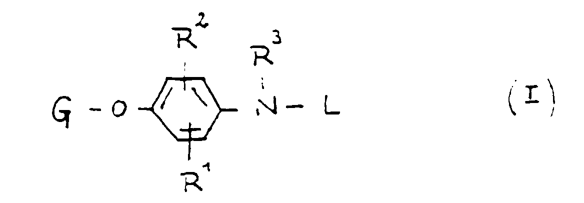 in der
- G: einen organischen oder anorganischen Säurerest oder einen Glycosidrest darstellt,
- R¹ und R²,: die gleich oder verschieden sind, Wasserstoff, Halogen, SO₃H, PO₃H₂ oder ein Salz dieser Säuregruppe, eine Hydroxy-, Nitro-, Carboxy-, Carboxamido- oder Cyanogruppe oder eine gegebenenfalls durch einen oder mehrere Hydroxy-, Carboxy,-Halogen-, Cyano-, SO₃H- oder PO₃H₂-Reste oder ein Salz eines dieser Säurereste substituierte Alkyl-, Alkenyl-, Alkoxy-, Alkylsulfinyl-, Alkylsulfonyl-, Alkoxycarbonyl-, Alkylcarbonyl-, Aryl- oder Aralkylgruppe bedeuten oder wenn sich beide Reste an benachbarten Kohlenstoffatomen befinden gemeinsam eine 1,4-Butadiendiylgruppe darstellen, die gegebenenfalls ein- oder mehrmals durch SO₃H, PO₃H₂ oder ein Salz dieser Säuregruppen, eine Alkyl- oder/und eine Carboxygruppe substituiert ist,
- R³: Wasserstoff, CO-COOH, SO₃H, PO₃H₂ oder ein Salz dieser Säuregruppen, eine gegebenenfalls ein- oder mehrfach durch Halogen, CO₂H, SO₃H oder/und PO₃H₂ oder ein Salz dieser Säuregruppen substituierte Alkylcarbonylgruppe oder eine gegebenenfalls ein- oder mehrfach durch SO₃H, PO₃H₂ oder ein Salz dieser Säurereste substituierte Arylcarbonylgruppe ist und
- L: einen Pyrazoloheterocyclus-Rest der allgemeinen Formel III 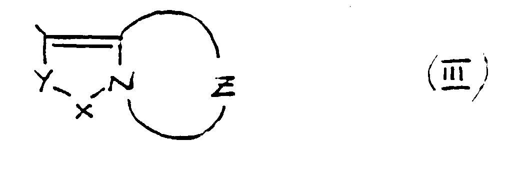 darstellt,
in der
X-Y NR⁸-CO oder N=CR⁹ bedeutet,
wobei
R⁸ Wasserstoff oder Alkyl und
R⁹ Alkyl, Alkenyl, Alkoxy, Alkylthio, Aryl, Aralkyl, gegebenenfalls jeweils substituiert durch Hydroxy, Dialkylphosphinyl, Carboxy, SO₃H, PO₃H₂, ein Salz
eines dieser Säurereste oder/und Alkoxycarbonyl;
Amino, das gegebenenfalls durch ein oder zwei gegebenenfalls einen oder mehrere Hydroxy-, Carboxy- oder/und Alkoxycarbonylreste tragenden Alkylrest substituiert ist wobei, wenn Amino durch 2 Alkylreste substituiert ist, diese Reste auch zu einem Ring geschlossen sein können, der außer durch das N-Atom der Aminogruppe gegebenenfalls auch durch Sauerstoff, Schwefel oder ein weiteres Stickstoffatom unterbrochen sein kann oder Amino gegebenenfalls durch ein oder zwei Acylgruppen, Alkoxy- oder/und Aralkoxycarbonylgruppen, H₂N-CO, Alkyl-, Aralkyl- oder/und Arylcarbamoylgruppen substituiert ist; oder
Wasserstoff, Carboxy, Alkoxycarbonyl, Carboxamido oder Halogen ist, und
Z NR¹⁰-N=N bedeutet
wobei R¹⁰ Wasserstoff, Alkyl oder Aralkyl ist oder
Z eine ungesättigte Kette mit 3 bis 5 Gliedern aus Stickstoffatomen oder aus Kohlenstoffatomen und gegebenenfalls einem oder mehreren Stickstoff- oder Schwefelatomen darstellt, wobei Kohlenstoffatome gegebenenfalls durch Alkyl, Alkoxy, Hydroxyalkyl, Alkylthio, Hydroxy, Aralkyl, Aryl, Carboxy, Carboxamido, Alkoxycarbonyl, Cyano, Amino, das gegebenenfalls durch ein oder zwei gegebenenfalls einen oder mehrere Hydroxy-, Carboxy- oder/und Alkoxycarbonylreste tragenden Alkylrest substituiert ist, oder/und Halogen sowie Stickstoffatome, die nicht über eine Doppelbindung gebunden sind, gegebenenfalls durch Alkyl oder Aralkyl substituiert sind oder zwei benachbarte Kettensubstituenten gegebenenfalls eine Alkylengruppe bilden, die ihrerseits gegebenenfalls mit Aryl substituiert oder anelliert ist oder ein entsprechender tautomerer Rest
unter der Bedingung, daß wenn
- G: einen Alkancarbonsäurerest,
- R³: Wasserstoff, eine Arylcarbonylgruppe oder eine gegebenenfalls ein- oder mehrfach durch Halogen substituierte Alkylcarbonylgruppe und
- L: einen Pyrazoloheterocyclus-Rest der allgemeinen Formel III mit X-Y in der Bedeutung N=CR⁹, wobei R⁹ die zuvor angegebene Bedeutung hat,
darstellen,
Z keinen 1,2,4-Triazolring bildet,bei dem ein nicht über eine Doppelbindung gebundener Stickstoff mit Wasserstoff substituiert ist.

Ein weiterer Gegenstand der Erfindung ist ein Verfahren zur Herstellung dieser Verbindungen, dadurch gekennzeichnet, daß eine Verbindung der allgemeinen Formel IV

G-D (IV)

in der
- G: einen organischen oder anorganischen Säurerest oder einen Glycosidrest und
- D: eine reaktive Gruppe bedeutet
wobei funktionelle Gruppen in G gegebenenfalls mit Schutzgruppen, wie sie in der Peptid- und Kohlenhydratchemie üblich sind, geschützt sind mit einer Verbindung der allgemeinen Formel V 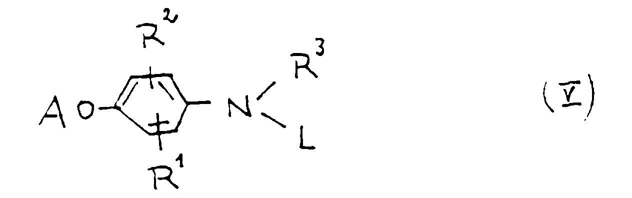 in der
R¹ -R³ und L die für Formel I angegebene Bedeutung haben, R³ zusätzlich auch eine Aminoschutzgruppe bedeuten kann und
A Wasserstoff, ein gegebenenfalls substituiertes Ammoniumion oder ein Alkalimetall darstellt, umgesetzt wird,
und gegebenenfalls abschließend Schutzgruppen abgespalten werden.

Außerdem sind Gegenstand der Erfindung Verbindungen der allgemeinen Formel V' 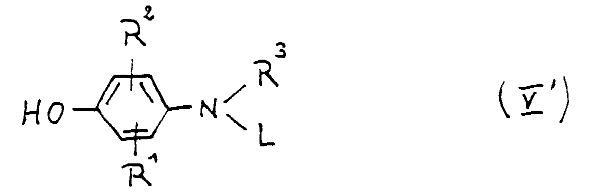 in der
R¹-R³ die für Formel I angegebene Bedeutung haben und
L einen Pyrazoloheterocyclus-Rest der allgemeinen Formel III 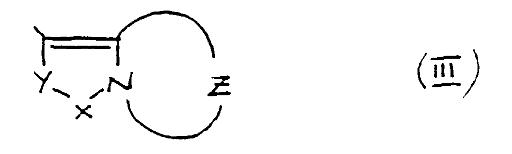 darstellt, in der X-Y und Z die für Formel I angegebene Bedeutung haben,
unter der Bedingung, daß, wenn
- R³: eine Arylcarbonylgruppe oder eine gegebenenfalls ein- oder mehrfach durch Halogen substituierte Alkylcarbonylgruppe darstellt, und
- X-Y: gleich C=NR⁹ ist,
- Z: keinen 1,2,4-Triazolring bildet, bei dem ein nicht über eine Doppelbindung gebundener Stickstoff mit Wasserstoff substituiert ist.

Des weiteren ist Gegenstand der Erfindung ein Verfahren zur Herstellung einer Verbindung der allgemeinen Formel V', dadurch gekennzeichnet, daß
a) eine Substanz der allgemeinen Formel VII 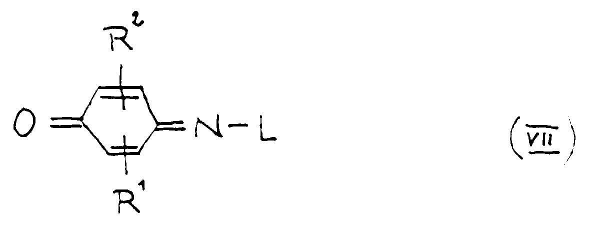 in der
   R¹, R² und L die für Formel V' angegebene Bedeutung haben, reduziert und, wenn R³ nicht Wasserstoff sein soll, die Anilinogruppe acyliert und abschließend eine gegebenenfalls vorliegende Esterbindung gespalten wird oder
b) eine Verbindung gemäß Formel I, wobei L einen Pyrazoloheterocyclus-Rest der allgemeinen Formel III 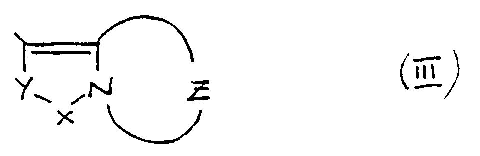 darstellt,
   in der X-Y, G und Z die für Formel I angegebene Bedeutung haben, mit einer entsprechenden Hydrolase umgesetzt wird.

Gegenstand der Erfindung ist auch eine Verbindung der allgemeinen Formel VII 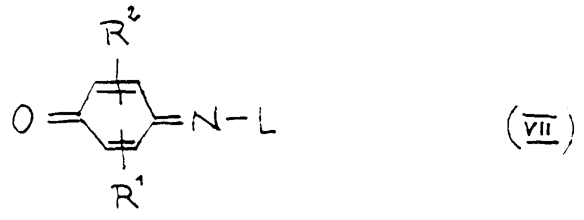 in der
R¹ und R² die für Formel I angegebene Bedeutung haben und
L einen Pyrazoloheterocyclus-Rest der allgemeinen Formel III 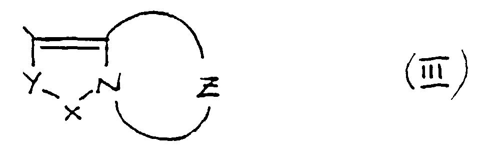 darstellt,
in der X-Y und Z die für Formel I angegebene Bedeutung haben.

Weiter ist Gegenstand der Erfindung ein Verfahren zur Herstellung einer Verbindung der allgemeinen Formel VII, dadurch gekennzeichnet, daß
a) eine Verbindung gemäß Formel I, wobei L einen Pyrazoloheterocyclus-Rest der allgemeinen Formel III 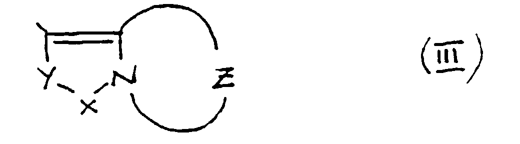 darstellt,
   in der X-Y und Z die für Formel I angegebene Bedeutung haben
   mit einer Hydrolase umgesetzt und das Reaktionsprodukt oxidiert wird oder b) eine Verbindung der allgemeinen Formel VIII

   NH₂-L (VIII)

   in der
   L einen Pyrazoloheterocyclus-Rest der allgemeinen Formel III 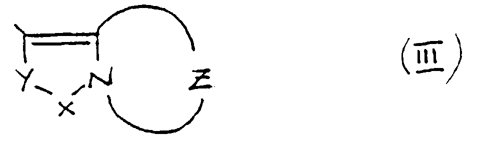 darstellt,
   in der X-Y und Z die für Formel I angegebene Bedeutung haben
   mit einem Phenol der allgemeinen Formel IX 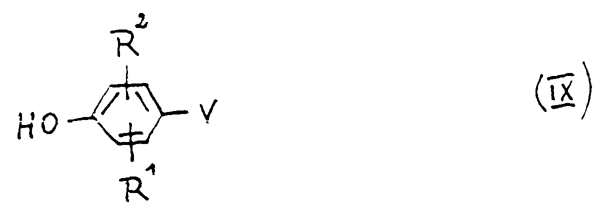 in der
   - R¹ und R²: die für Formel VII angegebene Bedeutung haben und
   - V: Wasserstoff, Halogen, CO₂H oder SO₃H darstellt, in Gegenwart eines Oxidationsmittels umgesetzt wird,
c) eine Verbindung der allgemeinen Formel X

   L-H (X)

   in der L einen Pyrazoloheterocyclus-Rest der allgemeinen Formel III 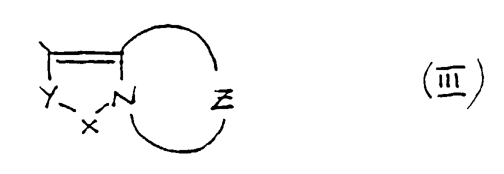 darstellt, wobei X-Y und Z die für Formel I angegebene Bedeutung haben,
   mit einer Verbindung der allgemeinen Formel XI 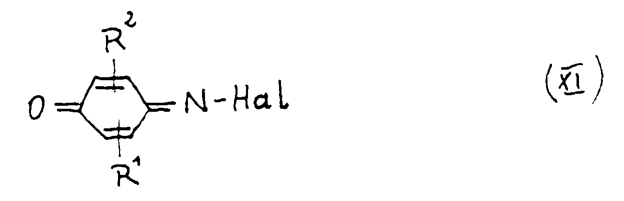 in der
   R¹ und R² die für Formel VII angegebene Bedeutung haben und
   Hal einen Halogenrest bedeutet,
   umgesetzt wird, oder
d) eine Verbindung der allgemeinen Formel XXV

   L-E (XXV)

   in der
   - E: eine Nitro- oder Nitrosogruppe bedeutet und
   - L: für einen Pyrazoloheterocyclus-Rest der allgemeinen Formel III 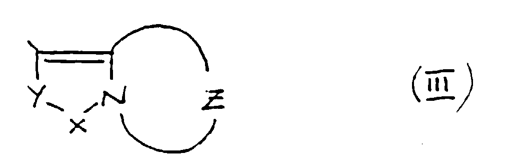
   steht,
   wobei X-Y und Z die für Formel I angegebene Bedeutung haben,
   mit einer metallorganischen Verbindung der allgemeinen Formel XXVI 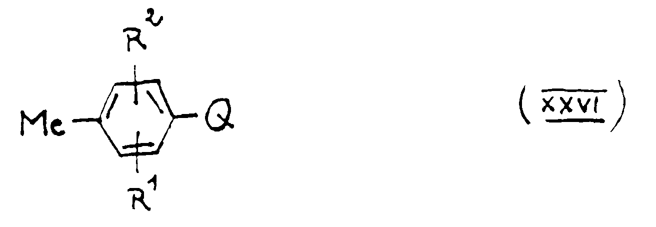 in der
   R¹ und R² die für Formel VII angegebene Bedeutung haben,
   Q eine Hydroxy- oder Dialkylaminogruppe und
   Me Lithium oder durch Halogen substituiertes Magnesium
   bedeuten,
   umgesetzt und anschließend wässrig aufgearbeitet wird.

Ebenso ist Gegenstand der Erfindung die Verwendung von Verbindungen der allgemeinen Formeln V' und VII zur Herstellung von neuen Verbindungen der allgemeinen Formel I.

Gegenstand der Erfindung ist auch ein Verfahren zur kolorimetrischen Bestimmung einer Hydrolase durch Umsetzung eines chromogenen Enzymsubstrats mit dem Enzym, Oxidation eines durch das Enzym aus dem Substrat freigesetzten Leukofarbstoffs und Bestimmung des resultierenden Farbstoffs als Maß für die Menge an Enzym, dadurch gekennzeichnet, daß als Enzymsubstrat eine Verbindung gemäß der allgemeinen Formel I eingesetzt wird.

Im übrigen ist Gegenstand der Erfindung ein diagnostisches Mittel zur Bestimmung einer Hydrolase enthaltend ein chromogenes Enzymsubstrat und eine geeignete Puffersubstanz, dadurch gekennzeichnet, daß als chromogens Enzymsubstrat eine Verbindung der allgemeinen Formel I eingesetzt wird.

Schließlich ist Gegenstand der Erfindung die Verwendung einer Verbindung der allgemeinen Formel I zur Herstellung eines diagnostischen Mittels zur Bestimmung einer Hydrolase.

Es wurde gefunden, daß Verbindungen der allgemeinen Formel I in der Bedeutung wie vorstehend ausgeführt, Hydrolase-Substrate sind, die die gestellte Aufgabe hervorragend lösen.

Unter einem anorganischen Säurerest in der Definition von G sind vor allem der Ortho- oder Pyrophosphorsäure- oder Schwefelsäurerest zu verstehen, die über eine Esterbindung an das Aminophenolgrundgerüst gebunden sind. Bevorzugt sind die Reste PO₃MM' und SO₃M, insbesondere PO₃MM', wobei im Falle der freien Säuren M und M' Wasserstoff bedeuten, während wenn die Säuren als Salz vorliegen, M und M' für Alkali-, Erdalkali- oder Ammoniumionen stehen.

Unter Alkaliionen in der Definition von M und M' sind vor allem Lithium-, Natrium- und Kaliumionen zu verstehen. Erdalkaliionen sind vor allem Magnesium-, Calcium- oder Bariumionen.

Ammoniumionen in der Definition von M und M' können das unsubstituierte Ammoniumion NH₄+ oder durch Alkyl- oder Aralkylreste ein- oder mehrfach substituierte Ammoniumionen sein. Hierbei ist unter Alkylrest ein Rest aus 1 - 6 Kohlenstoffatomen zu verstehen. Bevorzugt sind der Methyl- oder Ethylrest. Unter einem Aralkylrest ist ein Rest zu verstehen, bei dem eine wie vorstehend definierte Alkylgruppe durch einen Arylrest substituiert ist, wobei Aryl einen Kohlenstoffaromaten- oder Heteroaromatenrest, vorzugsweise einen solchen mit 6 - 10 Ring-Atomen, insbesondere die Phenyl- und die Naphthylgruppe bedeutet. Bevorzugt als Aralkylrest ist der Benzylrest. Die Substituenten substituierter Ammoniumionen können sowohl gleich als auch verschieden sein. Als Ammoniumionen können auch Kationen quaternierter Stickstoffheterocyclen eingesetzt werden. Beispiele hierfür sind das Piperidiniumkation oder das Pyridiniumion.

Unter einem organischen Säurerest in der Definition von G werden vor allem Alkancarbonsäure-, Aminosäure- oder Oligopeptidreste verstanden, welche mit ihrem Carboxylende als Ester an das Aminophenolgrundgerüst der allgemeinen Formel I gebunden vorliegen.

Alkancarbonsäurereste in der Definition von G sind Verbindungen mit 1 - 20 Kohlenstoffatomen. Besonders bevorzugt sind Essigsäure, Propionsäure, Buttersäure, Palmitinsäure und Stearinsäure. Neben gesättigten Säureresten kann G auch ein ungesättigter Säurerest, wie z. B. der Ölsäure-, Linolsäure- oder Linolensäurerest sein.

Als Aminosäurerest kommen vorzugsweise die Reste der natürlichen α-Aminosäuren in ihrer L- oder D-Form oder auch in ihrer racemischen Form in Frage. Besonders bevorzugt sind die Reste von Glycin, Alanin, Valin, Leucin, Isoleucin, Phenylalanin und Tyrosin, wobei jeweils die L-Form ganz besonders bevorzugt ist.

Unter einem Oligopeptidrest sind z. B. Di-, Tri-, Tetra- und Pentapeptide, vorzugsweise Di- und Tripeptide zu verstehen, wobei als Aminosäurekomponenten vorzugsweise die oben erwähnten Aminosäuren Verwendung finden.

Die Aminogruppen der esterartig an den Aminophenolrest gebundenen Aminosäure- oder Oligopeptidreste können frei oder geschützt vorliegen. Als Schutzgruppe sind hier alle üblichen Aminoschutzgruppen zu verstehen, insbesondere Acyl-, Oxycarbonyl-, Thiocarbonyl-, Sulfo-, Sulfino-, Vinyl-, Cyclohexenyl-, Phosphoryl-oder Carbamoyl-Gruppen. Besonders bevorzugte Aminoschutzgruppen sind der Tosyl-, Benzyloxycarbonyl- und tert.-Butoxycarbonyl-Rest.

Ein Glycosidrest in der Definition von G kann ein Mono- oder Oligosaccharid sein. Der Zuckerrest kann α- oder β-glycosidisch an das Aminophenol-Grundgerüst gebunden sein. Beispiele für bevorzugte Monosaccharide sind Galactose, Glucose oder Mannose. Besonders bevorzugt ist N-Acetyl-glucosamin. Ganz besonders bevorzugt ist β-glycosidisch gebundenes N-Acetyl-2-D-glucosamin.

Als Zuckerreste sind aber auch Oligosaccharide geeignet. Als Oligosaccharide werden insbesondere solche bezeichnet, die aus 2 bis 10, vorzugsweise aus 2 - 7 Monosaccharideinheiten aufgebaut sind. Besonders bevorzugt sind Heptaosen.

Aus der Gruppe der organischen oder anorganischen Säurereste und der Glycosidreste in der Bedeutung von G sind die Glycosidreste für Verbindungen der allgemeinen Formel I bevorzugt. Insbesondere der N-Acetylglucosaminrest ist besonders vorteilhaft.

Sofern nichts anderes angegeben ist, haben die folgenden Reste in den hier gebrauchten allgemeinen Formeln folgende Bedeutung: "Alkyl" -auch in Alkylsulfinyl-, Alkylsulfonyl-, Alkylcarbonyl, Alkylthio-, Alkylcarbamoyl-, Alkylamino-, Dialkylphosphinyl-, und Aralkylresten - bedeutet einen geradkettigen oder verzweigten Alkylrest mit 1-6, vorzugsweise 1-4 C-Atomen. Beispiele sind die Methyl-, Ethyl-, Propyl-, iso-Butyl- oder tert.-Butylgruppe.

Wenn eine Aminogruppe durch 2 Alkylreste substituiert ist, können diese Reste auch so zu einem Ring geschlossen sein, daß sie insgesamt einen durch ein Stickstoffatom unterbrochenen Ring darstellen. Bevorzugt sind hierbei solche Aminogruppen, die einen insgesamt 5- oder 6-gliedrigen Ring darstellen und der seiterseits gegebenenfalls durch Sauerstoff, Schwefel oder ein weiteres Stickstoffatom unterbrochen ist. Besonders bevorzugt ist der Morpholinorest.

Ein Hydroxyalkylrest ist ein durch eine Hydroxygruppe substituierter Alkylrest mit 1-6, vorzugsweise 1-4 C-Atomen. Der Hydroxyalkylrest kann einen primären, sekundären oder tertiären Alkoholrest darstellen. Besonders bevorzugt sind der 2-oder 1-Hydroxyethyl- und der Hydroxymethylrest.

"Alkoxy" - auch in Alkoxy- und Aralkoxycarbonylresten - steht für einen geradkettigen oder verzweigten Alkoxyrest mit 1 - 6, vorzugsweise 1 - 4 C-Atomen. Beispiele sind die Methoxy-, Ethoxy-, Propyloxy-, iso-Butyloxy- oder tert.-Butyloxygruppe.

"Aryl" - auch in Arylcarbonyl- und Arylcarbamoylresten - bezeichnet einen Kohlenstoffaromaten- oder Heteroaromatenrest, vorzugsweise einen solchen mit 6 - 10 Ring-Atomen, insbesondere die Phenyl- und die Naphthylgruppe, die zusätzlich noch durch Alkyl, Alkoxy oder/und Halogen substituiert sein können. Besonders bevorzugt ist der Phenylrest.

Ein "Aralkyl"-Rest - auch in einer Aralkylcarbamoylgruppe - bedeutet einen Rest, bei dem eine wie vorstehend definierte Alkylgruppe durch einen wie zuvor charakterisierten Arylrest substituiert ist. Bevorzugt ist die Benzylgruppe.

Ein "Aralkoxy"-Rest, beispielsweise in Aralkoxycarbonylgruppen, bezeichnet einen Rest, bei dem eine wie vorstehend definierte Alkoxygruppe durch einen wie zuvor charakterisierten Arylrest substituiert ist. Bevorzugt ist die Benzyloxygruppe.

"Halogen" steht für die Reste Fluor, Chlor, Brom und Jod. Fluor und Chlor sind bevorzugt.

"Alkenyl" bedeutet einen ungesättigten Kohlenwasserstoffrest mit 2 - 6, vorzugsweise 2 - 4 C-Atomen. Beispiele sind die Vinyl- und Allylgruppe.

Eine Acylgruppe bezeichnet einen Carbonsäurerest, der Alkyl-, Aralkyl- oder Arylreste enthalten kann. Bevorzugt sind Acetyl-, Phenylacetyl- oder Benzoylreste.

Unter einer Alkylengruppe wird eine geradkettige oder verzweigte, gesättigte oder ungesättigte Kohlenwasserstoffkette aus 3 - 5, vorzugsweise 3 oder 4 C-Atomen mit zwei freien Bindungsstellen verstanden.
Beispiele sind -CH₂-CH=CH-, 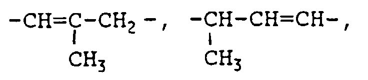 -(CH₂)₄- oder -CH=CH-CH=CH-.
Bevorzugt sind der Butadiendiylrest (-CH=CH-CH=CH-) und der Tetramethylenrest (-(CH₂)₄-).
Unter einer Dialkylphosphinylgruppe wird der Rest 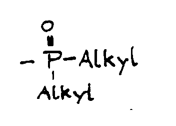 verstanden, wobei Alkyl die zuvor gegebene Bedeutung hat. Bevorzugt ist der Dimethylphosphinylrest. Als Salze von SO₃H, PO₃H₂ und Carboxyresten können Alkali- oder Erdalkalisalze oder Ammoniumsalze eingesetzt werden. Unter Alkalisalzen werden Lithium-, Natrium-, Kalium-, Rubidium- und Cäsiumsalze verstanden, wobei Lithium-, Natrium- und Kaliumsalze, vor allem aber Natrium- und Kaliumsalze bevorzugt sind. Erdalkalisalze sind solche des Berylliums, Magnesiums, Calciums, Strontiums oder Bariums. Bevorzugt sind Magnesium- und Calciumsalze, wobei Calciumsalze besonders bevorzugt sind. Als Ammoniumsalze können solche des unsubstituierten Ammoniumions, NH₄⁺, Verwendung finden. Es ist aber auch möglich, solche Ammoniumsalze einzusetzen, bei denen das Ammoniumion durch 1 - 4 Alkyl-, Aryl- oder Aralkylreste substituiert ist. Für diese Reste gelten die zuvor gegebenen Definitionen, wobei als Alkylrest Methyl, Ethyl und n-Propyl, als Arylrest die Phenylgruppe und als Aralkylrest die Benzylgruppe besonders bevorzugt sind. Als Ammoniumionen können auch Kationen quaternierter Stickstoffheterocyclen eingesetzt werden. Beispiele hierfür sind das Piperidiniumkation oder das Pyridiniumion.

Als Carboxamidorest wird der Rest CONH₂ verstanden, aber auch solche Reste, bei denen die Aminogruppe durch ein oder zwei gegebenenfalls einen oder mehrere Hydroxy-, Carboxy- oder/und Alkoxycarbonylreste tragende Alkylreste substituiert ist.

Vorteilhaft sind vor allem solche erfindungsgemäßen Substanzen, in denen R³ Wasserstoff oder einen ein- oder mehrfach durch Halogen substituierten Alkylcarbonylrest, insbesondere Trifluoracetyl, bedeutet. Ganz besonders bevorzugt in der Bedeutung von R³ ist Wasserstoff.

Erfindungsgemäße Substanzen sind die, bei denen L einen Pyrazoloheterocyclus-Rest der allgemeinen Formel III bedeutet. Hiervon insbesondere solche, in denen Z so angeordnet ist, daß mindestens eine Doppelbindung der ungesättigten Kette in Konjugation zu der Doppelbindung oder zu dem N-Atom der allgemeinen Formel III steht.

Außerdem sind erfindungsgemäße Substanzen, bei denen L einen Pyrazoloheterocyclus-Rest der allgemeinen Formel III bedeutet, besonders bevorzugt, die in der ungesättigten Kette Z, falls diese Stickstoffatome enthält, die nicht über eine Doppelbindung gebunden sind, diese nur mit Alkyl- oder Aralkylresten substituiert aufweist.

Gegebenenfalls sind für Reste der allgemeinen Formel III auch tautomere Formen möglich. Diese sollen ebenfalls als von der allgemeinen Formel III umfaßt gelten.

Erfindungsgemäß bevorzugt sind solche Substanzen der allgemeinen Formel I, in denen der Rest L einen Rest aus der Gruppe der allgemeinen Formeln XIII - XXIV 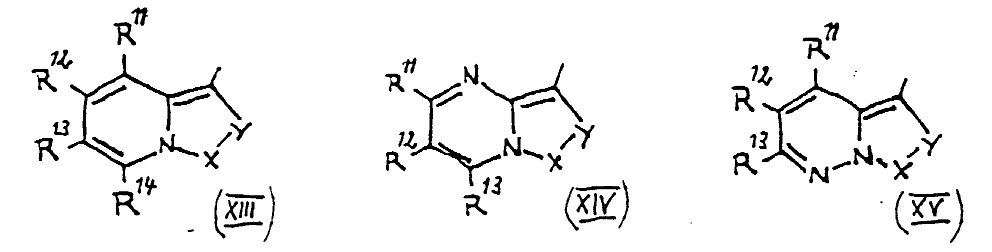 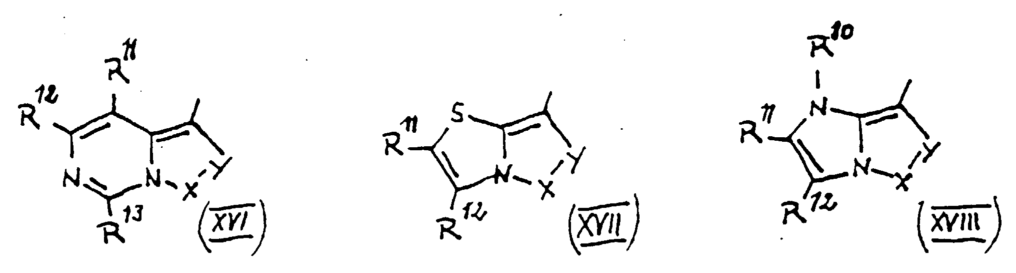 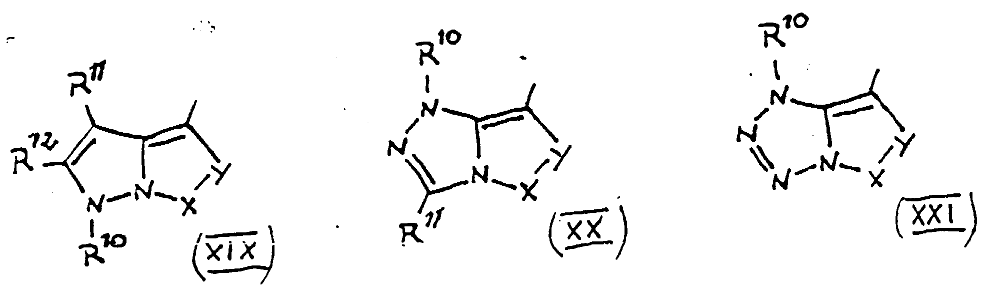 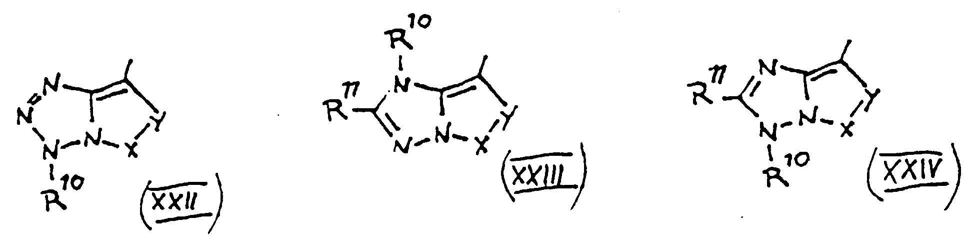 sowie gegebenenfalls entsprechende tautomere Formen bedeutet.

Hierbei haben X-Y und R¹⁰ die gleiche Bedeutung wie zuvor beschrieben. R¹¹, R¹², R¹³ und R¹⁴, die gleich oder verschieden sind, stehen für Wasserstoff, Hydroxy, Alkyl, Alkoxy, Alkylthio, Aralkyl, Aryl, Carboxy, Alkoxycarbonyl, Carboxamido, Cyano, Amino, das gegebenenfalls durch ein oder zwei gegebenenfalls einen oder mehrere Hydroxy-, Carboxy- oder/und Alkoxycarbonylreste tragende Alkylreste substituiert ist oder Halogen, wobei zwei benachbarte Reste gegebenenfalls eine Alkylengruppe bilden, die ihrerseits gegebenenfalls mit Aryl substituiert oder anelliert ist. Die Definitionen der Reste entsprechen den zuvor gegebenen.

Erfindungsgemäß besonders bevorzugt sind Substanzen der allgemeinen Formel I in der L für einen Rest aus der Gruppe der allgemeinen Formeln XIII, XIV, XV, XVII, XVIII und XX und gegebenenfalls entsprechende tautomere Formen steht. Ganz besonders bevorzugt sind solche Substanzen, in denen X-Y die Bedeutung N=CR⁹ hat, wobei R⁹ die Bedeutung haben kann wie für die allgemeine Formel III angegeben, bevorzugt aber Wasserstoff und Alkoxy darstellt.

Hervorragend geeignet im Sinne der vorliegenden Erfindung sind insbesondere das β-glycosidisch gebundene N-Acetyl-β-D-glucosaminid von (4-Hydroxyphenyl)-(2-methylpyrazolo-[1,5-a]-pyridin-3-yl)-amin und von (4-Hydroxyphenyl)-(pyrazolo-[1,5-a]-pyridin-3-yl)amin.

Die Verbindungen der allgemeinen Formel I sind neu. Sie können hergestellt werden, indem man einen Leukoazomethinfarbstoff der allgemeinen Formel V 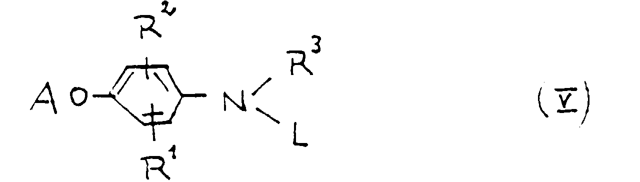 in der R¹-R³ und L die für Formel I gegebene Bedeutung haben und A Wasserstoff, ein gegebenenfalls substituiertes Ammoniumion oder ein Alkalimetall darstellt,
mit einer Verbindung der allgemeinen Formel IV

G-D (IV)

in der G einen organischen oder anorganischen Säurerest oder einen Glycosidrest in der für Formel I angegebenen Bedeutung darstellt, wobei in dem Glycosidrest sich befindliche funktionelle Gruppen wie beispielsweise Amino- oder/und Hydroxygruppen gegebenenfalls mit in der Peptid- und Kohlenhydratchemie üblichen Schutzgruppen substituiert sind, und D eine reaktive Gruppe bedeutet,
umsetzt und gegebenenfalls abschließend Schutzgruppen abspaltet.

Unter einem unsubstituierten Ammoniumion wird NH₄+ verstanden. Gegebenenfalls kann dieses Ion in der Bedeutung von A in der allgemeinen Formel V auch durch Alkyl- oder Aralkylreste wie sie zuvor bereits charakterisiert wurden ein- oder mehrfach substituiert sein. Die Substituenten substituierter Ammoniumionen können sowohl gleich als auch verschieden sein. Als Ammoniumionen können auch Kationen quaternierter Stickstoffheterocyclen eingesetzt werden. Beispiele hierfür sind das Piperidiniumkation oder das Pyridiniumion.

Als Alkalimetall in der Bedeutung von A in der allgemeinen Formel V kommen insbesondere Lithium, Natrium und Kalium in Frage, wobei Natrium bevorzugt ist.

D bedeutet eine reaktive Gruppe, die in der Lage ist, mit der Phenol- oder Phenolatgruppe OA der allgemeinen Formel V eine Reaktion einzugehen. Die Wahl der reaktiven Gruppe ist abhängig von der Natur des Restes G. Handelt es sich bei G um einen Zuckerrest, so ist D vorzugsweise eine gut zu substituierende Gruppe, beispielsweise ein Acetylrest oder ein Halogenatom, welches aus der Gruppe Fluor, Chlor, Brom und Jod ausgewählt werden kann, wobei Chlor, Brom und Jod bevorzugt sind.

Als in der Kohlenhydratchemie übliche Schutzgruppen sind insbesondere der Acetyl-, Benzoyl-, Benzyl- und der Trimethylsilylrest zu nennen.

Wenn G ein Aminosäure- oder Peptidrest bedeutet, der mit seinem Carboxylende mit einem Aminophenol der allgemeinen Formel V verestert werden soll, dann kommen als reaktive Gruppe D die in der Peptidchemie üblichen Gruppen in Frage. Als reaktive Derivate werden z. B. die Säurehalogenide, bevorzugt Säurechloride bzw. die bei der Peptidsynthese üblicherweise verwendeten Mischanhydride oder Aktivester eingesetzt. Die gleichen reaktiven Gruppen können auch für die Bindung von Alkancarbonsäuren an das Aminophenol-Gerüst eingesetzt werden.

Für den Fall, daß G einen anorganischen Säurerest bedeutet, werden Verbindungen der allgemeinen Formel V vorzugsweise mit den entsprechendeen Säurehalogeniden, insbesondere Säurechloriden umgesetzt.

In jedem Fall ist bei einer Veresterung darauf zu achten, daß falls R³ in Formel V Wasserstoff ist, diese Aminogruppe vor Durchführung der Veresterungsreaktion mit einer Schutzgruppe, beispielsweise einer aus der Peptidchemie zu diesem Zweck üblichen Gruppe, substituiert wird und die Schutzgruppe abschließend wieder entfernt wird.

Beispielhaft soll das Verfahren zur Herstellung von Verbindungen der allgemeinen Formel I am Beispiel der besonders bevorzugten Verbindungen, in denen G einen N-Acetyl-β-D-glucosaminidrest darstellt, vorgestellt werden. Dieses Verfahren ist entsprechend auch auf die Herstellung anderer Glycosidderivate der allgemeinen Formel I übertragbar.

Erfindungsgemäße N-Acetyl-β-D-glucosaminidylderivate der allgemeinen Formel I können hergestellt werden, indem als Verbindung der allgemeinen Formel IV
eine Verbindung der allgemeinen Formel VI 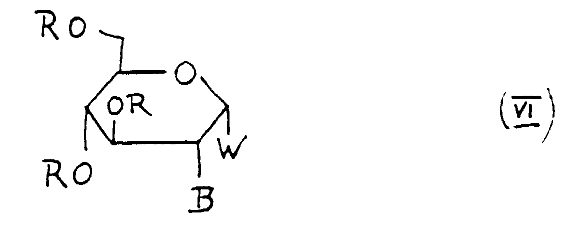 in der
W ein Halogenrest,
R eine in der Kohlenhydratchemime übliche Hydroxyschutzgruppe und
B ein Azidrest, eine geschützte Aminogruppe oder NHCOCH₃ darstellen oder
B und W zusammen die Gruppe 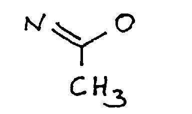 bedeutet,
mit einer Verbindung der allgemeinen Formel V 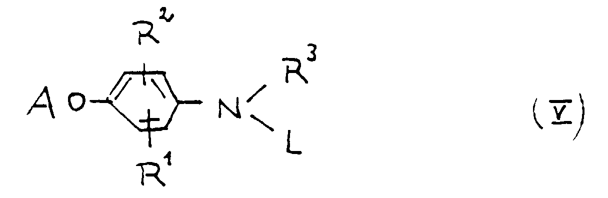 in der
R¹ -R³ und L die für Formel I angegebene Bedeutung haben und
A Wasserstoff, ein gegebenenfalls substituiertes Ammoniumion oder ein Alkalimetall darstellt,
umgesetzt wird,
wenn B eine geschützte Aminogruppe darstellt, die Amino-schutzgruppe entfernt oder
wenn B einen Azidrest darstellt, dieser durch Reduktion in eine NH₂-Gruppe überführt und die Aminogruppe durch Acetylierung in den NHCOCH₃-Rest umgewandelt wird, und abschließend die Hydroxyschutzgruppen abgespalten werden.

Eine Möglichkeit besteht beispielsweise darin, einen Leukoazomethinfarbstoff der allgemeinen Formel V 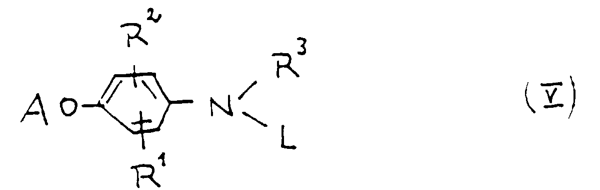 mit der zuvor angegebenen Bedeutung mit einem per-O-substituierten 1-Halogen-N-acetyl-Glucosamin der Formel VI, 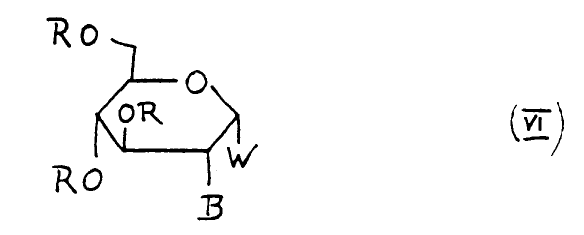 in der W Halogen, B NHCOCH₃ und R eine in der Kohlenhydratchemie gebräuchliche Schutzgruppe bedeuten, unter Walden-Umkehr am C-1Atom des Zuckerrestes
zu einem per-O-substituierten β-Glycosid umzusetzen und von letzterem nach an sich bekannten Methoden die Hydroxy-Schutzgruppen abzuspalten.

Die Umsetzung der Verbindungen der Formel V und VI in der zuvor genannten Bedeutung zu N-Acetyl-β-D-glucosaminiden der allgemeinen Formel I, wird vorzugsweise in Gegenwart von Säurefängern, wie Alkalihydroxid, -carbonat oder -bicarbonat in wässrigem Aceton oder unter Phasentransferbedingungen in einem Wasser/Benzol oder Wasser/Chloroform-Gemisch vorgenommen (vgl. Synthesis 223 (1988).

Des weiteren können die N-Acetyl-β-D-glucosaminide der allgemeinen Formel I hergestellt werden, indem man die Leukoazomethinfarbstoffe der allgemeinen Formel V mit A gleich Wasserstoff zunächst mit Alkalihydroxid oder - alkoholat in die Alkalisalze bzw. mittels gegebenenfalls substituierten Aminen in die Ammoniumsalze überführt, wobei Alkalimetall und Ammoniumion, die zuvor gegebene Bedeutung haben können und diese dann in dipolaren, aprotischen Lösungsmitteln wie Aceton, Dimethylsulfoxid Dichlormethan, oder Dimethylformamid mit den per-O-substituierten 1-Halogen-N-acetyl-glucosaminen umsetzt.

Ferner haben sich bei der Synthese von N-Acetylglucosaminiden der allgemeinen Formel I aus den Leukoazomethinfarbstoffen der allgemeinen Formel V und 1-Halogeno-N-acetylglucosaminen Zusätze von einzelnen Silbersalzen oder Gemischen von Silbersalzen (Silberoxid, - carbonat, auf Celite®, -triflat, -salicylat) und/oder von einzelnen Quecksilbersalzen oder Gemischen von Quecksilbersalzen (Quecksilberbromid, -cyanid, -acetat, -oxid), gegebenenfalls unter Verwendung von Trocknungsmitteln wie Calciumchlorid oder Drierit®, in Lösungsmitteln, wie Methylenchlorid, Chloroform, Benzol, Toluol oder Dioxan, bewährt.

Ebenso kann ein Oxazolin der allgemeinen Formel VI, in der B und W zusammen die Gruppe 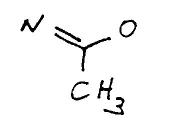 bedeuten, in Gegenwart von organischen Säuren wie z.B. p-Toluolsulfonsäuren oder Lewis-Säuren wie BF₃-Etherat oder FeCl₃ zur Synthese von N-Acetylglucosaminiden der allgm. Formel I verwendet werden. Beispiele für solche Glycosidierungsreaktionen sind in Carbohydrate Research 136, 309-323 (1985), und 64, 334-338 (1978) beschrieben.

Schließlich sind Verfahren zur Herstellung der N-Acetylglucosaminide der allgemeinen Formel I durchführbar, bei denen in der Substanz der allgemeinen Formel VI B eine mit einer Schutzgruppe z.B. Benzyloxycarbonyl, Allyloxycarbonyl-Dichloracetamido oder Phthalimido- substituierte Aminogruppe oder ein geeigneter unter den Glycosidierungsbedingungen stabiler Substituent, aus dem eine Aminogruppe freigesetzt werden kann z.B. ein Azidrest bedeutet. Es wird die Glycosidierungsreaktion durchgeführt, durch Abspaltung der Schutzgruppen nach den Methoden der Peptidchemie oder Reduktion einer Azidogruppe, die Aminogruppe freigesetzt, die in einem letzten Schritt selektiv N-acetyliert wird (siehe z.B. J. Org.Chem. 32, 3767 (1967)).

Die Abspaltung der Schutzgruppen erfolgt nach der in der Kohlehydratchemie bekannten Weise durch Hydrogenolyse bei Schutzgruppen auf Benzylbasis, durch Einwirkung von Natriummethylat, Natriumcyanid, Natriumcarbonat oder Natriumbicarbonat in Methanol zur Abspaltung von Acylgruppen z.B. Acetyl. Die Methoden der Schutzgruppenabspaltung sind beschrieben in Adv. Carbohydr. Chem. Biochem. 39, 13 (1981).

Die Synthese von 1-Halogeno-N-acetyl-glucosaminen ist beschrieben, z. B. in Org Synth. Vol. 46, S. 1, Methods in Carbohydrate Chem. 6 282 (1972) oder J. Org. Chem. 26 445 (1961).

Die zur Herstellung der erfindungsgemäßen Verbindungen der allgemeinen Formel I benötigten Leukoazomethinfarbstoffe der allgemeinen Formel V' 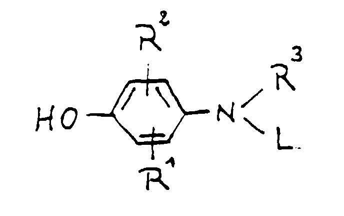 in der R¹-R³ und L die für die allgemeine Formel (I) angegebene Bedeutung haben, wobei L einen Pyrazoloheterocyclus-Rest der allgemeinen Formel III 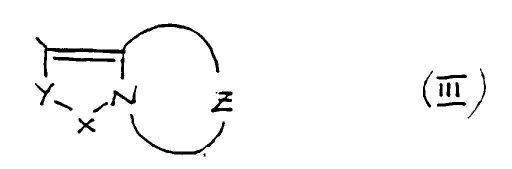 darstellt, in der X-Y und Z die für entsprechende Verbindungen der allgemeinen Formel I bereits angegebene Bedeutung haben, sind mit Ausnahme derjenigen, die gemäß GB-A-2 061 537 als R³ eine Arylcarbonylgruppe oder eine gegebenenfalls ein- oder mehrfach durch Halogen substituierte Alkylcarbonylgruppe tragen und wobei Z einen 1,2,4-Triazolring darstellt, bei dem ein nicht über eine Doppelbindung gebundener Stickstoff mit Wasserstoff substituiert ist, wenn X-Y C=NR⁹ bedeutet, ebenfalls neu.

Sie lassen sich durch Reduktion der entsprechenden Farbstoffe der allgemeinen Formel VII, 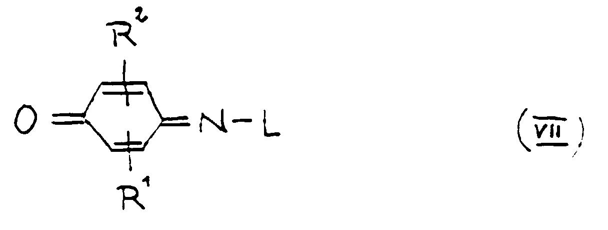 in der
R¹, R² und L die für Verbindungen der allgemeinen Formel V' angegebene Bedeutung haben, nach an sich bekannten Methoden mit Reduktionsmitteln, wie katalytische Hydrierung, Natriumborhydrid, Palladium/Hydrazin oder Natriumdithionit herstellen. Solche Reduktionsmittel sind in Houben/Weyl Bd. 4/1c, und 4/1d beschrieben.

Acylgruppen R³, wie sie für Verbindungen der allgemeinen Formel I angegeben sind, können entweder auf der Stufe des Leukofarbstoffs der allgemeinen Formel V' mit R³ gleich Wasserstoff oder des geschützten per-O -substituierten N-Acetyl-glucosaminids, wie es bei der Herstellung der Verbindungen der allgemeinen Formel I mit G gleich Glycosid vorkommt eingeführt werden. Es werden aktivierte Säurederivate z.B. Halogenide, Anhydride, gemischte Anhydride verwendet, wie sie aus der Peptidchemie bekannt sind.

Ausgehend von Verbindungen der allgemeinen Formel I lassen sich selbstverständlich Verbindungen der allgemeinen Formel V' auch durch Umsetzung mit einer entsprechenden Hydrolase erhalten.

Die für die Herstellung von Verbindungen der allgemeinen Formel V' erforderlichen Farbstoffe der allgemeinen Formel VII sind ebenfalls neu. Sie lassen sich am zweckmäßigsten durch oxidative Kupplung einer Aminoverbindung der allgemeinen Formel VIII

NH₂-L (VIII)

in der L einen Pyrazoloheterocyclusrest der allgemeinen Formel III darstellt, in der X-Y und Z die für Verbindungen der allgemeinen Formel I angegebenen Bedeutungen haben mit einem Phenol der allgemeinen Formel IX 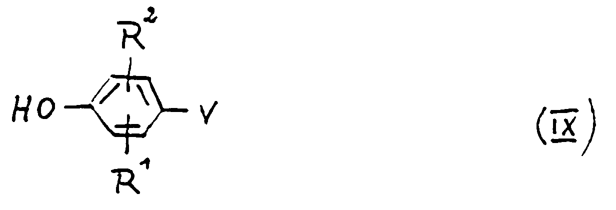 in der R¹ und R² die für Verbindungen der allgemeinen Formel I angegebenen Bedeutungen haben und V Wasserstoff, Halogen, Carboxy oder SO₃H darstellt, herstellen. Dazu werden eine Aminoverbindung der allgemeinen Formel VIII und ein Phenol der allgemeinen Formel IX bevorzugt mit V gleich Wasserstoff in Gegenwart eines Oxidationsmittels wie Kaliumferricyanid, Kaliumperoxodisulfat, Kaliumperoxomonosulfat, Jod, Wasserstoffperoxid/Peroxidase, Bleidioxid, Natriumhypochlorit, Natriumhypobromit oder organischen Oxidationsmitteln, wie N-Bromsuccinimid und verwandte Verbindungen umgesetzt.

Ferner lassen sich die Farbstoffe der allgemeinen Formel VII auch durch Umsetzung von N-Halogeniminen der allgemeinen Formel XI 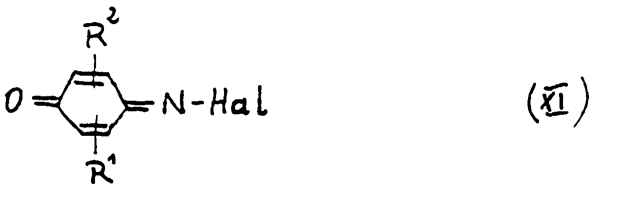 in der R¹ und R² die für Verbindungen der allgemeinen Formel I angegebene Bedeutung haben und Hal einen Halogenrest bedeutet, wobei Halogen Fluor, Chlor, Brom oder Jod, vorzugsweise Chlor, ist, mit Verbindungen der allgemeinen Formel (X)

L-H (X)

in der L einen Pyrazoloheterocyclus-Rest der allgemeinen Formel III bedeutet, wobei X-Y und Z die für Verbindungen der allgemeinen Formel I angegebene Bedeutung haben, herstellen.

Die Reaktionsbedingungen lassen sich analog den in Houben/Weyl Bd. 7/3 b S. 296 f. beschriebenen wählen. Ausgehend von Verbindungen der allgemeinen Formel I lassen sich Farbstoffe der allgemeinen Formel VII auch durch Umsetzung mit einer entsprechenden Hydrolase und anschließende Oxidation erhalten. Zur Oxidation sind grundsätzlich die gleichen Substanzen geeignet wie für die oxidative Kupplung zwischen Verbindungen der allgemeinen Formel VIII mit solchen der allgemeinen Formel IX beschrieben.

Vorteilhaft lassen sich Farbstoffe der allgemeinen Formel VII auch durch Umsetzung einer Verbindung der allgemeinen Formel XXV

L-E (XXV)

in der E eine Nitroso- oder Nitrogruppe bedeutet und L für einen Pyrazoloheterocyclus-Rest der allgemeinen Formel III steht, wobei X-Y und Z die für Formel I angegebene Bedeutung haben, mit einer metallorganischen Verbindung der allgemeinen Formel XXVI 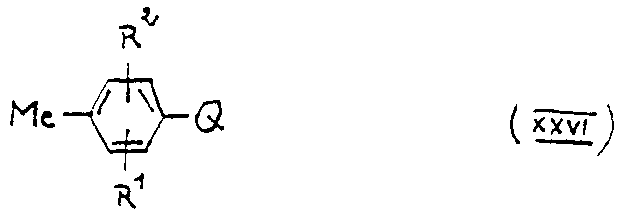 in der
- R₁ und R₂: die für Formel VII angegebene Bedeutung haben,
- Me: für Lithium oder durch Halogen substituiertes Magnesium und
- Q: für eine Hydroxy- oder Dialkylaminogruppe steht
und anschließende wässrige Aufarbeitung der Reaktionsmischung erhalten. Dabei sind eventuell in den Verbindungen der allgemeinen Formeln XXV und XXVI vorkommende Reste, die nicht mit der Grignard- oder Lithium organischen Verbindung reagieren sollen entsprechend zu schützen. Informationen über derartige Schutzgruppen finden sich z. B. in Th. Greene: "Protecting groups in Organic Synthesis", John Wiley, New York (1981), J.F.W. McOmie: "Protective groups in Organic Chemistry", Plenum Press, London (1973). Enthält die Verbindung der allgemeinen Formel XXV keine funktionellen Gruppen (außer einem Nitro- oder Nitrosorest), die mit metallorganischen Verbindungen eine Reaktion eingehen können, wie beispielsweise Ester, dann ist eine solche Reaktion bevorzugt zur Herstellung der Verbindungen der allgemeinen Formel VII. Störende Reste können gegebenenfalls geschützt und nach der Reaktion mit der metallorganischen Verbindung die Schutzgruppen wieder entfernt werden.

Steht Q für N,N-Dialkylamino, insbesondere Dimethylamino, so läßt sich der als Zwischenprodukt entstehende Farbstoff der allgemeinen Formel XXVI' 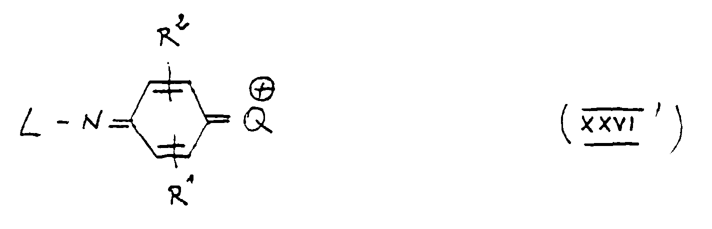 in der
- R¹ und R²: die gleiche Bedeutung wie für Formel XXVI haben,
- L: für den für Formel XXV näher bezeichneten Rest steht und
- Q: einen Dialkylaminorest bedeutet.
isolieren und durch alkalische Hydrolyse, beispielsweise mit Natriumcarbonat in Wasser/Ethanol in den gewünschten Farbstoff der allgemeinen Formel VII umwandeln.

Die Umsetzung von Nitroso- oder Nitroverbindungen mit lithiumorganischen oder Grignard-Verbindungen ist beschrieben (Houben/Weyl, Methoden der organischen Chemie, Band 10/1, S. 1087, 1126; J. Chem. Soc. C, 2119 (1971) und führt je nach dem angewandten Überschuß zu Hydroxylaminderivaten oder sekundären Aminen und weiteren Produkten. Überraschenderweise wurde gefunden, daß sich die Verbindungen der allgemeinen Formeln XXV und XXVI bei Einsatz in äquimolaren Mengen und anschließende wässrige Aufarbeitung der Reaktionsmischung mit hohen Ausbeuten zu Farbstoffen der allgemeinen Formel VII umsetzen lassen.

Verbindungen der allgemeinen Formel VIII, worin L einen Pyrazoloheterocyclusrest der allgemeinen Formel III bedeutet, können analog bekannten Methoden hergestellt werden, indem man eine Verbindung der allgemeinen Formel X, in der L einen Pyrazoloheterocyclus-Rest der allgemeinen Formel III bedeutet nach an sich bekannten Methoden in die entsprechende Aminoverbindung überführt.

Dies kann
a) durch Umsetzung mit Salpetersäure oder Salpetersäure im Gemisch mit Schwefelsäure und/oder Acetanhydrid zu der entsprechenden Nitro-Verbindung oder
b) durch Reaktion mit salpetriger Säure zu der entsprechenden Nitrosoverbindung oder
c) durch Reaktion mit einem aromatischen Diazoniumsalz zu der entsprechenden Arylazoverbindung
und anschließende Reduktion erreicht werden.

Nitro-, Nitroso- und Arylazoreste, das sind Reste Aryl-N=N-, wobei Aryl die gleiche Bedeutung haben kann, wie einleitend für Arylreste und andere solche Reste enthaltende Gruppen erläutert, lassen sich durch Reduktion mit Reagentien, wie Zink in Säure, beispielsweise Salzsäure oder Eisessig, Natriumd'ithionit, Zinn in Säure, beispielsweise Salzsäure, Zinn(II)-chlorid oder durch katalytische Hydrierung beispielsweise über Palladium/Kohle in die Aminoverbindung überführen. Solche Reaktionen sind in Houben-Weyl, Methoden der organischen Chemie, Bd. 11/1, S. 341 f beschrieben.

Die Einführung von Nitro, Nitroso oder Arylazogruppen ausgehend von Verbindungen der Formel X kann durch Nitrierung mit Salpetersäure oder Salpetersäure in Gemischen mit konzentrierter Schwefelsäure oder Acetanhydrid erfolgen.Durch Nitrosierung mit salpetriger Säure oder durch Azokupplung mit aromatischen Diazoniumsalzen, lassen sich die Nitrosogruppe bzw. eine Arylazogruppe einführen. Beispiele für solche Reaktionen sind in Houben-Weyl, Methoden der organischen Chemie Bd. 10/1 und 10/3 beschrieben.

Liegen Heterocyclen der allgemeinen Formel X vor, in der H nicht Wasserstoff, sondern Carboxyl, Alkoxycarbonyl oder Alkylcarbonyl bedeutet, so können diese durch Hydrolyse mit konzentrierter Salzsäure und - bei Carbonsäuren - thermischer Decarboxylierung in die Verbindungen der allgemeinen Formel X überführt werden. Daran schließt sich dann die Einführung einer Nitro-, Nitroso- oder Arylazogruppe an.

Stickstoffatome, die nicht an einer Doppelbindung stehen, und in den Resten X-Y oder Z der allgemeinen Formel III vorkommen, können gegebenenfalls alkyliert oder aralkyliert sein. Die N-Alkylierung bzw. N-Aralkylierung läßt sich durch Umsetzung der entsprechenden Verbindungen der Formel X, vorzugsweise jedoch solchen Heterocyclen, in denen H nicht Wasserstoff, sondern Nitro, Nitroso, Alkoxycarbonyl, Acyl und Arylazo bedeutet, mit Alkylierungs- bzw. Aralkylierungsmitteln, wie Alkyl- oder Aralkylhalogeniden, Dialkyl- bzw. Diaralkylsulfaten oder Arylsulfonsäurealkylestern bzw. -aralkylestern in Gegenwart einer Base, wie Natriumhydrid, tertiären Aminen, Alkalicarbonaten oder Natriumhydroxid in Lösungsmitteln, wie Dimethylformamid oder wässrigalkoholische Systeme durchführen.

Die benötigten Ausgangsprodukte der allgemeinen Formel X oder solche der allgemeinen Formel X entsprechende Verbindungen in denen H ersetzt ist durch Alkoxycarbonyl oder Acyl, wobei L einen Pyrazoloheterocyclen-Rest der allgemeinen Formel III bedeutet, sind beschrieben oder lassen sich analog zu bekannten Verbindungen synthetisieren. Informationen über die Herstellung der heterocyclischen Systeme sind in folgenden Publikationen enthalten: G.P. Ellis "Synthesis of fused Heterocyles", in "The Chemistry of Heterocyclic Compounds", E.C. Taylor Eds., 1987, John Wiley and Sons; P.N. Preston, "Condensed Imidazoles" in "The Chemistry of Heterocyclic Compounds", A. Weissberger und E.C. Taylor Eds., 1986, John Wiley and Sons; Adv. of Het. Chem. 36, 343 (1984); Chem. Pharm. Bull. 22, 482 (1974); J. Het. Chem. 12, 481 (1975); Chem. Pharm. Bull. 22, 1814 (1974); Ann. 660, 104 (1962); Chem. Pharm. Bull. 23, 452 (1975); J. Het. Chem. 10, 411 (1973); J. Chem. Soc. Perkin I 2047 (1977).

Die Verbindungen der allgemeinen Formel I sind hervorragend zur Verwendung als Hydrolase-Substrate geeignet. Besonders bevorzugt für diese Verwendung sind Verbindungen der allgemeinen Formel I, wie sie einleitend bereits charakterisiert wurden. Ganz besonders bevorzugt sind Substanzen der allgemeinen Formel I in der G einen N-Acetyl-β-D-glucosaminidylrest bedeutet zur Verwendung als Substrate für das Enzym N-Acetyl-β-D-glucosaminidase (NAGase).

Zur Durchführung des erfindungsgemäßen Verfahrens zur kolorimetrischen Bestimmung einer Hydrolase wird eine Verbindung der allgemeinen Formel I als chromogenes Enzymsubstrat mit dem zu bestimmenden Enzym umgesetzt, der durch Substratspaltung resultierende Leukofarbstoff wird oxidiert und der sich daraus ergebende Farbstoff wird visuell oder photometrisch bestimmt. Er stellt ein Maß für die Menge an zu bestimmendem Enzym dar.

Für die Oxidation des durch Hydrolase-Einwirkung zunächst entstehenden Leukofarbstoffs der allgemeinen Formel V kann jedes Oxidationsmittel verwendet werden, welches die Aktivität des zu bestimmenden Enzyms nicht beeinflußt und welches stark genug ist, den resultierenden Leukofarbstoff zu oxidieren. In der Regel werden als Oxidationsmittel Kaliumhexacyanoferrat-(III), Perborat, Bilirubinoxidase, Peroxidase/Wasserstoffperoxid oder vorzugsweise Jodat eingesetzt.

Wie sich den vorstehenden Ausführungen entnehmen läßt, ist eine Umsetzung des Substrats durch das Enzym in Gegenwart des Oxidationsmittels bevorzugt. Es ist aber auch möglich, Hydrolase-Reaktion und Oxidation getrennt durchzuführen. Nach letzterem ist allerdings dann keine kinetische, sondern nur eine Endpunktbestimmung möglich.

Daß für die Durchführung des Bestimmungsverfahrens ein auf das Enzym abgestimmtes Puffersystem vorliegen muß, ist für den Fachmann selbstverständlich. Welches Puffersystem für welches Enzym am geeignetsten ist, ist bekannt. Beispielsweise kann das erfindungsgemäße Bestimmungsverfahren für N-Acetyl-β-D-glucosaminidase als Hydrolase bei einem pH-Wert von 3,5 - 7,0, vorzugsweise 4,0 - 6,5 durchgeführt werden.

Das diagnostische Mittel zur Bestimmung einer Hydrolase gemäß der vorliegenden Erfindung enthält neben einem oder mehreren der erfindungsgemäßen Substrate der allgemeinen Formel I, ein geeignetes Puffersystem sowie gegebenenfalls weitere geeignete, üblicherweise für solche Reagenzien verwendete Zusatzstoffe, wie beispielsweise weitere Hilfsenzyme, Stabilisatoren usw. Das zur Bildung des Farbstoffs nach Spaltung des Enzymsubstrats notwendige Oxidationsmittel kann zusammen mit den übrigen für das Bestimmungsverfahren notwendigen Substanzen oder getrennt von diesen vorliegen. Das erfindungsgemäße Reagenz kann in Form einer Lösung, als Lyophilisat, als Pulvergemisch, Reagenztablette oder auf ein geeignetes Trägermaterial aufgebracht vorliegen, wobei die Reagenzbestandteile zusammen, getrennt oder je nach Verträglichkeit und Zweckmäßigkeit miteinander kombiniert zu dem diagnostischen Mittel verarbeitet sein können.

Das erfindungsgemäße diagnostische Mittel in Form einer Lösung enthält vorzugsweise sämtliche für den Test benötigten Reagenzien. Als Lösungsmittel kommen Wasser oder Gemische von Wasser mit einem wasserlöslichen organischen Lösungsmittel, wie z. B. Methanol, Ethanol, Aceton oder Dimethylformamid in Frage. Aus Haltbarkeitsgründen kann es vorteilhaft sein, die für den Test benötigten Reagenzien auf zwei oder mehr Lösungen zu verteilen, die erst bei der eigentlichen Untersuchung vermischt werden.

Zur Herstellung des diagnostischen Mittels in Form eines Lyophilisats im Gesamtgewicht von jeweils etwa 5 - 20 mg, vorzugsweise etwa 10 mg, wird eine Lösung getrocknet, die neben sämtlichen für den Test benötigten Reagenzien übliche Gerüstbildner, wie z. B. Polyvinylpyrrolidon und eventuelle weitere Füllstoffe, wie z. B. Mannit, Sorbit oder Xylit enthält. Das Oxidationsmittel kann in dem diagnostischen Mittel aber auch getrennt von den anderen Substanzen enthalten sein. Dies läßt sich durch separate Lyophilisation oder Zumischung des ungelösten Oxidationsmittels erreichen.

Ein Reagenz in Form von Pulvermischungen oder Reagenztabletten läßt sich herstellen, indem die Bestandteile des Testes mit üblichen galenischen Zusatzstoffen versetzt und granuliert werden. Zusatzstoffe dieser Art sind z. B. Zuckeralkohole, wie Mannit, Sorbit oder Xylit, oder andere lösliche inerte Verbindungen, wie Polyethylenglykole oder Polyvinylpyrrolidon. Die Pulvermischungen oder Reagenztabletten weisen im allgemeinen ein Endgewicht von ungefähr 30 - 200 mg, vorzugsweise 50 - 80 mg, auf.

Feste Reagenzmischungen, wie Lyophilisate, Pulvermischungen oder Tabletten werden vor Gebrauch in Wasser oder einem anderen geeigneten Lösungsmittel gelöst und so die Reagenzlösung(en) bereitet. Nach Vermischen der Probe mit einem ausreichendem Teil der Reagenzmischung wird die entstehende Farbe an einem Photometer vermessen, und über den molaren Extinktionskoeffizienten und die zugesetzten Reagenz- bzw. Probenvolumina die jeweilige Enzym-Konzentration berechnet. Es sind sowohl kinetische als auch Endpunktmessungen möglich.

Zur Herstellung des Reagenzes in Form eines Teststreifens wird ein saugfähiger Träger, vorzugsweise Filterpapier, Zellulose oder Kunstfaservlies, mit Lösungen der erforderlichen, üblicherweise zur Herstellung von Teststreifen verwendeten Reagenzien in leicht flüchtigen Lösungsmitteln, wie z. B. Wasser, Methanol, Ethanol oder Aceton, imprägniert. Dies kann in einem Imprägnierschritt erfolgen. Oft ist es jedoch zweckmäßig, die Imprägnierung in mehreren Schritten durchzuführen, wobei Lösungen eingesetzt werden, die jeweils einen Teil der Bestandteile des Reagenzes enthalten. So kann beispielsweise in einem ersten Schritt mit einer wässrigen Lösung, die das Oxidationsmittel, den Puffer und gegebenenfalls andere wasserlösliche Zusatzstoffe enthält, und dann in einem zweiten Schritt mit einer Lösung, die das Hydrolase-Substrat enthält, imprägniert werden. Die fertigen Teststreifen können als solche verwendet werden oder in an sich bekannter Weise an Griffen angeklebt oder vorzugsweise zwischen Kunststoffen und feinmaschigen Netzwerken gemäß DBP 21 18 455 eingeriegelt werden.

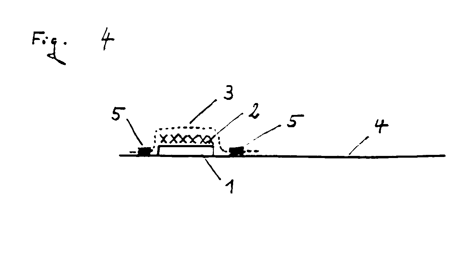

Als bevorzugtes diagnostisches Mittel gemäß der vorliegenden Erfindung wird ein Testträger gemäß Fig. 4 vorgestellt. Dieser Testträger besteht aus einem Puffer und Hydrolase-Substrat enthaltenden saugfähigen Trägermaterial wie Papier (1), einem hierauf angeordneten, in flächigem Kontakt mit dem saugfähigen Träger (1) sich befindlichem, das Oxidationsmittel enthaltendem Netz (2), beispielsweise aus einem Polymermaterial und einem (1) und (2) an einen Griff aus Plastikfolie (4), der die Handhabung erleichtern soll, fixierenden Abdecknetz (3), das ebenfalls aus Polymermaterial bestehen kann. Das Abdecknetz (3) selbst ist mittels Schmelzkleber (5) an der Folie (4) befestigt.

Zur Durchführung einer Hydrolase-Bestimmung wird die zu untersuchende Probe auf das Abdecknetz (3) des Testträgers gemäß Fig. 4 aufgegeben oder dieses in die zu untersuchende flüssige Probe eingetaucht. Die Probe dringt schnell durch das Abdecknetz (3) und unter Lösung des Oxidationsmittels durch das Netz (2) in das saugfähige Trägermaterial (1), wo bei Anwesenheit des Enzyms enzymatische Spaltung des dort befindlichen Enzymsubstrats und Oxidation des intermediär gebildeten Leukofarbstoffs zu dem bestimmbaren Farbstoff erfolgt.

Eine semiquantitative Bestimmung ist möglich, indem man die entstandene Farbe einer Vergleichsfarbe zuordnet.

Eine quantitative Auswertung läßt sich remissionsphotometrisch durchführen.

In der Regel sind die Verbindungen der allgemeinen Formel I zur Verwendung in erfindungsgemäßen diagnostischen Mitteln ausreichend lagerstabil. Es hat sich jedoch herausgestellt, daß die Stabilität der erfindungsgemäßen Hydrolase-Substrate noch weiter erhöht werden kann, wenn sie zusammen mit Verbindungen der allgemeinen Formel XII

Ar-NH-NH-CONH₂ (XII)

in der Ar einen gegebenenfalls durch Alkyl, Alkoxy oder Halogen substituierten Arylrest bedeutet, vorliegen.

"Alkyl", "Alkoxy", "Halogen" und "Aryl" haben in diesem Zusammenhang die gleiche Bedeutung wie einleitend für die Reste der allgemeinen Formel I ausgeführt. Besonders vorteilhafte Verbindungen der allgemeinen Formel XII sind o-Chlor, m-Chlor-, p-Chlor-, o-Methyl-, m-Methyl-, p-Methyl-, o-Methoxy-, m-Methoxy-, p-Methoxy- und unsubstituiertes Phenylsemicarbazid sowie Naphthylsemicarbazid.

Als ganz besonders vorteilhaft haben sich die Verbindungen der allgemeinen Formel (XII) zur Erhöhung der Lagerstabilität von N-Acetylglucosaminiden gemäß der vorliegenden Erfindung erwiesen.

Im Rahmen der vorliegenden Erfindung weisen die Verbindungen der allgemeinen Formel I viele Vorteile auf. Insbesondere ist es von erheblichem Vorteil, daß die erfindungsgemäßen Substrate farblos sind und bei Durchführung des erfindungsgemäßen Verfahrens farbige Substanzen gebildet werden, deren rote oder blaue Farbe deutlich feststellbar ist. Der große Wellenlängenshift, d. h. die Differenz der maximalen Absorptionswellenlängen von Substrat und gebildetem Farbstoff ermöglicht sehr empfindliche Bestimmungen. Insbesondere in biologischen Flüssigkeiten, wie Plasma, Serum, vor allem aber in Harn sind solche Bestimmungen möglich, da sich die entstehende Farbe sehr deutlich vom Probenmaterial unterscheidet. Die nach der Spaltung der erfindungsgemäßen Substrate durch Oxidation gebildeten Farbstoffe werden über einen sehr breiten pH-Bereich gebildet. Vor allem wichtig ist, daß erfindungsgemäß nicht nur im neutralen und basischen pH-Bereich, sondern bereits bei pH-Werten im ziemlich sauren Bereich, d. h. ab pH 3,5 Farbstoff gebildet werden kann und so kein Umpufferungsschritt bei der Bestimmung "saurer" Enzyme, d. h. solcher Enzyme, die im sauren pH-Bereich ihr Aktivitätsoptimum aufweisen, notwendig ist. Dies macht die erfindungsgemäßen Verbindungen der allgemeinen Formel I insbesondere zu geeigneten Substraten für "saure" Enzyme, wie beispielsweise β-NAGase. Die Durchführung der erfindungsgemäßen Hydrolasebestimmung im pH-Optimum der Enzyme mit nicht gefärbten Substraten macht die schnelle Detektion auch von geringen Enzymkonzentrationen möglich. Schließlich sind die erfindungsgemäßen Substrate sowohl für naßchemische Bestimmungen in Lösungen als auch für trockenchemische Bestimmungen auf Testträgern geeignet. Die Auswertung kann visuell und photometrisch in Transmission oder Remission erfolgen.

Die folgenden Beispiele sollen die Erfindung weiter erläutern, ohne sie hierauf zu beschränken.

### Beispiel 1

### 4-(4'-Dimethylaminophenylamino)-phenyl-2-acetamido-2-deoxy-β-D-glucopyranosid

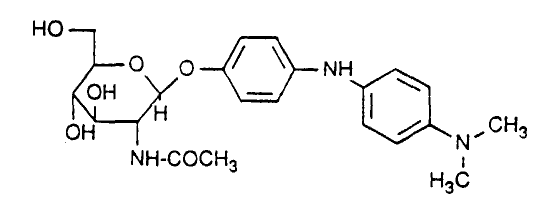

1.1 6 g N,N-Dimethylindoanilin (J. Amer,Chem.Soc 61, 376 (1939) werden zu einer Lösung von 7 g Natriumhydroxid in 250 ml Wasser gegeben. Unter kräftigem Rühren gibt man solange portionsweise Natriumdithionit zu, bis die blaue Farbe verschwunden ist. Die Lösung wird filtriert, das Filtrat auf etwa 5 °C abgekühlt und tropfenweise mit Eisessig versetzt, bis der Niederschlag vollständig ausgefallen ist. Der Niederschlag wird abfiltriert und in 25 ml konzentrierter Salzsäure gelöst. Die Lösung wird mit Kohle behandelt, filtriert und eingedampft. Der Rückstand wird aus Ethanol umkristallisiert. Man erhält 3,1 g (51 % der Theorie) 4-Dimethylamino-4'-hydroxy-diphenylamin.
1.2 2,6 g des in 1.1 erhaltenen Leukofarbstoffes, 8,83 g 2-Acetamido-3,4,6-tri-0-acetyl-2-deoxy-α-D-glucosylchlorid und 4,21 g Benzyltriethylammoniumbromid werden zu einem Gemisch von 125 ml Chloroform und 125 ml Wasser gegeben. Die Mischung wird heftig gerührt, mit 8,7 g Kaliumcarbonat versetzt und 6 Stunden unter Rückfluß gekocht, wobei nach 3 Stunden nochmals 4,4 g der Halogenose und 4,3 g Kaliumcarbonat zugegeben wird. Die Chloroformphase wird abgetrennt, mit Natriumsulfat getrocknet und eingeengt.
   Der Rückstand wird an Kieselgel mit Essigsäureethylester chromatographiert. Die produkthaltigen Fraktionen werden eingeengt. Man erhält 500 mg (9 % der Theorie) des geschützten Zuckerderivates folgender Struktur: 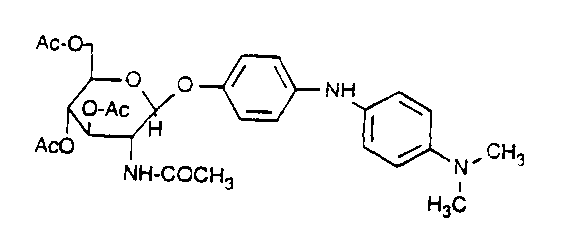
1.3 460 mg des in 1.2 erhaltenen geschützten Zuckerderivates werden in 10 ml Methanol gelöst, mit 0,9 g Natriumbicarbonat versetzt und 3 Stunden bei Raumtemperatur kräftig gerührt. Die Reaktionsmischung wird filtriert, das Filtrat eingedampft und der Rückstand an Kieselgel mit Essigsäureethylester/Methanol (8:2) chromatographiert. Die produkthaltigen Fraktionen werden vereinigt und eingedampft. Der Rückstand wird in wenig Methanol aufgenommen und die Lösung mit Ether versetzt.
   Der so entstehende Niederschlag wird abgesaugt und mit Ether gewaschen. Man erhält 0,34 g (96 % der Theorie) der Titelverbindung mit Fp 207-209°C (Zers.).

### Beispiel 2

Analog Beispiel 1.2 und 1.3 erhält man aus dem entsprechenden Leukofarbstoff und 2-Acetamido-3,4,6-tri-0-acetyl-2-deoxy-a-D-glucosylchlorid die in der folgenden Tabelle 1 aufgeführten Verbindungen.

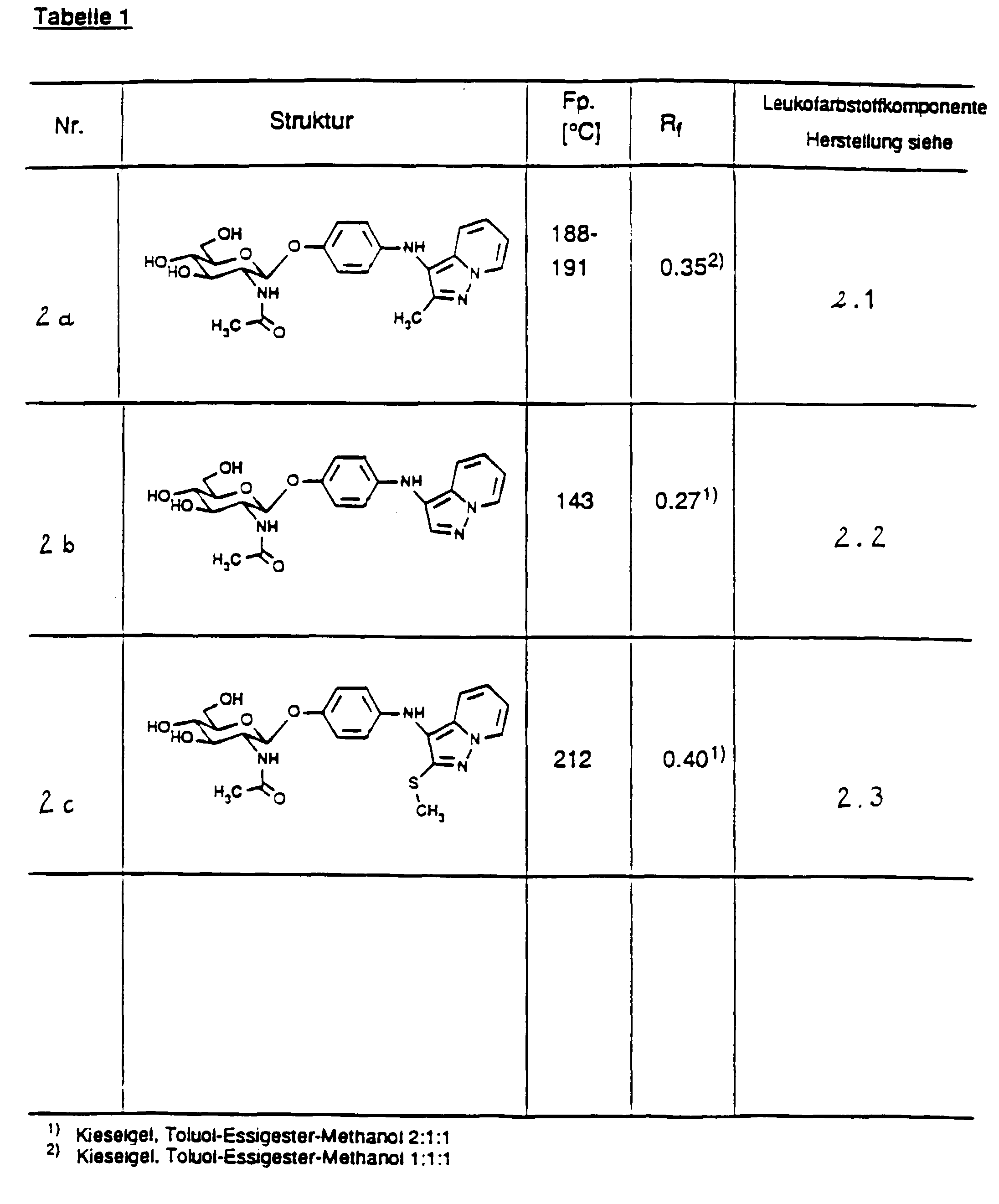

2.1 (4-Hydroxyphenyl)-(2-methylpyrazolo-[1,5-a]-pyridin-3-yl)-amin
   2.1.1 7 g 3-Acetyl-2-methyl-pyrazolo-[1.5-a]-pyridin werden in 140 ml 6 N Salzsäure gelöst und bei 0°C tropfenweise mit einer Lösung von 5,52 g Natriumnitrit in Wasser versetzt. Nach 2 Stunden wird das Eisbad entfernt, die Reaktionsmischung über Nacht bei Raumtemperatur stehen gelassen und dann auf pH 9 gestellt. Die ausgefallene Nitrosoverbindung (6.4 g) wird abgesaugt und in ca. 150 ml 2 N Salzsäure gelöst. Analog Beispiel 2.2.2 wird die Nitrosoverbindung mit Zinn(II)chlorid reduziert. Das Rohprodukt wird an Kieselgel mit Essigsäureethylester chromatographiert. Die Produktfraktionen werden eingeengt, in Ethanol gelöst und mit ethanolischer Salzsäure versetzt. Der nach einiger Zeit ausfallende Niederschlag wird abgesaugt und getrocknet. Man erhält 3.1 g (45 % der Theorie) 3-Amino-2-methyl-pyrazolo-[1,5-a]-pyridin-hydrochlorid mit Fp. > 275°C, R_{f} (Kieselgel, Essigsäureethylester/Aceton/Eisessig/Wasser 50:25:12,5:12,5) = 0.6.
   2.1.2 2,82 g Phenol werden in 75 ml Pyridin gelöst und die Lösung mit 450 ml Wasser versetzt. Dann fügt man eine Lösung von 5,5 g 3-Amino-2-methylpyrazolo-[1,5-a]-pyridin-hydrochlorid aus 2.1.1 in 150 ml Wasser hinzu und versetzt die Mischung anschließend unter Rühren mit einer Lösung von 78 g Kaliumferricyanid in 450 ml Wasser. Der ausgefallene blaue Farbstoff wird abgesaugt, mit Wasser gewaschen und getrocknet. Ausbeute 4,85 g. DC (Kieselgel, Essigsäureethylester/Methylenchlorid 1:1): R_{f} = 0,5).
      2.1.2.1 Alternative Synthese des Farbstoffs N-(2-Methylpyrazolo-[1,5-a]-pyridin-3-yl)-chinonimin
      Aus 7,7 g 4-Bromdimethylanilin und 1,4 g Magnesiumgrieß wird in 60 ml trockenem Tetrahydrofuran die entsprechende Grignardverbindunghergestellt (J. Chem. Soc. 465 (1961). Die Reaktionslösung wird mit einer Spritze abgesaugt und zu einer auf - 15°C gekühlten Lösung von 3,05 g 3-Nitroso-2-methyl-pyrazolo-[1,5-a]pyridin (hergestellt gemäß Beispiel 2.1.1) in 60 ml Tetrahydrofuran getropft. Die Reaktionsmischung wird nach dem Aufwärmen in 100 ml 2 N Essigsäure gegossen. Das Tetrahydrofuran wird am Rotationsverdampfer abgezogen. Die Reaktionsmischung wird mit 100 ml Ethanol und einem Überschuß Natriumcarbonat bis zur alkalischen Reaktion versetzt. Zur Oxidation von entstandenem Leukofarbstoff gibt man noch insgesamt 40 g Kaliumferricyanid zu und rührt die Mischung bei Raumtemperatur. Nach beendeter Hydrolyse wird mit Essigsäureethylester extrahiert. Die organische Phase wird eingedampft und der Rückstand an Kieselgel mit dem Laufmittel Essigsäureethylester/Ligroin (1:1) chromatographiert. Man erhält 4,4 g (98 % der Theorie bezogen auf die Nitrosoverbindung) des Farbstoffes. R_{f} (Kieselgel,Methylenchlorid/Essigsäureethylester (1:1) = 0,6
   2.1.3 Zur Reduktion zum Leukofarbstoff wird der Farbstoff in 200 ml Essigsäureethylester gelöst und die Lösung mit ca. 100 ml gesättigter Sodalösung versetzt. Die Lösung wird bis zur Entfärbung unter kräftigem Schütteln mit Natriumdithionit versetzt. Die organische Phase wird abgetrennt, getrocknet und bis auf ein kleines Volumen eingeengt. Man versetzt mit Ligroin und filtriert den ausgefallenen Niederschlag ab, der mit Ligroin gewaschen und anschließend getrocknet wird. Man erhält 4,45 g der Titelverbindung, die für die weitere Verarbeitung rein genug ist. Eine Reinigung ist durch Chromatographie über Kieselgel mit Methylenchlorid/Methanol (98:2) möglich.
      R_{f} (Kieselgel, Essigsäureethylester/Methylenchlorid (1:1)) = 0,6
      R_{f} (Kieselgel, Methylenchlorid/Methanol (95:5)) = 0,3
2.2 (4-Hydroxyphenyl)-pyrazolo-[1,5-a]-pyridin-3-yl)-amin
   2.2.1 2 g Pyrazolo-[1.5-a]-pyridin werden in 6 N Salzsäure (30 ml) gelöst, die Lösung auf 0°C gekühlt und dazu langsam eine Lösung von 6.9 g Natriumnitrit in 30 ml Wasser getrcpft. Nach 1 Stunde ist die Nitrosierung beendet. Man setzt ca. 100 ml Wasser zu und extrahiert mehrfach mit Essigsäureethylester. Die organische Phase wird getrocknet und eingeengt. Man erhält 9.6 g 3-Nitrosopyrazolo-[1.5-a]-pyridin.
   2.2.2 9 g der nach 2.2.1 erhaltenen Nitrosoverbindung werden in eine Lösung von 22 g Zinn (II)-chlorid-Dihydrat in 180 ml konzentrierter Salzsäure eingetragen. Die Reaktionsmischung wird 1 Stunde bei Raumtemperatur gerührt und zur Vervollständigung der Reduktion mit 8 g Zinn (II)-chlorid-Dihydrat in 30 ml konzentrierter Salzsäure versetzt.
      Die Suspension wird auf ca. 150 g Eis gegossen, mit Natriumhydroxid auf pH 12 gestellt und rasch mit Essigsäureethylester extrahiert. Die Essigesterphase wird getrocknet und eingeengt. Der Rückstand wird in ca. 350 ml Ether gelöst und mit etherischer Chlorwasserstoffsäure versetzt. Der ausgefallene Niederschlag wird abfiltriert, mit Ether gewaschen und getrocknet. Man erhält 11.3 g (100 % der Theorie) 3-Amino-pyrazolo-[1,5-a]-pyridin-hydrochlorid mit Fp. 228-232°C R_{f} (Kieselgel, Essigsäureethylester/Methanol 9:1) = 0,52
   2.2.3 Der Leukofarbstoff (4-Hydroxyphenyl)-(pyrazolo[1,5-a]-pyridin-3-yl)-amin wird analog 2.1.2 und 2.1.3 unter Verwendung von 3-Amino-pyrazolo-[1,5-a]-pyridin als Ausgangsmaterial erhalten R_{f}(Kieselgel, Essigsäureethylester/Ether (1:1)) = 0,68.
2.3 (4-Hydroxyphenyl)-(2-methylthio-pyrazolo-[1.5-a]-pyridin-3-yl)amin
   2.3.1 4 g 2-Methylthio-3-nitro-pyrazolo-[1.5-a]-pyridin (Heterocycles 6, 379 (1977), Chem. Pharm. Bull, 25, 1528 (1977)) werden in 200 ml konzentrierter Salzsäure gelöst und mit 20 g Zinn (II)chlorid-dihydrat versetzt. Nach einer Stunde werden nochmals 15 g Zinn (II)chlorid-dihydrat, 15 ml konzentrierter Salzsäure und 200 ml Wasser zugegeben. Nach einer weiteren Stunde Reaktionszeit wird die Mischung auf Eis gegossen, die gelbe Lösung mit Natriumhydroxid alkalisch gestellt und mit Essigsäureethylester exextrahiert. Die organische Phase wird getrocknet und eingedampft. Der Rückstand wird in wenig Ethanol gelöst und mit etherischer Chlorwasserstoffsäure versetzt, um das Hydrochlorid zu bilden.
      Man erhält 3.9 g (95 % der Theorie) 3-Amino-2-methylthio-pyrazolo-[1,5-a]-pyridin-hydrochlorid mit Fp 226°C.
      R_{f} (Kieselgel, Methylenchlorid/tert.-Butylmethylether (2:8) = 0,66
   2.3.2 Der Leukofarbstoff (4-Hydroxyphenyl)-(2-methylthiopyrazolo-[1,5-a]-pyridin-3-yl)-amin wird analog 2.1.2 und 2.1.3 unter Verwendung von 3-Amino-2-methylthio-pyrazolo-[1,5-a]-pyridin erhalten. R_{f} (Kieselgel, Essigsäureethylester/Ligroin (1:1) = 0,47

### Beispiel 3

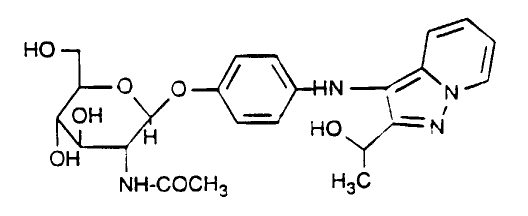

### 4-((R,S-2-(1-Hydroxyethyl)-pyrazolo-[1,5-a]-pyridin-3-yl) -amino) -phenyl-2-acetamido-2-deoxy-β-glucopyranosid

3.1 (4-Hydroxyphenyl)-(2-(1-hydroxyethylpyrazolo-[1,5-a]-pyridin-3-yl)-amin
   25 g N-Aminopyridin-hydrochlorid werden in 250 ml trockenem Dimethylformamid gelöst und unter Rühren mit 17,5 g Kaliumcarbonat versetzt. Anschließend tropft man unter Rühren 20,5 g R,S-2-Hydroxy-5-oxo-butin-3 (J. Chem. Soc. Perkin I 1908 (1976)) zu. Die Mischung erwärmt sich dabei und wird über Nacht stehengelassen. Nach Zugabe von 1,25 l Wasser wird mehrfach mit Essigsäureethylester extrahiert. Die vereinigten Extrakte werden getrocknet und eingedampft. Das verbleibende Öl wird mit etwas Ether verdünnt. Die nach einiger Zeit ausgefallenen Kristalle werden abgesaugt. Man erhält 9,8 g (42 % der Theorie) R,S-3-Acetyl-2-(1-hydroxyethyl)-pyrazolo-[1,5-a]-pyridin (R_{f} (Kieselgel, Methylenchlorid/Essigsäureethylester 1:1 = 0,35), das analog Beispiel 2.1.1 nitrosiert wird. Die Nitrosoverbindung wird in Ethanol suspendiert und mit 2 g Palladium auf Kohle versetzt. Die Mischung wird auf 80°C erhitzt und 2,5 ml Hydrazin-Hydrat werden portionsweise zugegeben. Nach 10 Minuten wird die Palladium-Kohle abfiltriert und das Filtrat eingedampft. Der Rückstand wird in Ethanol gelöst und mit ethanolischer Chlorwasserstoffsäure versetzt. Man erhält 6,8 g R,S-3-Amino-2-(1-hydroxyethyl)-pyrazolo-[1,5-a]-pyridin-hydrochlorid als Kristalle, die 1,8 Mol HC1 enthalten und bei 229-231°C schmelzen R_{f} (Kieselgel, Essigsäureethylester/ Methanol 3:1) = 0,65
   Analog Beispiel 2.1.2 setzt man 3,08 g Phenol mit der erhaltenen Aminoverbindung um und reduziert den blauen Farbstoff analog Beispiel 2.1.3. Das Rohprodukt wird über Kieselgel mit Essigsäureethylester/Methylenchlorid (1:1) als Laufmittel chromatographiert. Man erhält 3,4 g der Titelverbindung. Rf (Kieselgel, Essigsäureethylester/Methylenchlorid 1:1) = 0,2
3.2 Analog Beispiel 1.2 und 1.3 erhält man aus dem in 3.1 erhaltenen Leukofarbstoff und 2-Acetamido-3,4,6-tri-O-acetyl-2-deoxy-α-D-glucosylchlorid die Titelverbindung. R_{f} (Kieselgel, Toluol/Essigsäureethylester/Methanoi (1:1:1)) = 0,25, Fp=165-170° C (Zersetzung)

### Beispiel 4

### Naßchemische Bestimmung von N-Acetyl-β-D-glucosaminidase (β-NAGase)

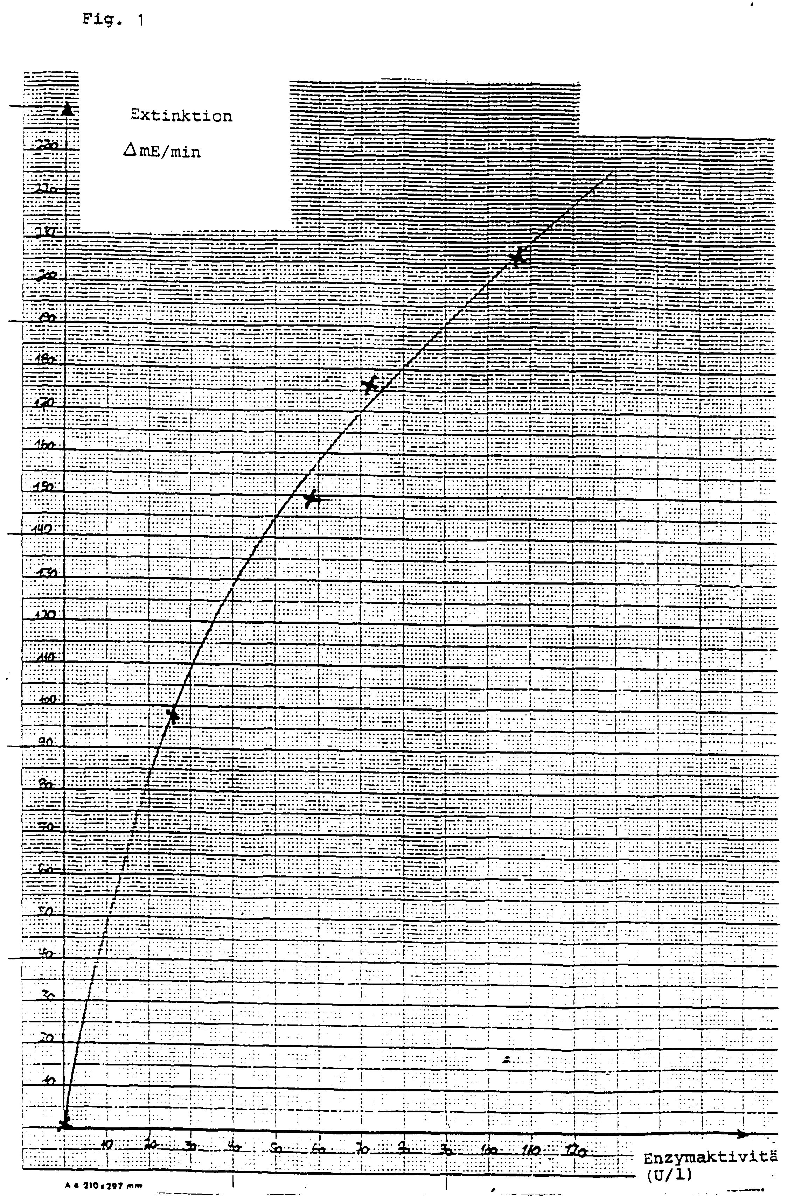

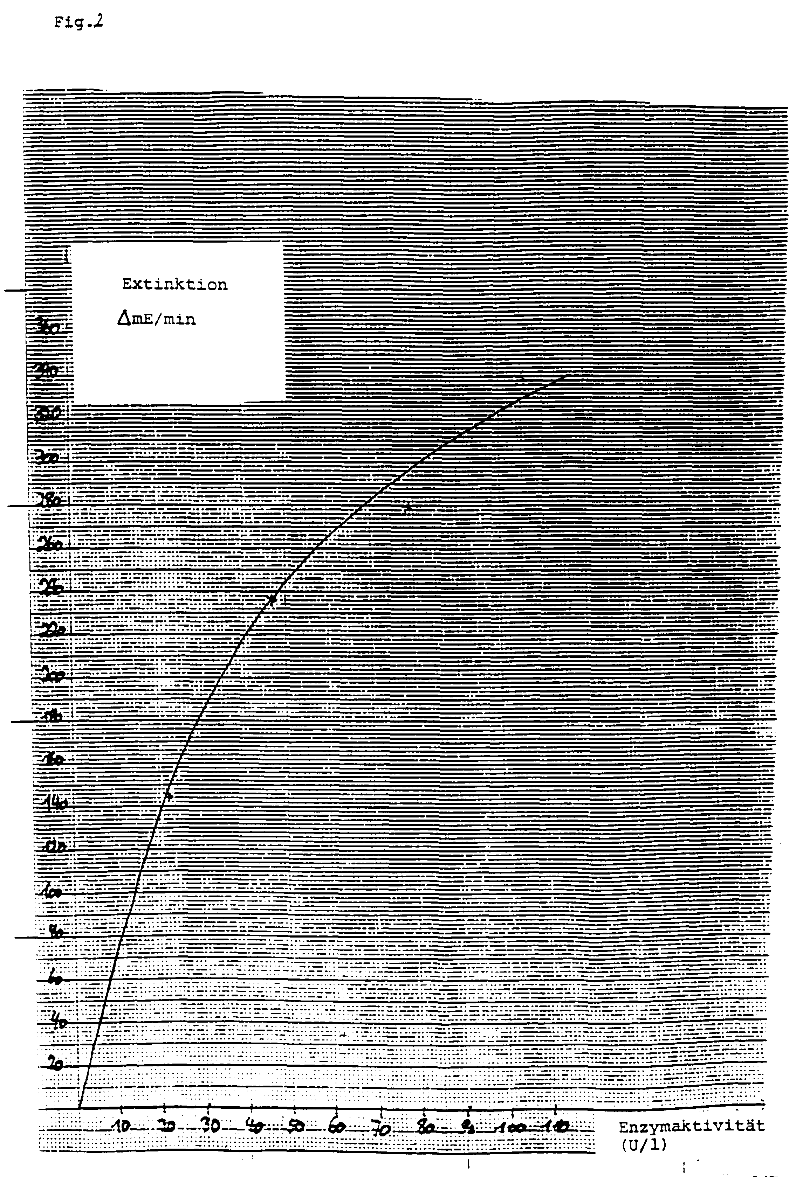

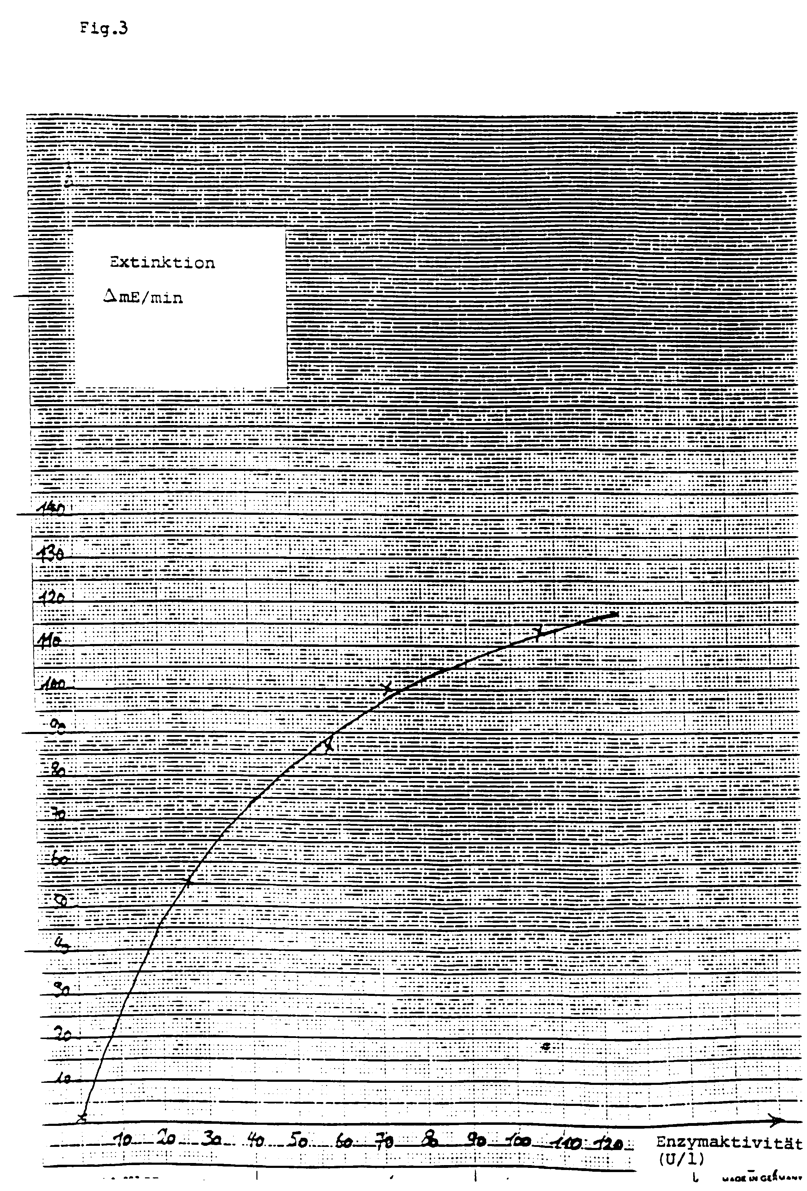

900 µl Lösung von 4 mM Substrat in 200 mM Citratpuffer, pH 5, werden in einer Küvette vorgelegt. Man pipettiert
1. 100 µl einer 220 mM Kaliumjodat-Lösung und
2. 100 µl einer β-NAGase-haltigen Lösung zu,
   mischt und mißt die Kinetik bei einer dem Substrat entsprechenden Wellenlänge. Mit bekannten β-NAGase-haltigen Lösungen lassen sich mit erfindungsgemäßen Substraten Eichkurven der Fig. 1 - 3 erhalten.
4.1 Das Substrat aus Beispiel 2a) ergibt bei λₘₐₓ 560 nm eine Eichkurve zur Bestimmung von β-NAGase gemäß Fig. 1. Bei Anwesenheit von β-NAGase verändert das Substrat seine Farbe von farblos nach blauviolett.
4.2 Das Substrat aus Beispiel 2b) ergibt bei λₘₐₓ 525 nm eine Eichkurve zur Bestimmung von β-NAGase gemäß Fig. 2. Bei Anwesenheit von β-NAGase verändert das Substrat seine Farbe von farblos nach rotviolett.
4.3 Das Substrat aus Beispiel 3 ergibt bei λₘₐₓ 565 nm eine Eichkurve zur Bestimmung von β-NAGase gemäß Fig. 3. Bei Anwesenheit von β-NAGase verändert das Substrat seine Farbe von farblos nach blauviolett.

### Beispiel 5

### Teststreifen zur Bestimmung von β-NAGase

a) Ein Filterpapier der Firma Schleicher & Schüll (23 SL) wird nacheinander mit folgenden Lösungen imprägniert und getrocknet.
   1. Citratpuffer 200 mMol/1,
      pH 5 Kaliumjodat 20 mMol/l
   2. Indikator 10 mMol/l
      in Methanol

   Als Indikator wird eingesetzt:
   a) die Verbindung aus Beispiel 2 a),
   b) die Verbindung aus Beispiel 2 b).

   Taucht man so gefertigte Teststreifen in β-NAGase-haltige Lösung, so erhält man nach ca. 5 Minuten ab ca. 10 U/l β-NAGase (die Aktivität ist bestimmt nach der derzeitigen Referenzmethode mit Sulfophthaleinyl-N-acetyl-β-D-glucosaminid als Enzymsubstrat) eine Verfärbung der Teststreifen von farblos nach blau, wenn die Verbindung aus Beispiel 2 a) eingesetzt wird und von farblos nach rot im Fall der Verwendung der Verbindung aus Beispiel 2 b). Höhere Enzymaktivitäten führen zu intensiverer Verfärbung.
b) Ein vergleichbares Ergebnis wird erzielt, wenn der Teststreifen aus a) so modifiziert wird, daß das Reagenzpapier wie oben, jedoch mit einer Lösung ohne Jodat imprägniert wird. Das Oxidationsmittel wird aus einer 40 mMol/l wässrigen Kaliumjodat-Lösung auf ein Netz aus Nylon (NY75HC der Firma Zürcher Beuteltuchfabrik, Zürich, Schweiz) mit einer Fadendicke von 60 m imprägniert. Aus dem Puffer und Indikator enthaltenden Papier (1), dem das Oxidationsmittel enthaltenden Netz (2), einem Abdecknetz (3) aus dem gleichen Material wie dem Netz (2) und einer steifen Plastikfolie (4) aus Polystyrol wird ein Testträger gemäß Fig. 4 hergestellt. Hierzu werden das Papier (1) und das Netz (2) in 6 mm x 6 mm große Stücke geschnitten und mit einem 12 mm x 6 mm großen Stück Abdecknetz (3) mittels Schmelzkleber (5) auf einem 100 mm x 6 mm großen Stück Plastikfolie (4) befestigt.
   Beim Eintauchen solcher Teststreifen in β-NAGase-haltige Lösungen tritt eine Verfärbung von farblos nach blau für die Verbindung 2 a) und von farblos nach rot für die Verbindung 2 b) auf. Höhere Enzymaktivitäten führen zu intensiverer Verfärbung.

### Beispiel 6

### N-(p-Toluolsulfonyl)-L-alanin-(4-((2-methyl-pyrazolo-[1.5-a]-pyridin-3-yl)-amino)-phenyl)-ester

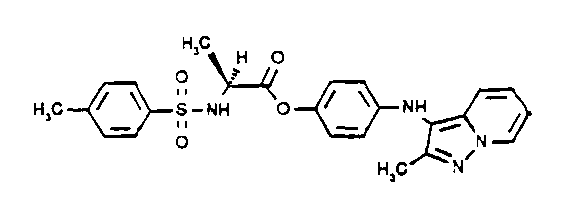

0,48 g des in Beispiel 2.1 erhaltenen Leukofarbstoffs werden in 8 ml Pyridin gelöst und unter Rühren tropfenweise mit einer Lösung von p-Toluoleulfonylchlorid in 12 ml Chloroform versetzt. Nach kurzem Nachrühren wird mit Wasser versetzt und die organische Phase abgetrennt. Die organische Phase wird mit Wasser gut gewaschen, getrocknet und eingeengt. Der Rückstand wird mit dem Laufmittel Wasser/Methanol an dem Adsorberharz HP 20SS (Fa. Mitsubishi) chromatographiert. Die produkthaltigen Fraktionen werden aus Ether/Hexan kristallisiert. Man erhält 0.54 g der Titelverbindung vom Fp. 68-78°C (Zersetzung). R_{f} = 0,7; Kieselgel (Essigsäureethylester/Methylenchlorid 1:1)

### Beispiel 7

Phosphorsäure-mono-(4-((2-methyl-pyrazolo-[1,5-a]-pyridin-3-yl)-amino)phenylester, Pyridiniumsalz

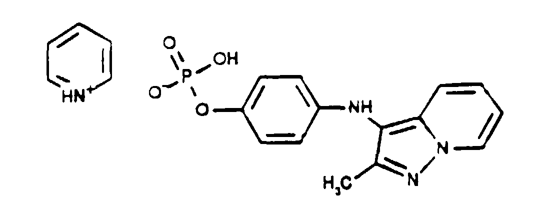

0,35 g des in Beispiel 2.1 erhaltenen Leukofarbstoffes werden in 3 ml Pyridin gelöst. Die Lösung wird auf -15°C gekühlt und unter Rühren tropfenweise mit einer Lösung von 0,15 ml Phosphoroxychlorid in 2,5 ml Pyridin versetzt. Man rührt das Gemisch 30 Minuten bei -15°C und 2 Stunden bei Raumtemperatur, versetzt mit Eis, säuert mit 2 N Schwefelsäure auf pH 2 an und läßt über Nacht im Kühlschrank stehen. Von dem ausgefallenen Niederschlag wird abfiltriert und das Filtrat über eine Säule mit dem Adsorberharz HP 20SS (Fa. Mitsubishi) gegeben. Man eruiert das Produkt mit Wasser. Die produkthaltigen Fraktionen werden vereinigt, eingeengt und der Rückstand aus Methanol/Ether kristallisiert. Man erhält 0.06 g der Titelverbindung vom Fp. 158-161°C (Zers.)

### Beispiel 8

### 4-(2,4-Dimethyl-pyrazolo-[1,5-a]-imidazol-3-yl)-amino)-phenyl-2-acetamido-2-deoxy-β-D-gluco-pyranosid

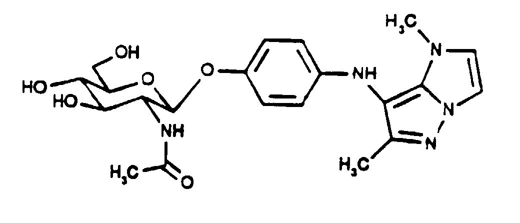

8.1 1,92 g 1,2-Dimethylimidazol werden in 10 ml Methylenchlorid gelöst und unter Eiskühlung mit einer Lösung von O-p-Toluolsulfonyl-hydrcxylamin versetzt, die durch Umsetzung von 15,4 g O-p-Toluolsulfonylacethydroxamsäureethylester mit 118 ml 60proz. wässriger Perchlorsäure erhalten wurde. Das Gemisch wird 3 Stunden bei Raumtemperatur gerührt. Der ausgefallene Niederschlag wird abfiltriert und mit Ether gewaschen. Man erhält 5 g (88 % der Theorie) N-Amino-1,2-dimethylimidazolium-p-toluolsulfonat.
8.2 5 g des in 8.1 erhaltenen Produktes werden mit 5,4 g Natriumacetat und 125 ml Acetanhydrid 1 Stunde auf 140°C (Badtemperatur) erhitzt. Die Reaktionsmischung wird im Vakuum eingedampft und in Wasser aufgenommen. Man stellt den pH-Wert auf ca. 9-10 und extrahiert mit Methylenchlorid.
   Die organische Phase wird getrocknet und eingedampft. Der Rückstand wird an Kieselgel mit Essigsäureethylester chromatographiert. Man erhält 0,45 g (11 % der Theorie) 3-Acetyl-2,4-dimethylpyrazolo-[1,5-a]3-imidazol mit Fp. 165-167°C.
8.3 Die in 10 erhaltene Verbindung wird analog Beispiel 2.1.1 nitrosiert. Man erhält 0,35 g (94 % der Theorie) der entsprechenden 3-Nitrosoverbindung mit Fp. 179-183°C, die in 100 ml verdünnter wässriger Natriumbicarbonatlösung gelöst wird und mit Natriumdithionit reduziert wird. Die Reaktionsmischung wird eingedampft und mit Ethanol digeriert. Die Ethanollösung wird eingeengt und das Produkt durch Zugabe von etherischer Chlorwasserstoffsäure ausgefällt. Man erhält 0,3 g (80 % der Theorie) der Aminoverbindung mit Fp. 199-204°C (Zers.). DC (Kieselgel, Essigsäureethylester/ Aceton/Eisessig/Wasser = 50:25:12,5:12,5): R_{f} = 0,3
8.4
   Analog Beispiel 2.1.2 setzt man die oben erhaltene Aminoverbindung mit K₃Fe(CN)₆ und Phenol um und reduziert den erhaltenen Farbstoff analog Bsp. 2.1.3. Rf des erhaltenen Leukofarbstoffes: Kieselgel, Toluol/Essigsäureethylester/ Methanol 2:1:1 = 0,5.
8.5
   0,46 g des oben erhaltenen Leukofarbstoffes werden in 40 ml Methylenchlorid gelöst und unter Eiskühlung zunächst mit 0,65 ml Di-isopropylethylamin und dann tropfenweise mit 0,51 ml Trifluoressigsäureanhydrid versetzt. Nach 1 Stunde gibt man nochmals nacheinander 0,35 ml Diisopropylethylamin und 0,25 ml Trifluoressigsäureanhydrid zu. Nach 30 minütigem Rühren wird die Mischung eingedampft und der Rückstand über Kieselgel mit Methylenchlorid/Essigsäureethylester 1:1 chromatographiert. Man erhält.O,4 g N-(2,4-Dimethyl-pyrazolo[1,5-a]-imidazol-3-yl)-N-(4-hydroxyphenyl)-trifluoracetamid. R_{f} = 0.3 (Kieselgel, Methylenchlorid/Essigsäureethylester 1:1)
8.6. Das oben erhaltene Phenolderivat wird analog Beispiel 1.2 mit 2-Acetamido-3,4,6-tri-O-acetyl-2-deoxy-α-D-glucosylchlorid glykosidiert. Zur Abspaltung der Schutzgruppen einschließlich der Trifluoracetylgruppe wird das durch Chromatographie an Kieselgel (Methylenchlorid/Essigsäureethylester 1:1) gereinigte Produkt in Methanol gelöst und die Lösung mit einem großen Überschuß an wasserfreiem Natriumcarbonat versetzt. Man rührt das Gemisch 1,5 Stunden bei 45°C, saugt die Salze ab, wäscht den Rückstand mit etwas Methanol und engt das Filtrat bis auf ca. 20 ml ein. Diese Lösung wird auf eine Säule mit dem Adsorberharz HP 20SS (Fa. Mitsubishi) gegeben und mit einem Stufengradienten von Methanol/Wasser 1:9 bis 1:1 eruiert. Man erhält die Titelverbindung vom Fp. 112-115°C (Zers.), R_{f} = 0.2 (Kieselgel, Toluol/Essigsäureethylester/Methanol 1:1:1.

### Beispiel 9

### 4-((2,4-Dimethyl-pyrazolo-[1,5-a]-imidazol-3-yl)-N-trifluoracetyl)amino)phenyl-2-acetamido-2-deoxy-β-D-glucopyranosid

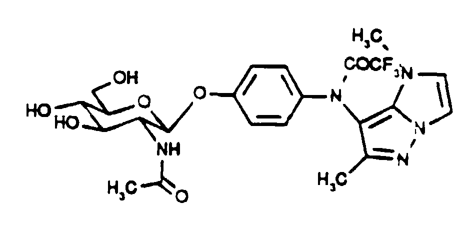

Führt man die Schutzgruppenabspaltung des in Beispiel 8.6 erhaltenen Glykosids mit Natriumbicarbonat in Methanol analog Beispiel 1.3 durch, werden nur die Acetylgruppen abgespalten und man erhält nach chromatographischer Reinigung analog Beispiel 10.6 die Titelverbindung vom Fp. 223-226°C (Zersetzung) R_{f} = 0,21 (Kieselgel, Toluol/Essigsäureethylester/Methanol 1:1:1).

### Beispiel 10

Analog Beispiel 8.5 und 8.6 erhält man ausgehend von dem entsprechenden Leukofarbstoff und 2-Acetamido-3,4,6-tri-O-acetyl-2-deoxy-α-D-glucosylchlorid die in der Tabelle 2 aufgeführten Verbindungen.

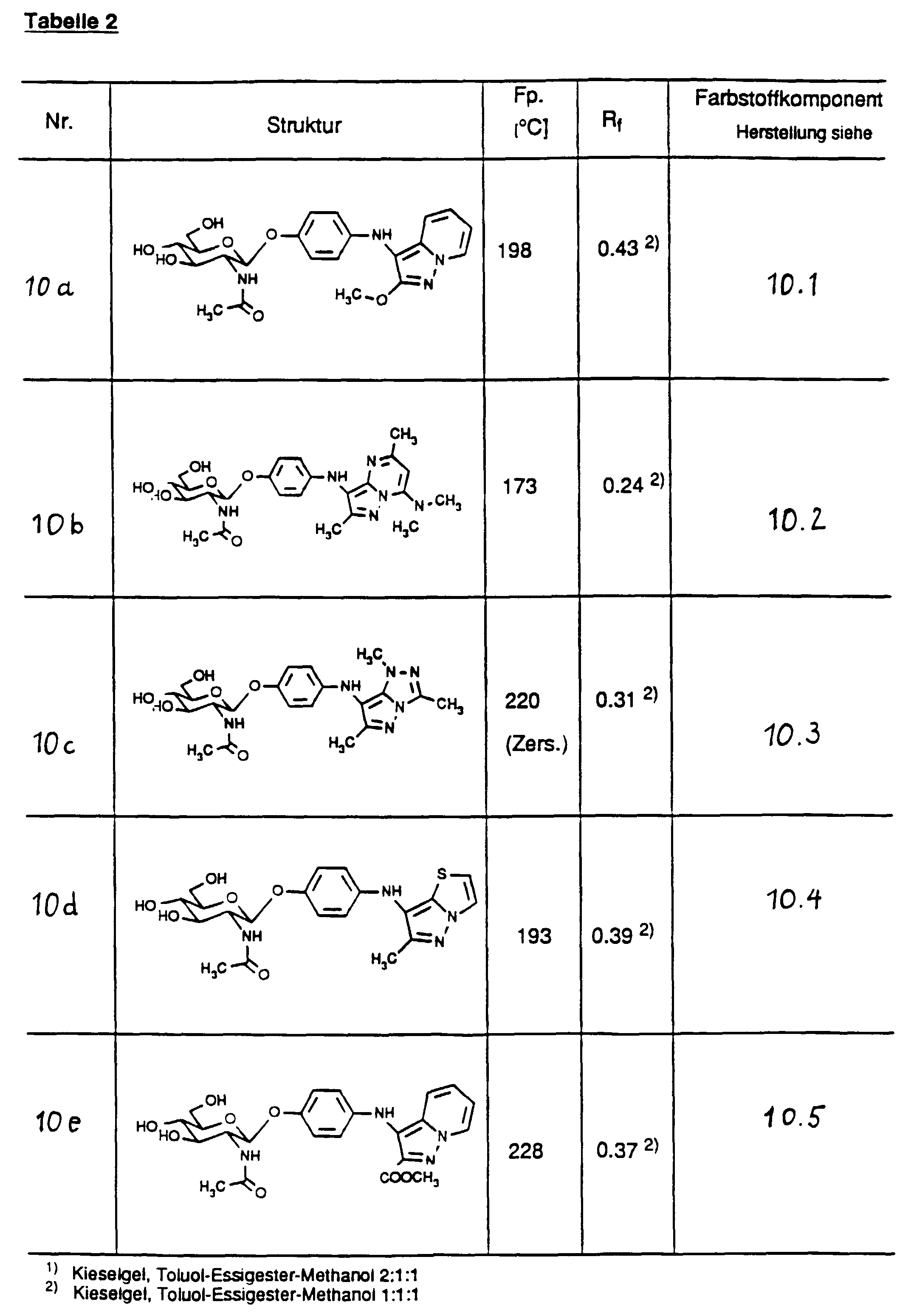

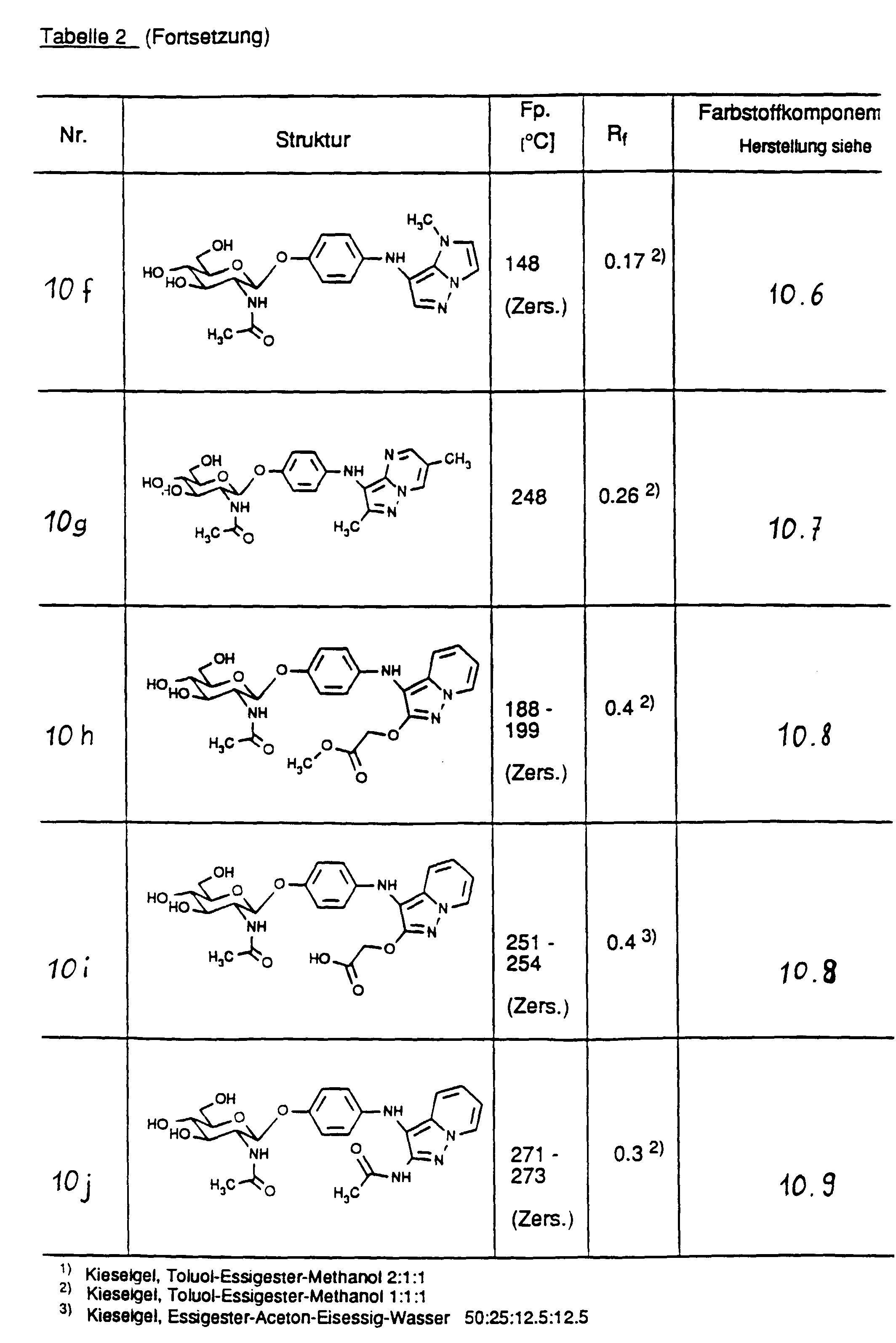

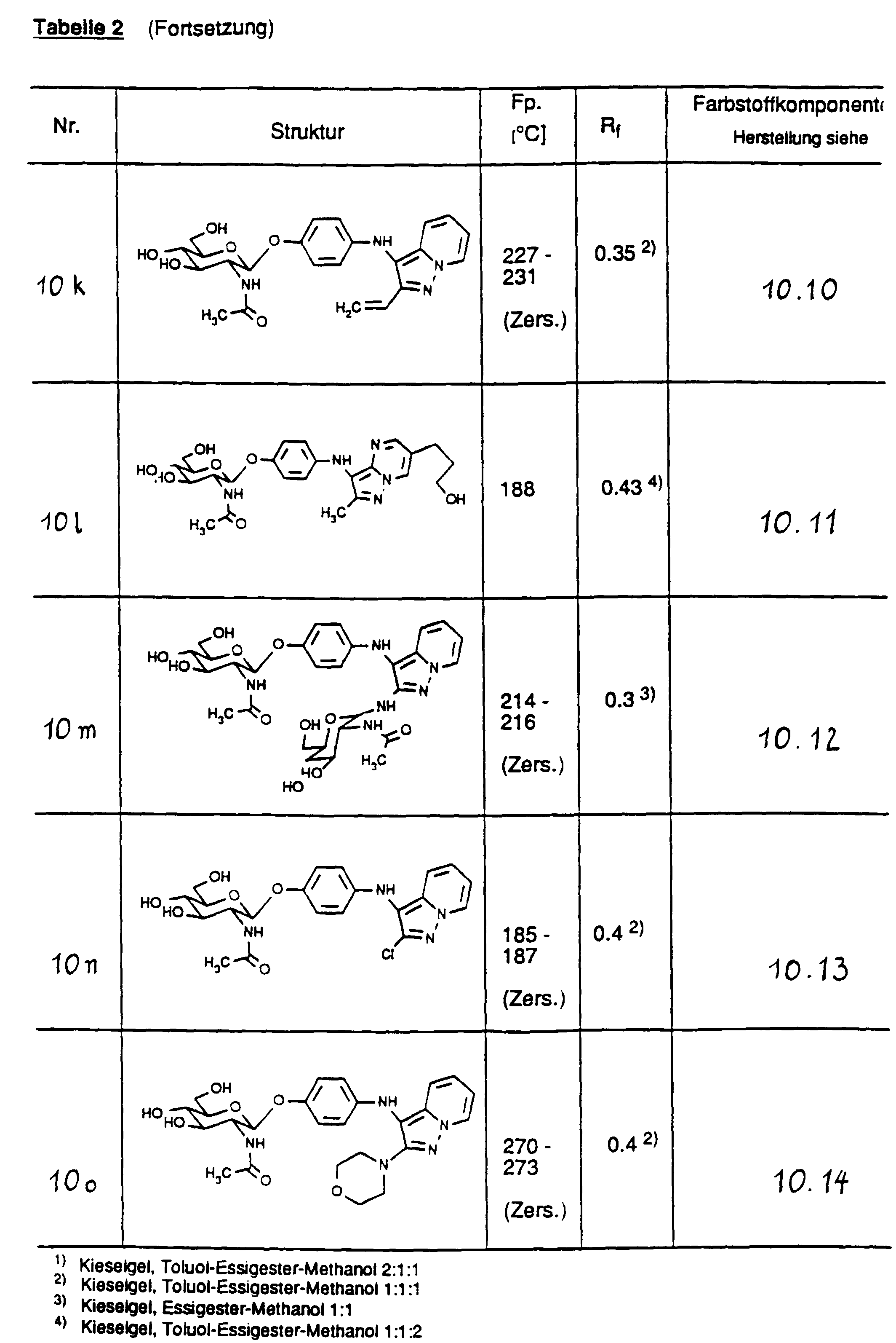

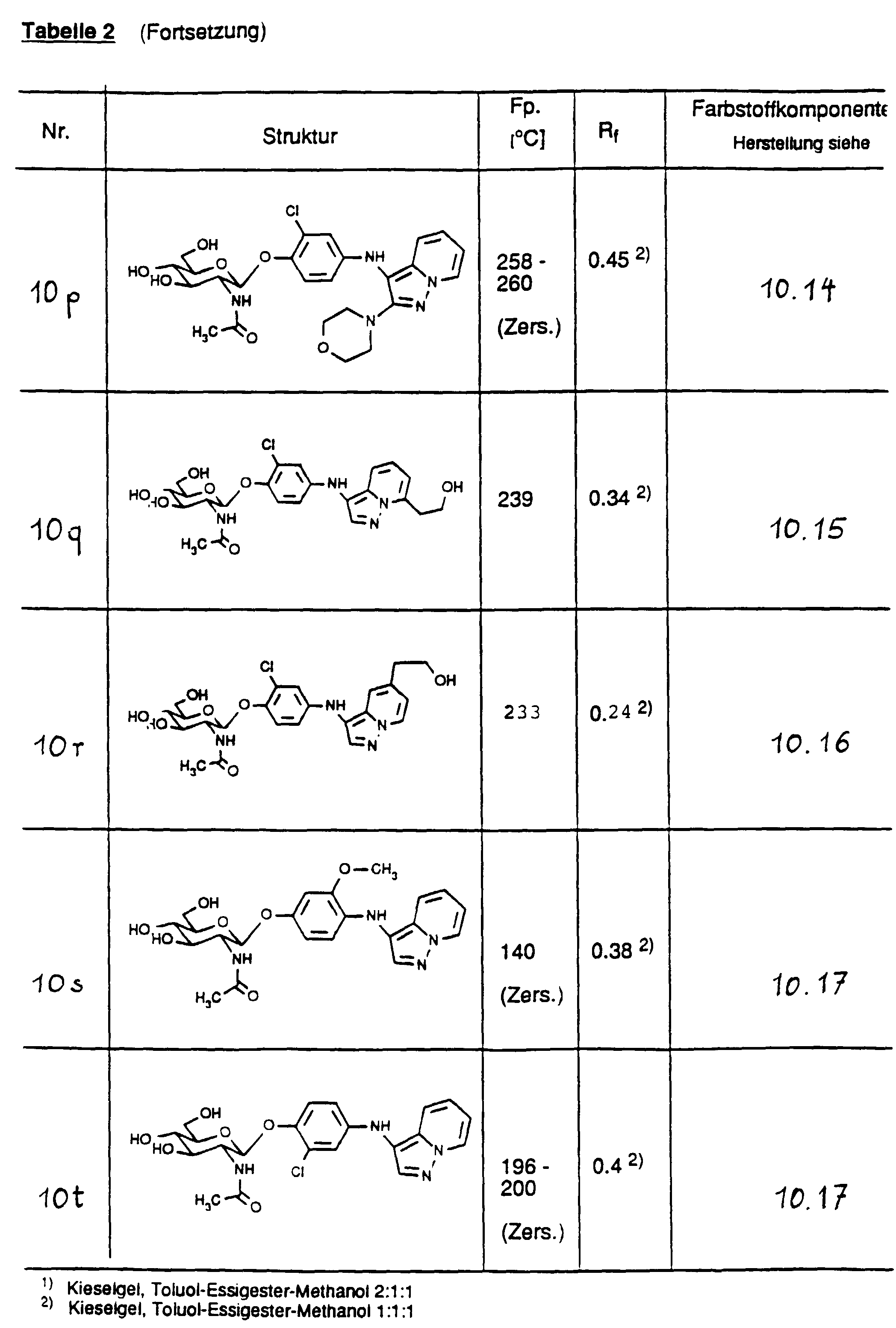

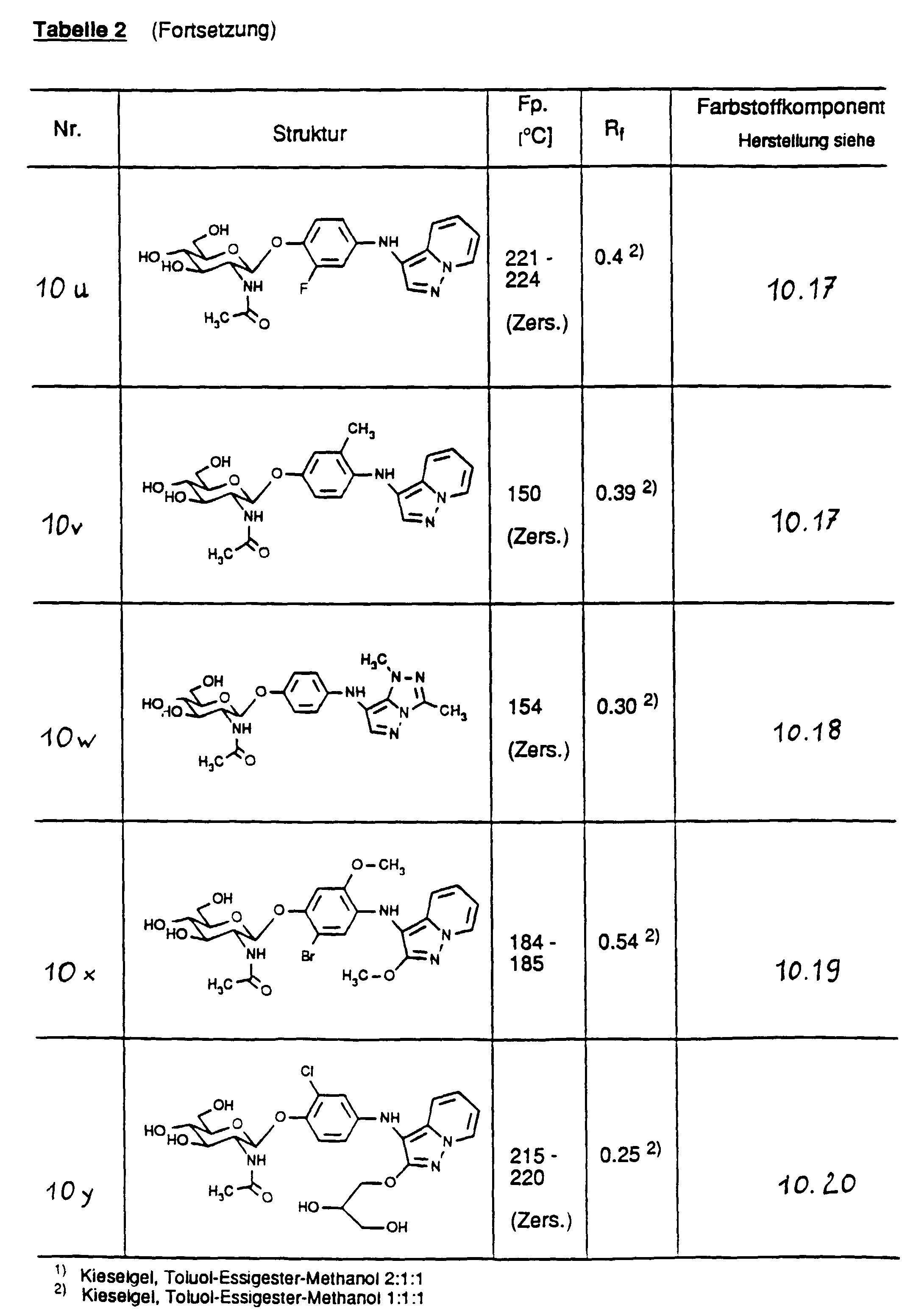

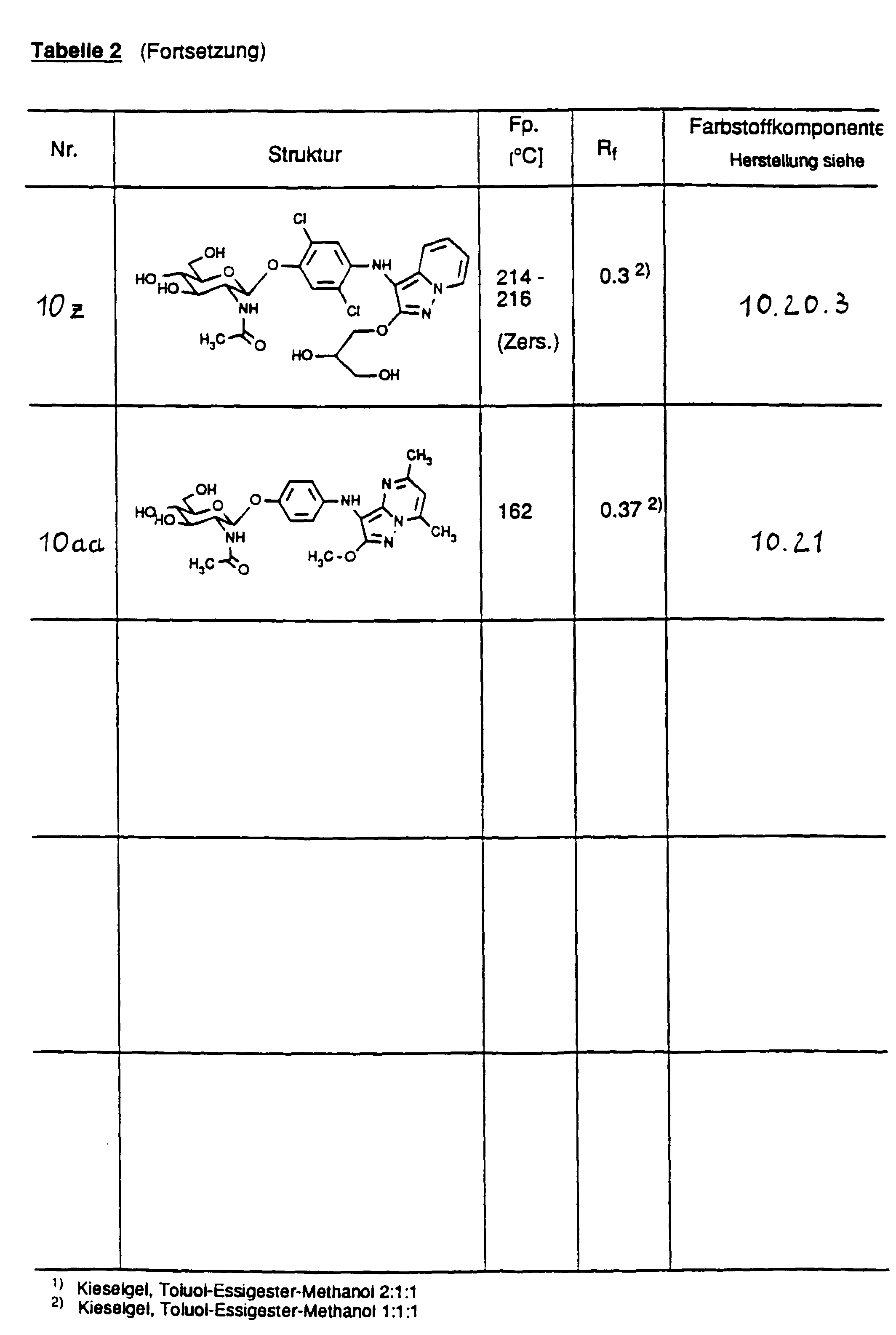

### Herstellung der Leukofarbstoffkomponenten

10.1.
   (4-Hydroxyphenyl)-(2-ethoxy-pyrazolo-[1,5-a]-pyridin-3-yl)-amin
10.1.1
   3-Amino-2-methoxy-pyrazolo-[1.5-a]-pyridin-hydrochlorid
   1,48 g 2-Methoxy-pyrazolo-[1,5-a]-pyridin (Bull. Chem. Soc. Jap. 49, 1980 (1976) werden unter Eiskühlung in 20 ml konzentrierter Salpetersäure gelöst und tropfenweise mit 10,5 ml rauchender Salpetersäure versetzt. Man rührt das Gemisch 30 Minuten im Eisbad und 1 Stunde bei Raumtemperatur. Die Reaktionslösung wird auf Eis gegossen, der ausgefallene Niederschlag abgesaugt und mit Wasser gewaschen. Man erhält 1 g (52 % der Theorie) 2-Methoxy-3-nitro-pyrazolo-[1,5-a]-pyridin (Fp. 213-216°C).
   0,8 g der oben erhaltenen Nitroverbindung werden in 80 ml 2 N Salzsäure suspendiert und unter heftigem Rühren mit Zinkstaub versetzt. Nach mehrmaliger Zugabe von Zinkstaub erhält man nach ca. 1 Stunde eine klare Lösung über einem Bodensatz von überschüssigem Zink. Dieser wird abfiltriert und das Filtrat mit Natriumhydroxid auf pH 7 gestellt. Die Mischung wird mit Essigsäureethylester extrahiert. Die organische Phase wird eingedampft und das verbleibende 01 mit Ethanol über eine kurze Schicht Kieselgel filtriert. Das Eluat wird eingedampft, der Rückstand in Ether gelöst und mit etherischer Salzsäure versetzt. Der ausgefallende Niederschlag wird abgesaugt und nochmals aus Isopropanol umkristallisiert. Man erhält 0.33 g (42 % der Theorie) der Titelverbindung (10.1.1) mit Fp. 238-241°C. DC (Kieselgel, Aceton/Methylenchlorid/ Eisessig 50:45:5): R_{f} = 0,6.
10.1.2
   Analog Beispiel 2.1.2 erhält man aus der oben erhaltenen Aminoverbindung durch oxidative Kupplung mit Phenol den Farbstoff, der analog Beispiel 2.1.3 reduziert und analog Beispiel 8.5 trifluoracetyliert wird.
10.2
   (4-Hydroxyphenyl)-(2,5-dimethyl-7-{dimethylamino}-pyrazolo-[1,5-a]-pyrimidin-3-yl)-amin
10.2.1
   3-Amino-2,5-dimethyl-7-(dimethylamino)-pyrazolo-[1,5-a]-pyrimidin-hydrochlorid
   1,6 g 2,5-Dimethyl-7-hydroxy-pyrazolo-[1,5-a]-pyrimidin werden mit 16,6 ml Phosphoroxychlorid und 0,8 ml N,N-Dimethylanilin 40 Minuten unter Rückfluß erhitzt. Das überschüssige Phosphoroxychlorid wird abdestilliert und der Rückstand auf Eis gegossen. Man extrahiert mit Methylenchlorid, wäscht die organische Phase mit Na₂CO₃-Lösung, trocknet und engt ein. Der Rückstand wird in 28 ml Ethanol gelöst und mit 2 g einer 40proz. Lösung von Dimethylamin in Wasser versetzt. Die Mischung wird 2,5 Stunden bei Raumtemperatur gerührt, eingedampft und der Rückstand über Kieselgel mit Essigsäureethylester chromatographiert. Man erhält 0,86 g (46 % der Theorie) 2,5-Dimethyl-7-(N,N-dimethylamino)-pyrazolo-[1,5-a]-pyrimidin das analog Beispiel 4.3.1 nitrosiert wird.
   Die erhaltene Nitrosoverbindung (1 g) wird in Ethanol (20 ml) gelöst, mit 0,16 g Palladium/Kohle versetzt und in der Siedehitze mit 0,4 ml Hydrazinhydrat versetzt. Man kocht noch 15 Minuten unter Rückfluß, saugt ab und engt das Filtrat ein. Der Rückstand wird in wenig Ethanol gelöst und mit etherischer HCl versetzt, um das Hydrochlorid auszufällen. Man erhält 0,8 g 3-Amino-2,5-dimethyl-7-(dimethyl-amino-pyrazolo-[1,5-a]-pyrimidin-hydrochlorid R_{f} = 0,54 (Kieselgel, Chloroform/Methanol/ Methylethylketon/Eisessig/Wasser 75:35:25:5:8).
10.2.2
   6,94 g Phenol werden in 124 ml Pyridin und 743 ml Wasser gelöst. Dazu gibt man eine Lösung von 11,4 g der oben erhaltenen Aminoverbindung in 248 ml Wasser hinzu. Schließlich versetzt man mit einer Lösung von 0,33 g Peroxidase (aus Meerrettich, Boehringer Mannheim, Mannheim, Deutschland) in 120 ml Wasser und tropft sofort 11,23 ml Wasserstoffperoxid (30proz.) zu. Nach 30 minütigem Rühren wird der Niederschlag abgesaugt. Man erhält 12,9 g blaues N-(2,5-Dimethyl-6-dimethylaminopyrazolo-[1,5-a]-pyrimidin-3-yl)-chinonimin. R_{f} = 0,43 (Kieselgel, Essigsäureethylester/Methylenchlorid 95:5). Der Farbstoff wird analog Beispiel 2.1.3 reduziert und analog Beispiel 8.5 trifluoracetyliert. Fällt der Farbstoff nicht aus, so wird mit Essigsäureethylester extrahiert. Unter Umständen empfiehlt sich die Weiterverarbeitung zum Leukofarbstoff durch Zugabe von Natriumdithionit und Sodalösung analog Beispiel 2.1.3.
10.3
   (4-Hydroxyphenyl)-(2,4,6-trimethyl-pyrazolo-[3,2-c]-s-triazol-3-yl)-amin
10.3.1
   3-Amino-2,4,6-trimethyl-pyrazolo-[3,2-c]-s-triazolhydrochlorid
   Ausgehend von 4-Ethoxycarbonyl-3-methyl-pyrazol-5-yl-hydrazin (Chem. Ber. 89, 2552 (1956)) stellt man wie in DE-A-1810462 (Bsp. 6) beschrieben 2,6-Dimethyl-4H-pyrazolo-[3,2-c]-S-triazol-3-carbonsäureethylester her.
   0,4 g dieses Esters werden in 15 ml trockenem Dimethylformamid gelöst und mit 0,46 g p-Toluolsulfonsäuremethylester versetzt. In das Gemisch trägt man portionsweise 0,11 g Natriumhydrid (55proz.) ein und rührt anschließend 1 Stunde bei Raumtemperatur. Die Reaktionsmischung wird auf Eis gegossen und 5 g Kochsalz zugesetzt. Man extrahiert mehrfach mit Essigsäureethylester. Der Extrakt wird getrocknet und eingedampft. Das verbleibende Öl wird durch Chromatographie an Kieselgel mit Essigsäureethylester/Ligroin als Laufmittel gereinigt.
   Als Zwischenprodukt erhält man so 2,4,6-Trimethylpyrazolo-[3,2-c]-s-triazol-3-carbonsäureethylester (R_{f} = 0,52; Kieselgel, Essigsäureethylester:Ligroin 1:1), der durch Kochen mit konzentrierter Salzsäure verseift und gleichzeitig decarboxyliert wird. Man erhält 2,4,6-Trimethyl-pyrazolo-3,2-c]-s-triazol (R_{f} = 0.24, Kieselgel, Essigsäureethylester/Ligroin 1:1) als schwach gelbliches Öl, das nach einiger Zeit kristallisiert.
   1,83 g des Heterocyclus werden in 40 ml Eisessig gelöst und mit 1 g Natriumacetat und 3,3 g p-Methoxybenzoldiazoniumtetrafluoroborat versetzt. Man rührt das Gemisch bei 40°C und setzt nach 3 Stunden zur Vervollständigung der Reaktion nochmals 0,2 g Natriumacetat und 0,66 g des Diazoniumsalzes zu. Nach weiteren 3 Stunden bei 40°C wird die Mischung auf Eis gegossen. Man extrahiert mit Essigsäureethylester und reinigt das Rohprodukt durch Chromatographie an Kieselgel mit Essigsäureethylester/ Ligroin 1:1. Man erhält 3,7 g der zitronengelben Azoverbindung.
   Die Azoverbindung (3,1 g) wird in 50 ml Eisessig gelöst und unter leichter Kühlung mit 5 g Zinkpulver versetzt. Nach 1 Stunde wird die Mischung filtriert und das Filtrat eingeengt. Zur Reinigung wird das Rohprodukt in Dioxan gelöst und mit 3,9 g tert.Butyldicarbonat versetzt. Nach 2 Stunden Reaktionszeit wird das Gemisch eingedampft und der Rückstand an Kieselgel mit Ligroin/Aceton (7:3) chromatographiert. Man erhält 1,5 g der entsprechenden N-tert.-Butoxycarbonylverbindung, die zur Abspaltung der Amino-Schutzgruppe in 25 ml ethanolischer Salzsäure gelöst wird. Die Lösung wird nach 1 Stunde bei Raumtemperatur eingeengt und der Rückstand aus Methanol/Ether umkristallisiert. Man erhält 1,6 g der Titelverbindung (10.2.1) vom Fp. 240°C. R_{f} = 0,5 (Kieselgel; Essigsäureethylester/Aceton/Eisessig/Wasser 50:25:12,5:12,5)
10.3.2
   Die oben erhaltene Aminoverbindung wird analog Beispielen 2.1.2 und 2.1.3 mit Phenol zum Farbstoff gekuppelt, reduziert und der Leukofarbstoff analog Beispiel 8.5 trifluoracetyliert.
10.4
   (4-Hyroxyphenyl)-(2-methyl-pyrazolo-[3,2-b]-thiazol-3-yl)-amin
   Analog den Beispielen 2.1.1, 2.1.2 und 2.1.3 erhält man den Leukofarbstoff ausgehend von 3-Acetyl-2-methylpyrazolo-[3,2-b]-thiazol (Chem. Pharm. Bull 22, 482 (1974)) und trifluoracetyliert analog Beispiel 8.5.
10.5
   (4-Hydroxyphenyl)-(2-methoxycarbonyl-pyrazolo-[1,5-a]-pyridin-3-yl)-amin
   Analog den Beispielen 2.1.1 (Nitrosierung); 10.1 (Reduktion mit Zn/HCl); 10.2.2 (Oxidative Kupplung mit Phenol), und 2.1.3 (Reduktion zum Leukofarbstoff) erhält man ausgehend von 2-Pyrazolo-[1,5-a]-pyridin-carbonsäureethylester (J. Het. Chem. 18, 1149 (1981) die Leukofarbstoffkomponente, die analog Beispiel 8.5 trifluoracetyliert wird.
   Bei der Abspaltung der Acetyl-Schutzgruppen im letzten Reaktionsschritt wird der Ethylester in den Methylester umgeestert.
10.6
   (4-Hydroxyphenyl)-(4-methyl-pyrazolo-[1,5-a]-imidazol-3-yl)-amin
10.6.1.
   3-Amino-4-methyl-pyrazolo-[1.5-a]-imidazol-hydrochlorid
   Analog dem in "Organikum" (VEB Deutscher Verlag der Wissenschaften, Berlin) auf S. 514/515 beschriebenen Verfahren setzt man 65 g Ethoxymethylencyanessigsäureethylester mit 55 g 2,2-Diethoxyethylhydrazin um und erhält 100,2 g 5-Amino-1-(2,2-diethoxyethyl)-pyrazol-4-carbonsäure, die in 4 l Ethanol gelöst und mit 2 l 20proz. Schwefelsäure versetzt wird.
   Man erhitzt 3 Stunden unter Rückfluß und neutralisiert durch Zugabe von festem Natriumbicarbonat. Von ausgefallenem Salz wird abfiltriert und das Filtrat eingeengt. Der Rückstand wird mehrfach mit siedendem Methylenchlorid ausgezogen. Die Filtrate werden vereinigt. Man erhält 58,2 g (86 %) Pyrazolo-[1,5-a]-imidazol-3-carbonsäure-ethylester, vom Fp. 126-127°C. (R_{f} = 0,64; Essigsäureethylester, Kieselgel)
   18,7 g des oben erhaltenen Carbonsäureesters werden in 190 ml Dimethylformamid gelöst und mit 17 g p-Toluolsulfonsäuremethylester versetzt. Unter Rühren werden 3,98 g Natriumhydrid (55proz.) portionsweise eingetragen. Man rührt 30 Minuten bei Raumtemperatur, gießt die Mischung auf Eis/Wasser und extrahiert mit Essigsäureethylester. Man erhält 21,9 g (100 % der Theorie) des 4-Methylpyrazolo-[1,5-a]-imidazol-3-carbonsäureethylesters, der durch Kochen in 600 ml konzentrierter Salzsäure verseift wird. Gleichzeitig decarboxyliert die entstehende Carbonsäure und man erhält 16,7 g (83 % der Theorie) 4-Methylpyrazolo-[1,5-a]-imidazol-dihydrochlorid (R_{f} = 0,39; Kieselgel, Methylenchlorid mit 5 % Methanol).
   12,8 g Anilin werden in einer Lösung von 12,8 ml konzentrierter Schwefelsäure in 64 ml Wasser durch Zugabe einer Lösung von 9,5 g Natriumnitrit in 42 ml Wasser diazotiert. Die Lösung wird durch Zugabe von NaOH auf pH 5 gestellt und bei 5-0° C tropfenweise mit einer Lösung von 16,5 g des oben erhaltenen 4-Methyl-pyrazolo-[1,5a]-imidazols in 165 ml Wasser und 14 ml Eisessig versetzt. Man rührt 1 Stunde bei 5-10° C und 3 Stunden bei Raumtemperatur und saugt dann den ausgefallenen Niederschlag ab. Man erhält 12 g 4-Methyl-3-phenylazo-pyrazolo-[1,5-a]-imidazol, das analog Beispiel 10.3 mit Natriumdithionit reduziert und gereinigt wird. Man erhält 3,3 g 3-Amino-4-methyl-pyrazolo-[1,5-a]-imidazol-hydrochlorid. Fp. ab 210°C (Zers.) R_{f} = 0,23; Kieselgel, Essigsäureethylester/Aceton/Eisessig/Wasser 50:25:12,5:12,5).
10.6.2
   Die Weiterverarbeitung zur Leukofarbstoffkomponente erfolgt analog Beispiel 10.3.2.
10.7
   (4-Hydroxyphenyl)-(2,6-dimethyl-pyrazolo-[1,5-a]-pyrimidin-3-yl)-amin
10.7.1
   3-Amino-2,6-dimethyl-pyrazolo-[1.5-a]-pyrimidin-hydrochlorid
   3,2 g 2,6-Dimethyl-3-nitro-pyrazolo-[1,5-a]-pyrimidin (Synthesis 673 (1982) werden in 320 ml Ethanol gelöst und mit 320 ml 5proz. Natriumbicarbonatlösung in Wasser versetzt. Zu diesem Gemisch gibt man unter Rühren und Kühlen portionsweise 14,2 g Natriumdithionit zu, bis das Dünnschichtchromatogramm die Abwesenheit von Ausgangsmaterial zeigt. Man engt das Reaktionsgemisch etwas ein und extrahiert 3 mal mit Essigsäureethylester. Die organische Phase wird getrocknet und eingedampft. Der Rückstand wird in wenig Ethanol gelöst und mit einer äquimolaren Menge Chlorwasserstoff in Ether versetzt. Die ausgefallenen Kristalle werden abgesaugt. Man erhält 2,9 g (80 % der Theorie) der Titelverbindung (10.7.1) mit Fp. 224 °C (Zersetzung). DC (Kieselgel, Chloroform/Methanol/Methylethylketon/Eisessig/Wasser (75:35:25:5:8): R_{f} = 0,73.
10.7.2
   Die Leukofarbstoffkomponente erhält man durch oxidative Kupplung mit Phenol analog Beispiel 2.1.2 und Reduktion analog Beispiel 2.1.3 Trifluoracetylierung erfolgt analog Beispiel 8.5.
10.8
   (4-Hydroxyphenyl)-(2-ethoxycarbonylmethyloxy-pyrazolo[1, 5-a]-pyridin-3-yl)-amin
   Analog Beispiel 10.1 erhält man ausgehend von 2-Ethoxycarbonylmethyl-oxy-pyrazolo-[1,5-a]-pyridin, das man analog der 2-Methoxyverbindung (Bull. Chem. Soc. Jap. 49, 1980 (1976) durch Alkylierung des 2-Hydroxyheterocyclus erhält, die N-trifluoracetylierte Leukofarbstoffkomponente. Bei der Abspaltung der Acetylschutzgruppen auf der letzten Reaktionsstufe wird ein Teil des Carbonsäureesters verseift. Durch Chromatographie über das Adsorberharz HP 20SS (Fa. Mitsubishi) lassen sich Ester und Säure glatt trennen (Laufmittel: Methanol/Wasser).
10.9
   (4-Hydroxyphenyl)-(2-acetamido-pyrazolo-[1,5-a]-pyridin-3-yl)-amin
   4 g 2-Acetamido-pyrazolo-[1,5-a]-pyridin (Chem. Pharm. Bull. 21, 2146 (1973)) werden analog Beispiel 2.1.1 nitrosiert. Die Nitrosoverbindung wird analog Beispiel 3.1 mit Palladium/Kohle/Hydrazinhydrat reduziert. Man erhält 3,9 g (67 % der Theorie) 2-Acetamido-3-amino-pyrazolo-[1,5-a]-pyridin-hydrochlorid vom Fp. 255-259° C (Zersetzung). R_{f} = 0,5; Kieselgel, Essigsäureethylester/Aceton/Eisessig/Wasser = 50:25:12,5:12,5
   Die Aminoverbindung wird analog Beispiel 2.1.2 mit Phenol zum Farbstoff gekuppelt, der analog den Beispielen 2.1.3 reduziert und 8.5 N-trifluoracetyliert wird.
10.10
   (4-Hydroxyphenyl-(2-vinyl-pyrazolo-[1,5-a]-pyridin-3-yl)-amin
   25 g R,S-3-Acetyl-2-(1-hydroxyethyl)-pyrazolo-[1,5-a]-pyridin (aus Beispiel 3.1) werden mit 50 ml konzentrierter Schwefelsäure versetzt und bei 95°C Badtemperatur 2 Stunden erhitzt. Die Mischung wird auf viel Eis gegossen, mit NaOH alkalisch gestellt und mit Essigsäureethylester extrahiert. Das Rohprodukt wird über Kieselgel mit Ligroin/Essigsäureethylester (95:5 bis 90:10) chromatographiert. Man erhält 3,4 g 2-Vinyl-pyrazolo-[1,5-a]-pyridin (R_{f} = 0,6; Kieselgel, Ligroin/Essig- säureethyl-ester 3:1),-das analog Beispiel 10.3.1 mit Phenyldiazoniumsalz umgesetzt, reduziert und gereinigt wird. Man erhält so das entsprechende Aminopyrazol. R_{f} = 0,65; Kieselgel, Ligroin/Aceton/Eisessig 50:45:5)
   Die Aminoverbindung wird analog Beispiel 10.2.2 mit Phenol zum Farbstoff gekuppelt, der analog den Beispielen 2.1.3 reduziert und 8.5 N-trifluoracetyliert wird.
10.11
   4-Hydroxyphenyl-((6-(3-acetoxypropyl)-2-methylpyrazolo-[1,5-a]-pyrimidin-3-yl)-amin
10.11.1
   6-(3-Acetoxypropyl)-2-methyl-pyrazolo-[1.5-a]-pyrimidin
   Analog Beispiel 10.7.1 erhält man durch Reduktion von 6-(3-Acetoxy-propyl)-2-methyl-3-nitro-pyrazolo-[1,5-a]-pyrimidin (Synthesis 673 (1982)) die Titelverbindung (10.11.1). R_{f} = 0,21 (Kieselgel, Methylenchlorid/Methanol 90:1)
10.11.2
   Die Aminoverbindung wird analog Beispiel 2.1.2 mit Phenol zum Farbstoff gekuppelt, der analog den Beispielen 2.1.3 reduziert und 8.5 N-trifluoracetyliert wird.
10.12
   4-Hydroxyphenyl-(2,3-diamino-pyrazolo-[1,5-a]-pyridin-3-yl)-amin
   2,66 g 2 Amino-pyrazolo-[1,5-a]-pyridin (Chem. Pharm. Bull. 21, 2146 (1973)) werden analog Beispiel 10.3.1 mit p-Methoxybenzoldiazoniumsalz umgesetzt, die entstandene Azoverbindung mit Natriumdithionit reduziert und das Rohprodukt gereinigt. Man erhält 2.3-Diamino-pyrazolo[1,5-a]-pyridin vom FP. 190°C. R_{f} = 0,6; Kieselgel, Ligroin/ Aceton/Eisessig 60:40:1
   Die Diaminoverbindung wird analog dem Beispiel 2.1.2 mit Phenol zum Farbstoff gekuppelt, der analog den Beispielen 2.1.3 reduziert und 8.5 N-trifluoracetyliert wird.
   Bei der anschließenden Glycosidierung wird der Leukofarbstoff sowohl an der phenolischen OH-Gruppe als auch an der 2-Aminogruppe glycosidiert.
10.13
   4-Hydroxyphenyl-(2-chlor-pyrazolo-[1,5-a]-pyridin-3-yl)amin
10.13.1.
   3-Amino-2-chlor-pyrazolo-[1,5-a]-pyridin-hydrochlorid
   2-Amino-pyrazolo-[1,5-a]-pyridin (Chem. Pharm. Bull. 21, 2146 (1973)) wird analog Beispiel 10.3.1 mit p-Methoxybenzoldiazoniumsalz umgesetzt. 0,53 g der erhaltenen 3-Azoverbindung werden in 10 ml 6 N Salzsäure suspendiert und bei +5°C unter Rühren mit einer Lösung von 104 mg Natriumnitrit in 0,2 ml Wasser versetzt. Nach 30 Minuten gibt man eine kalte Lösung von 198 mg Kupfer(I)-chlorid in 6 ml 6 N Salzsäure zu und rührt die Mischung 40 Stunden bei Raumtemperatur. Die Mischung wird mit Wasser versetzt und 3 bis 4 mal mit Essigsäureethylester extrahiert. Man erhält nach der Reinigung des Rohproduktes durch Chromatographie über Kieselgel (Laufmittel: Essigsäureethylester/Ligroin 1 1) 320 mg des 2-Chlor-3-(p-methoxyphenylazo)-pyrazolo-[1,5-a]-pyridins (R_{f}: Kieselgel, Essigsäureethylester/Ligroin 1:1 = 0,65), das analog Beispiel 10.3.1 mit Natriumdithionit reduziert wird. Man erhält die Titelverbindung vom Fp. 249-251° C (Zersetzung) R_{f} = 0,2 (Kieselgel, Essigsäureethylester/Ligroin 1:1).
10.13.2
   Die Aminoverbindung wird analog Beispiel 2.1.2 mit Phenol zum Farbstoff gekuppelt, der analog den Beispielen 2.1.3 reduziert und 8.5 N-trifluoracetyliert wird.
10.14
   4-Hydroxyphenyl-(2-morpholino-pyrazolo-[1,5-a]-pyridin-3-yl)-amin
10.14.1
   3-Amino-2-morpholino-pyrazolo-[1.5-a]-pyridin-hydrochlorid
   2-Amino-pyrazolo-[1,5-a]-pyridin (Chem. Pharm. Bull. 21, 2146 (1973) wird analog Beispiel 10.3.1 mit p-Methoxybenzoldiazoniumsalz umgesetzt. 4 g der Azoverbindung werden in 100 ml Dimethylformamid gelöst und mit 5,33 g β,β'-Dibromdiethylether und 2 g Natriumhydrid (55proz.) versetzt. Man rührt das Gemisch 1,5 Stunden bei Raumtemperatur und setzt Wasser und Essigsäureethylester zu. Die organische Phase wird abgetrennt und die wässrige Phase nochmals mit Essigsäureethylester extrahiert. Die vereinigten organischen Phasen werden getrocknet und eingeengt. Der Rückstand wird mit Hexan verrieben. Man erhält 4,75 g 3-(4-Methoxyphenyl)-azo-2-morpholinopyrazolo-[1,5-a]-pyridin (R_{f}= 0,7; Kieselgel, Essigsäureethylester/ Methylenchlorid 1:1).
   Die Azoverbindung wird analog Beispiel 10.3.1 mit Zink in Eisessig reduziert, gereinigt und die t-Butoxycarbonylgruppe mit HCl im Ethanol abgespalten. Man erhält 2,43 g der Titelverbindung (10.14.1) vom Fp. 105-110° C (Zersetzung).
   R_{f} = 0,4 (Kieselgel, Essigsäureethylester)
10.14.2
   Die Aminoverbindung wird analog Beispiel 2.1.2 mit Phenol zum Farbstoff gekuppelt, der analog den Beispielen 2.1.3 reduziert und 8.5 trifluoracetyliert wird. Analog dazu erhält man durch Kupplung mit o-Chlorphenol den entsprechenden halogenhaltigen Leukofarbstoff 3-Chlor-4-hydroxyphenol-(2-morpholino-pyrazolo-[1,5-a]pyridin-3-yl)-amin.
10.15
   3-Chlor-4-hydroxyphenyl-(7-(2-hydroxyethyl)-pyrazolo[1,5-a]-pyridin-3-yl)-amin
10.15.1
   3-Amino-7-(2-hydroxyethyl)-pyrazolo-[1.5-a]-pyridin
   19,1 g 2-Hydroxyethylpyridin werden in 50 ml Methylenchlorid gelöst und unter Eiskühlung mit einer Lösung von O-p-Toluolsulfonylhydroxylamin versetzt, die nach Literaturangaben durch Umsetzung von 40 g O-p-Toluolsulfonylacethydroxamsäureethylester mit 300 ml Perchlorsäure (60proz.) erhalten wird.
   Die Reaktionsmischung wird 15 Minuten im Eisbad und 1 Stunde bei Raumtemperatur gerührt und dann zur Trockene eingedampft. Der Rückstand wird in 40 ml Dimethylformamid gelöst und mit 28 g Kaliumcarbonat versetzt. Man tropft 15 g Propiolsäureethylester zu und leitet gleichzeitig Luft in die Reaktionsmischung ein. Nach 1 Stunde Reaktionszeit wird die Mischung eingeengt und über Kieselgel mit Ligroin/Essigsäureethylester (1:1) chromatographiert. Man erhält 10.8 g einer öligen, gelbbraunen Substanz, die in 200 ml konzentrierter Salzsäure gelöst und 3 Stunden unter Rückfluß erhitzt wird. Das Reaktionsgemisch wird unter Eiskühlung mit Natronlauge alkalisch gestellt und das Produkt mit Essigsäureethylester extrahiert. Man erhält 4,6 g eines braunen Öls.
   Analog Beispiel 2.2.1 und 2.2.2 wird die Titelverbindung (10.15.1) erhalten, Fp. 208°C R_{f} = 0,38 (Kieselgel, Essigsäureethylester/Methanol 9:1)
10.15.2
   Analog Beispiel 2.1.2 wird durch Kupplung mit 2-Chlorphenol der Farbstoff erhalten, der an der Hydroxylethylgruppe durch Umsetzung mit Acetanhydrid in Gegenwart einer katalytischen Menge p-Dimethylaminopyridin acetyliert wird und analog den Beispielen 2.1.3 reduziert und 8.5 trifluoracetyliert wird.
10.16
   (3-Chlor-4-hydroxyphenyl)-(5-(2-hydroxyethyl)-pyrazolo[1,S-a]-pyridin-3-yl)-amin
   Analog Beispiel 10.15 wird der N-trifluoracetylierte Leukofarbstoff ausgehend von 4-Hydroxyethylpyridin erhalten.
10.17
   (2-Methoxy-4-hydroxyphenyl)-(pyrazolo-[1,5-a]-pyridin-3-yl)-amin,
   (3-Chlor-4-hydroxyphenyl)-(pyrazolo-[1,5-a]-pyridin-3-yl)-amin,
   (3-Fluor-4-hydroxyphenyl)-(pyrazolo-[1,5-a]-pyridin-3yl)-amin und
   (2-Methyl-4-hydroxyphenyl)-(pyrazlo-[1,5-a]-pyridin-3-yl)-amin
   Analog Beispiel 2.1.2 und 2.1.3 wird die Leukofarbstoffkomponente durch oxidative Kupplung von 3-Amino-pyrazolo-[1,5-a]-pyridin (Bsp. 2.2.2) mit den entsprechend substituierten Phenolen erhalten. Die N-trifluoracetylierung wird analog Beispiel 8.5 durchgeführt.
10.18
   4-Hydroxyphenyl-(4,6-dimethyl-pyrazolo-[3,2-c]-s-triazol-3-yl)-amin
10.18.1
   3-Amino-4.6-dimethyl-ovrazolo-3.2-cl-s-triazol-hydrochlorid
   24,4 g 3-Amino-pyrazol-4-carbonsäureethylester (Organikum, S. 514, VEB Verlag der Wissenschaften, Berlin) werden in 100 ml konzentrierter Salzsäure suspendiert und bei 0-5°C durch Zugabe einer Lösung von 10,35 g Natriumnitrit in 50 ml Wasser diazotiert. Danach gibt man eine Lösung von 100 g Zinn(II)-chlorid in 100 ml konzentrierter Salzsäure zu und rührt die Mischung noch 2 Stunden im Eisbad. Der gelbe Niederschlag wird abfiltriert, in 150 ml Wasser gelöst und mit 20 ml Acetanhydrid versetzt. Die Mischung wird auf 80°C Badtemperatur erhitzt und portionsweise während 2 Stunden mit insgesamt 25 g Natriumbicarbonat versetzt.
   Die neutrale Lösung wird dann 24 Stunden kontinuierlich mit Essigsäureethylester extrahiert. Nach dem Trocknen und Einengen der organischen Phase erhält man 17 g 3-Acethydrazinopyrazol-4-carbonsäureethylester. (R_{f} = 0,33; Kieselgel, Aceton/Methylenchlorid/Eisessig 50:45:5), der mit Phosphoroxychlorid analog dem in DE-A1810462 Beispiel 6 beschriebenen Verfahren cyclisiert wird. Man erhält 4,8 g (32 % der Theorie) 6-Methylpyrazolo-[3,2-c]-s-triazol-3-carbonsäureethylester. (R_{f} = 0,70; Kieselgel, Toluol/Essigsäureethylester/Methanol 2:1:1)
   Analog Beispiel 10.3.1 wird der oben erhaltene Heterocyclus methyliert und das Produkt durch Chromatographie über Kieselgel mit Essigsäureethylester/Ligroin 2:1 gereinigt. Durch 6-stündiges Erhitzen mit 6 N Salzsäure wird der Ester verseift und die als Zwischenprodukt entstehende Carbonsäure decarboxyliert. Die Säure wird durch Zugabe von Natriumcarbonat neutralisiert und das Produkt mit Essigsäureethylester extrahiert. Man erhält 1,1 g öliges 4,6-Dimethyl-pyrazolo-[3,2-c]-s-triazol. (R_{f} = 0, 26; Kieselgel, Essigsäureethylester/Ligroin 1:1)
   Der Heterocyclus wird analog Beispiel 2.2.1 nitrosiert und die Nitrosogruppe analog Beispiel 3.1 mit Palladium/Kohle/Hydrazin reduziert. Man erhält die Titelverbindung (10.18.1) als Hydrochlorid vom Fp 190° C (Zersetzung) R_{f} = 0, 53; Kieselgel, Essigsäureethylester/Aceton/ Eisessig/Wasser 50:25:12,5:12,5
10.18.2.
   Die Aminoverbindung wird analog Beispiel 2.1.2 mit Phenol zum Farbstoff gekuppelt, der entsprechend den Beispielen 2.1.3 reduziert und 8.5 N-trifluoracetyliert wird.
10.19
   (5-Brom-2-methoxy-4-hydroxyphenyl)-(2-methoxy-pyrazolo[1,5-a]-pyridin-3-yl)-amin
   Analog Beispiel 10.1.3 erhält man den Leukofarbstoff ausgehend von 2-Brom-5-methoxyphenol.
10.20
   (3-Chlor-4-hydroxyphenyl)-(2-(2,3-dihydroxypropyl)pyrazolo-[1,5-a]-pyridin-3-yl)-amin
10.20.1
   7 g 3-Acetyl-2-hydroxy-pyrazolo-[1,5-a]-pyridin (Chem. Pharm. Bull. 23, 452 (1975) werden analog Beispiel 10.3.1 in Dimethylformamid mit 14,3 g R,S-(2,2-Dimethyl-1,3-dioxolan-4-yl)-methyl-p-toluolsulfcnat und Natriumhydrid als Base 0-alkyliert. Das Rohprodukt wird durch Chromatographie über Kieselgel mit Essigsäureethylester/ Ligroin als Laufmittel gereinigt. Man erhält 1,47 g 3-Acetyl-2-(R,S-2,2-dimethyl-1,3-dioxolan-4-yl)-methyloxy)-pyrazolo-[1,5-a]-pyridin, R_{f} = 0,45; (Kieselgel, Essigsäureethylester/Ligroin 1:1)
10.20.2
   Analog Beispielen 2.1.1 und 2.1.2 erhält man aus der oben erhaltenen Verbindung durch Kupplung mit 2-Chlorphenol den Farbstoff, wobei bei der Nitrosierung das Ketal gespalten wird und auf der Farbstoffstufe, die beiden freien, aliphatischen Hydroxygruppen durch Acetylierung mittels Acetanhydrid und p-Dimethylaminopyridin als Katalysator geschützt werden.
   Analog Beispiel 2.1.3 wird der Farbstoff zum Leukofarbstoff reduziert, und entsprechend Bsp. 8.5 N-trifluoracetyliert. Die Acetoxygruppen werden bei der Schutzgruppenabspaltung nach der Glycosidierung mit abgespalten.
10.20.3.
   Analog Beispielen 10.20.1. - 10.20.2 erhält man auch die Leukofarbstoffkomponente mit 2,5-Dichlorphenol
10.21
   4-Hydroxphenyl-(2-methoxy-5,7-dimethyl-pyrazolo-[1,5-a]-pyrimidin-3-yl)-amin
   Analog Beispiel 2.1.1 erhält man ausgehend von 2-Methoxy-5,7-dimethyl-pyrazolo-[1,5-a]-pyrimidin (Anm. Chem. 647, 116 (1961) 3-Amino-5,7-dimethyl-2-methoxypyrazolo-[1,5-a]-pyrimidin Hydrochlorid vom Fp. 187-190°C, das analog Beispiel 2.1.2 mit Phenol zum Farbstoff gekuppelt wird, der analog den Beispielen 2.1.3 reduziert und 8.5 trifluoracetyliert wird.

### Beispiel 11

Analog Beispiel 9 erhält man die in der nachfolgenden Tabelle 3 aufgeführten Verbindungen.

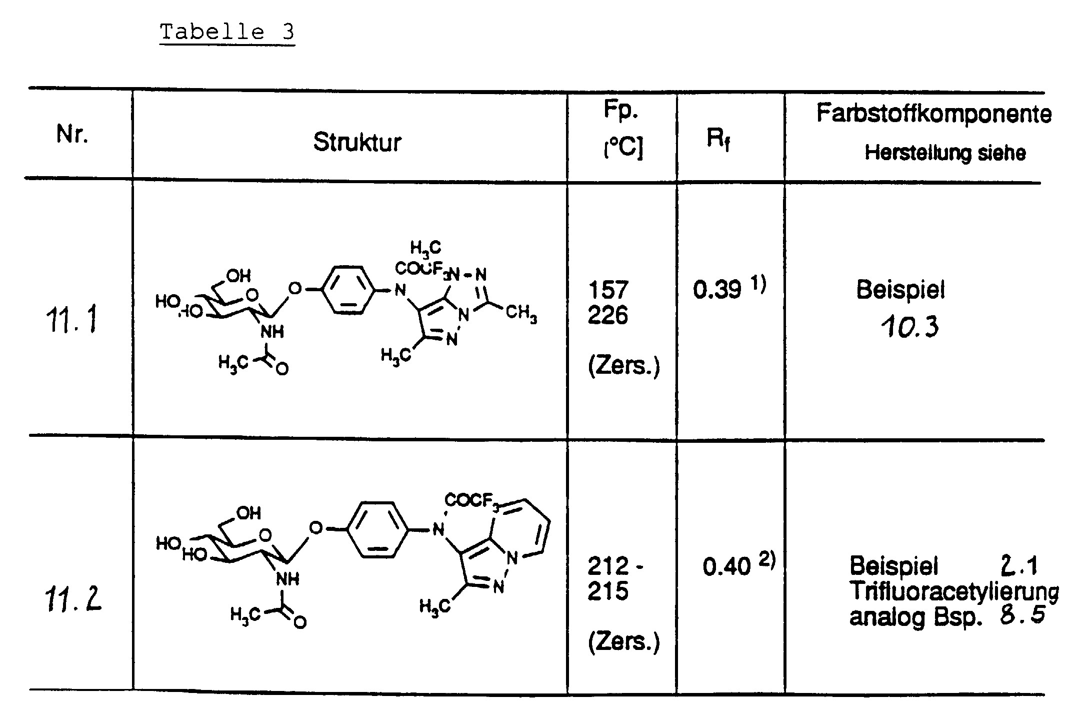

### Beispiel 12

### 4-((2-Methyl-pyrazolo-[1,5-a]-pyridin-3-yl)-amino)-phenyl-β-D-galactosid

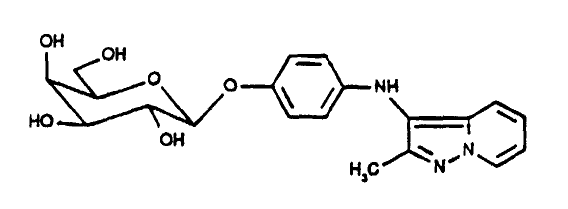

Analog Beispiel 1.2 und 1.3 erhält man unter Verwendung des in Beispiel 2.1 erhaltenen Leukofarbstoffes und 2, 3, 4,6-Tetra-O-acetyl-α-D-galactosylbromid die Titelverbindung vom Fp. 137°C R_{f} = 0,5 (Kieselgel, Toluol/Essigsäureethylester/Methanol 1:1:1)

### Beispiel 13

### Teststreifen zur Bestimmung von Leukozytenesterase (Leukozytentest)

Ein Filterpapier der Firma Schleicher und Schüll (23 SL) wird nacheinander mit folgenden Lösungen imprägniert und getrocknet:
1. Boratpuffer 50 mMol/l, pH 8
2. Indikator aus Beispiel 6 in Methanol 4 mMol/l

Ein Netz aus Nylon (NY 75 HC der Firma Züricher Beuteltuchfabrik, Zürich, Schweiz) mit einer Fadendicke von 60 m, wird mit einer 50 mMol/l wäßrigen Kaliumjodatlösung getränkt und getrocknet.

Aus dem mit Puffer und Indikator getränktem Papier (1), Jodatgewebe (2), Abdecknetz (3), ebenfalls aus Nylon (NY 75 HC) und einer steifen Plastikfolie (4) wird ein Teststreifen analog Fig. 4 gefertigt.

Beim Eintauchen eines solchen Teststreifens in eine leukozytenhaltige Lösung tritt eine Verfärbung von farblos bis blau ein. So ist z. B. eine Konzentration von 500 Leukozyten/µl nach 1 Minute durch Verfärbung nachzuweisen.

### Beispiel 14

### Bestimmung von alkalischer Phosphatase

### a) Teststreifen

Wie in Beispiel 13 beschrieben, wird ein Filterpapier der Firma Schleicher und Schüll (23 SL) getränkt mit:
1. Hepes Puffer 50 m/Mol/l, pH 7,5
2. Indikator aus Beispiel 7 in Methanol 4 mMol/l,
getrocknet und zusammen mit einem wie in Beispiel 13 hergestellten Jodatgewebe eingenetzt.

Beim Eintauchen eines solchen Teststreifens in eine alkalische Phosphatase-Lösung tritt eine Verfärbung von farblos bis blau auf. Eine Aktivität von 176 U/l ist nach 5 Minuten durch Verfärbung nachzuweisen.

### b) Naßchemische Bestimmung

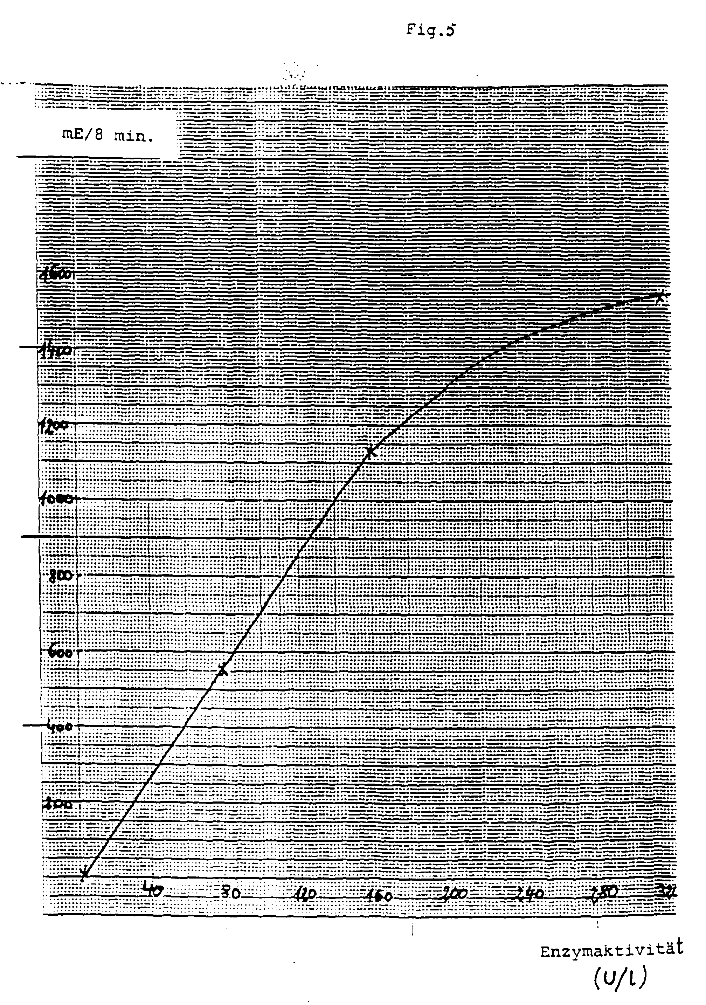

900 µl einer Lösung von 4 mMol/l Substrat aus Beispiel 7 in Hepespuffer (50 mMol/l) pH 7,5 werden vorgelegt. Man pipettiert:
1. 100 µl einer 220 mMol/l Kaliumjodatlösung und
2. 100 µl einer alkalischen-Phosphatase-haltigen Lösung zu,
mischt und mißt die Kinetik bei einer Wellenlänge von 573 nm. Mit bekannten enzymhaltigen Lösungen läßt sich eine Eichkurve entsprechend Fig. 5 erhalten. Bei dieser Eichkurve sind Leerwerte (entsprechendes Verfahren ohne Enzym) abgezogen.

### Beispiel 15

### Bestimmung von β-D Galaktosidase

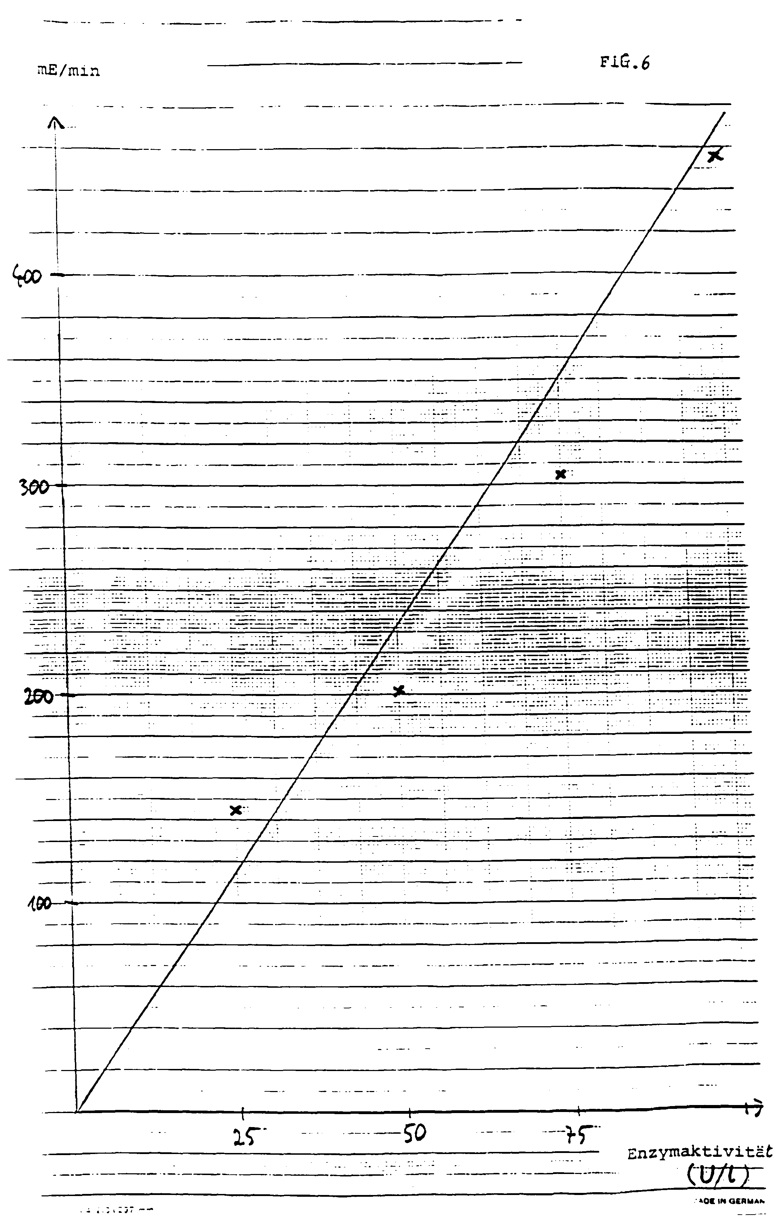

900 µl einer Lösung von 4 mMol/l Substrat aus Beispiel 12 in 50 mMol/l Hepes-Puffer, pH 7,5 werden in einer Küvette vorgelegt. Man pipettiert:
1. 100 µl einer 220 mMol/l Kaliumjodat-Lösung und
2. 100 µl einer β-G-Galaktosidase-haltigen Lösung zu,
mischt und mißt die Kinetik bei einer Wellenlänge von 560 nm. Mit bekannten β-Galaktosidase-haltigen Lösungen läßt sich eine Eichkurve wie in Fig. 6 erhalten.

### Beispiel 16

### Teststreifen zur Bestimmung von N-Acetyl-β-D-glucosaminidase (β-NAGase)

Ein Filterpapier der Firma Schleicher und Schüll (23 SL) wird nacheinander mit folgenden Lösungen imprägniert und getrocknet:
1. Citratpuffer 200 mMol/l, pH 5
2. Indikator 10 mMol/l und Phenylsemicarbazid 4 mMol/l in Methanol.

Ein Netz aus Nylon (NY 75 HC der Firma Züricher Beuteltuchfabrik, Zürich, Schweiz) mit einer Fadendicke von 60 µm wird mit einer 40 mMol/l wässrigen Kaliumjodat-Lösung getränkt und getrocknet.

Aus dem mit Puffer und Indikator imprägnierten Papier (1), Jodatgewebe (2), Abdecknetz (3), (ebenfalls aus Nylon NY 75 HC) und einer steifen Plastikfolie (4) wird ein Teststreifen analog Fig. 4 hergestellt.

Beim Eintauchen eines solchen Teststreifens in β-NAGase-haltige Lösung (20 U/l), tritt eine Verfärbung des Streifens ein. Im folgenden ist angegeben, welche Indikatoren welche Verfärbung ergeben:

| Substrat aus Beispiel | Verfärbung | |
|---|---|---|
| | Von | nach |
| 10 a | Farblos | rot |
| 10 c | Farblos | rot |
| 10 d | farblos | rota |
| 10 e | farblos | violett |
| 10 g | farblos | rot |
| 10 h | farblos | rot |
| 10 i | farblos | rot |
| 10 j | farblos | rot |
| 10 k | farblos | blau-grau |
| 10 l | farblos | rot |
| 10 m | farblos | orange |
| 10 n | farblos | rot |
| 10 t | farblos | rot |
| 10 u | farblos | rot |
| 10 v | farblos | rot |
| 10 y | farblos | rot |

## Patentansprüche

1. N- und O-substituiertes Aminophenolderivat der allgemeinen Formel I 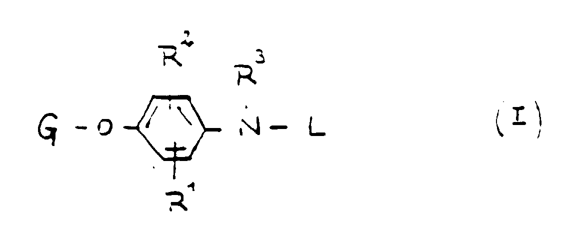 in der
G einen organischen oder anorganischen Säurerest oder einen Glycosidrest darstellt,
R¹ und R², die gleich oder verschieden sind, Wasserstoff,
Halogen, SO₃H, PO₃H₂ oder ein Salz dieser Säuregruppe, eine Hydroxy-, Nitro-, Carboxy-, Carboxamido- oder Cyanogruppe oder eine gegebenenfalls durch einen oder mehrere Hydroxy-, Carboxy-, Halogen-, Cyano-, SO₃H- oder PO₃H₂-Reste oder ein Salz eines dieser Säurereste substituierte Alkyl-, Alkenyl-, Alkoxy-, Alkylsulfinyl-, Alkylsulfonyl-, Alkoxycarbonyl-, Alkylcarbonyl-, Aryl- oder Aralkylgruppe bedeuten oder
wenn sich beide Reste an benachbarten Kohlenstoffatomen befinden gemeinsam eine 1,4-Butadiendiylgruppe darstellen, die gegebenenfalls ein- oder mehrmals durch SO₃H, PO₃H₂ oder ein Salz dieser Säuregruppen, eine Alkyl- oder/und eine Carboxygruppe substituiert ist,
R³ Wasserstoff, CO-COOH, SO₃H, PO₃H₂ oder ein Salz dieser Säuregruppen, eine gegebenenfalls ein- oder mehrfach durch Halogen, CO₂H, SO₃H oder/und PO₃H₂ oder ein Salz dieser Säuregruppen substituierte Alkylcarbonylgruppe oder eine gegebenenfalls ein- oder mehrfach durch SO₃H, PO₃ H₂ oder ein Salz dieser Säurereste substituierte Arylcarbonylgruppe ist und
L einen Pyrazoloheterocyclus-Rest der allgemeinen Formel III 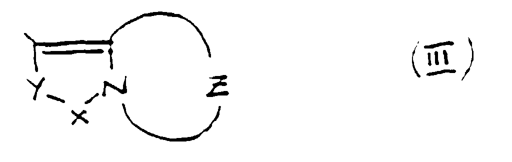 darstellt,
in der
X-Y NR⁸-cO oder N=CR⁹ bedeutet,
wobei
R⁸ Wasserstoff oder Alkyl und
R⁹ Alkyl, Alkenyl, Alkoxy, Alkylthio, Aryl, Aralkyl, gegebenenfalls jeweils substituiert durch Hydroxy, Dialkylphosphinyl, Carboxy, SO₃H, PO₃H₂, ein Salz eines dieser Säurereste oder/und Alkoxycarbonyl;
Amino, das gegebenenfalls durch ein oder zwei gegebenenfalls einen oder mehrere Hydroxy-, Carboxy- oder/und Alkoxycarbonylreste tragenden Alkylrest substituiert ist wobei, wenn Amino durch 2 Alkylreste substituiert ist, diese Reste auch zu einem Ring geschlossen sein können, der außer durch das N-Atom der Aminogruppe gegebenenfalls auch durch Sauerstoff, Schwefel oder ein weiteres Stickstoffatom unterbrochen sein kann, oder Amino gegebenenfalls durch ein oder zwei Acylgruppen, Alkoxy- oder/und Aralkoxyzarbonylgruppen, H₂N-CO, Alkyl-, Aralkyl- oder Arylcarbamoylgruppen substituiert ist; oder
Wasserstoff, Carboxy, Alkoxycarbonyl, Carboxamido oder Halogen ist, und
Z NR¹⁰-N=N bedeutet
wobei R¹⁰ Wasserstoff, Alkyl oder Aralkyl ist oder
Z eine ungesättigte Kette mit 3 bis 5 Gliedern aus Stickstoffatomen oder aus Kohlenstoff- atomen und gegebenenfalls einem oder mehreren Stickstoff- oder Schwefelatomen darstellt, wobei Kohlenstoffatome gegebenenfalls durch Alkyl, Alkoxy, Hydroxyalkyl, Alkylthio, Hydroxy, Aralkyl, Aryl, Carboxy, Carboxamido, Alkoxycarbonyl, Cyano, Amino, das gegebenenfalls durch ein oder zwei gegebenenfalls einen oder mehrere Hydroxy-, Carboxy- oder/und Alkoxycarbonylreste tragenden Alkylrest substituiert ist, oder/und Halogen sowie Stickstoffatome, die nicht über eine Doppelbindung gebunden sind, gegebenenfalls durch Alkyl oder Aralkyl substituiert sind oder zwei benachbarte Kettensubstituenten gegebenenfalls eine Alkylengruppe bilden, die ihrerseits gegebenenfalls mit Aryl substituiert oder anelliert ist oder ein entsprechender tautomerer Rest
unter der Bedingung, daß, wenn
G einen Alkancarbonsäurerest,
R³ Wasserstoff, eine Arylcarbonylgruppe oder eine gegebenenfalls ein- oder mehrfach durch Halogen substituierte Alkylcarbonylgruppe und
L einen Pyrazoloheterocyclus-Rest der allgemeinen Formel III mit X-Y in der Bedeutung N=CR⁹, wobei R⁹ die zuvor angegebene Bedeutung hat,
darstellen,
Z keinen 1,2,4-Triazolring bildet, bei dem ein nicht über eine Doppelbindung gebundener Stickstoff mit Wasserstoff substituiert ist.

2. Verbindung gemäß Anspruch 1, dadurch gekennzeichnet, daß
G einen Galactosid-, Glycosid-, Mannosid-, N-Acetyl-glucosaminid- oder einen Oligosaccharidrest mit 2 - 10 Monosaccharid-Einheiten bedeutet.

3. Verbindung gemäß Anspruch 1, dadurch gekennzeichnet, daß
G einen N-Acetyl-β-D-glucosaminidylrest darstellt.

4. Verbindung gemäß Anspruch 1, dadurch gekennzeichnet, daß
G PO₃MM', SO₃M, einen carboxylgebundenen Alkancarbonsäure-, Aminosäure- oder Oligopeptidrest und M und M' Wasserstoff, ein Alkali-, Erdalkali- oder Ammoniumion bedeuten.

5. Verfahren zur Herstellung einer Verbindung gemäß Anspruch 1, dadurch gekennzeichnet, daß
eine Verbindung der allgemeinen Formel IV
G-D (IV)
in der
G einen organischen oder anorganischen Säurerest oder einen Glycosidrest und
D eine reaktive Gruppe bedeutet,
wobei funktionelle Gruppen in G gegebenenfalls mit Schutzgruppen, wie sie in der Peptid- und Kohlenhydratchemie üblich sind, geschützt sind mit einer Verbindung der allgemeinen Formel V 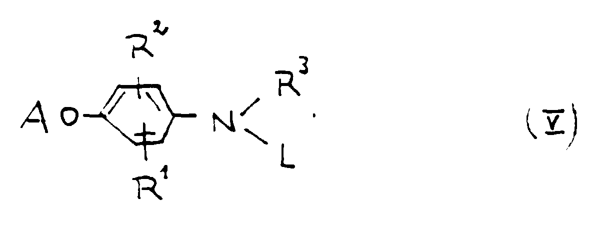 in der
R¹-R³ und L die in Anspruch 1 angegebene Bedeutung haben und
A Wasserstoff, ein gegebenenfalls substituiertes Ammoniumion oder ein Alkalimetall darstellt,
umgesetzt wird,
und gegebenenfalls abschließend Schutzgruppen abgespalten werden.

6. Verfahren gemäß Anspruch 5, dadurch gekennzeichnet, daß zur Herstellung einer Verbindung gemäß Anspruch 3 als Verbindung der allgemeinen Formel IV
eine Verbindung der allgemeinen Formel VI 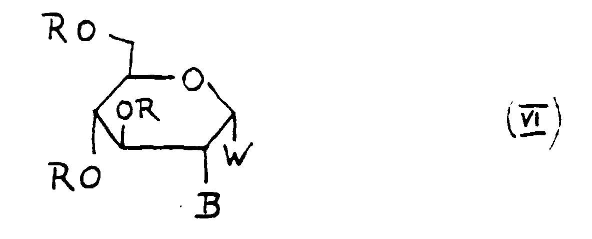 in der
W ein Halogenrest,
R eine in der Kohlenhydratchemime übliche Hydroxyschutzgruppe und
B ein Azidrest, eine geschützte Aminogruppe oder NH-COCH₃ darstellen oder
B und W zusammen die Gruppe 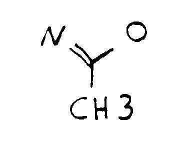 bedeutet,
mit einer Verbindung der allgemeinen Formel V 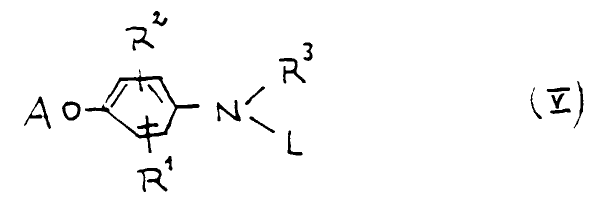 in der
R¹-R³ und L die in Anspruch 1 angegebene Bedeutung haben und
A Wasserstoff, ein gegebenenfalls substituiertes Ammoniumion oder ein Alkalimetall darstellt,
umgesetzt wird,
wenn B eine geschützte Aminogruppe darstellt, die Aminoschutzgruppe entfernt oder
wenn B einen Azidrest darstellt, dieser durch Reduktion in eine NH₂-Gruppe überführt und die Aminogruppe durch Acetylierung in den NHCOCH₃-Rest umgewandelt wird, und abschließend die Hydroxyschutzgruppen abgespalten werden.

7. Verbindung der allgemeinen Formel V' 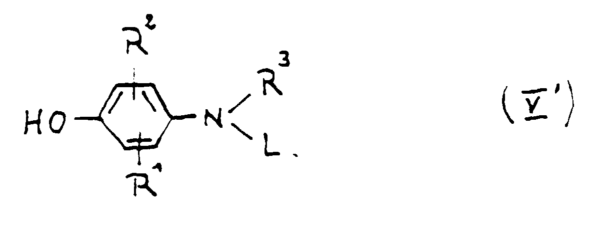 in der
R¹-R³ die in Anspruch 1 angegebene Bedeutung haben und
L einen Pyrazoloheterocyclus-Rest der allgemeinen Formel III 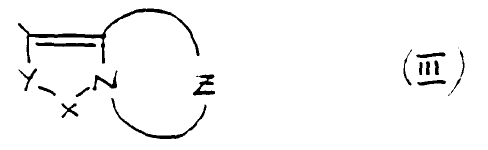 darstellt, in der X-Y und Z die in Anspruch 1 angegebene Bedeutung haben unter der Bedingung, daß, wenn
R³ eine Arylcarbonylgruppe oder eine gegebenenfalls ein- oder mehrfach durch Halogen substituierte Alkylcarbonylgruppe darstellt, und
X-Y gleich C=NR⁹ ist,
Z keinen 1,2,4-Triazolring bildet, bei dem ein nicht über Doppelbindung gebundener Stickstoff mit Wasserstoff substituiert ist.

8. Verfahren zur Herstellung einer Verbindung gemäß Anspruch 7, dadurch gekennzeichnet, daß
a) eine Substanz der allgemeinen Formel VII 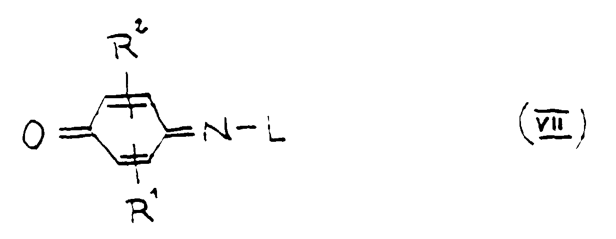 in der
R¹, R² und L die in Anspruch 7 angegebene Bedeutung haben, reduziert und, wenn R³ nicht Wasserstoff sein soll, die Anilinogruppe acyliert und abschließend eine gegebenenfalls vorliegende Esterbindung gespalten wird oder
b) eine Verbindung gemäß Anspruch 1, wobei L einen Pyrazoloheterocyclus-Rest der allgemeinen Formel III 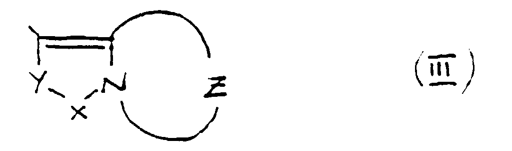 darstellt,
in der X-Y, G und Z die in Anspruch 1 angegebene Bedeutung haben, mit einer entsprechenden Hydrolase umgesetzt wird.

9. Verbindung der allgemeinen Formel VII 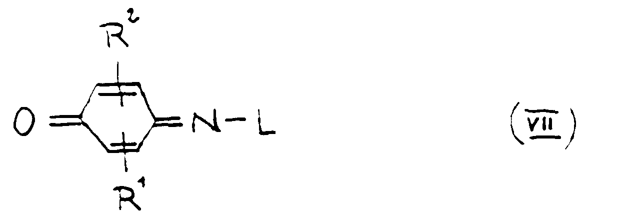 in der
R¹ und R² die in Anspruch 1 angegebene Bedeutung haben und
L einen Pyrazoloheterocyclus-Rest der allgemeinen Formel III 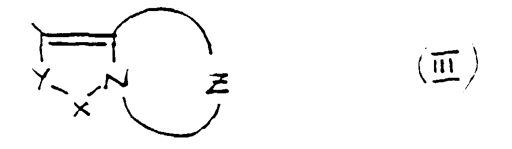 darstellt,
in der X-Y und Z die in Anspruch 1 angegebene Bedeutung haben.

10. Verfahren zur Herstellung einer Verbindung gemäß Anspruch 9, dadurch gekennzeichnet, daß
a) eine Verbindung gemäß Anspruch 1, wobei L einen Pyrazoloheterocyclus-Rest der allgemeinen Formel III 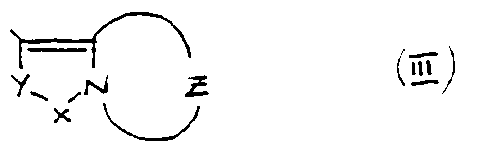 darstellt,
in der X-Y und Z die in Anspruch 1 angegebene Bedeutung haben
mit einer Hydrolase umgesetzt und das Reaktionsprodukt oxidiert wird oder
b) eine Verbindung der allgemeinen Formel VIII
NH₂-L (VIII)
in der
L einen Pyrazoloheterocyclus-Rest der allgemeinen Formel III 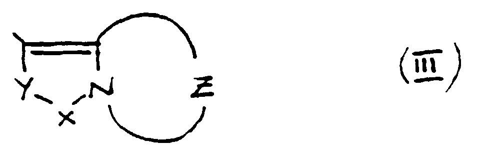 darstellt,
in der X-Y und Z die in Anspruch 1 angegebene Bedeutung haben
mit einem Phenol der allgemeinen Formel IX 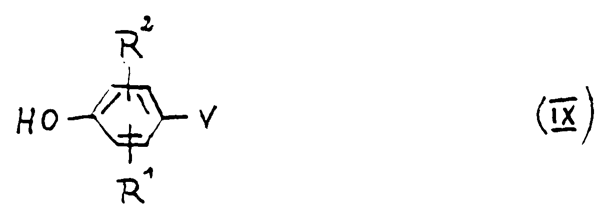 in der
R¹ und R² die in Anspruch 1 angegebene Bedeutung haben und
V Wasserstoff, Halogen, CO₂H oder SO₃H darstellt
in Gegenwart eines Oxidationsmittels umgesetzt wird oder
c) eine Verbindung der allgemeinen Formel X
L-H (X)
in der L einen Pyrazoloheterocyclus-Rest der allgemeinen Formel III 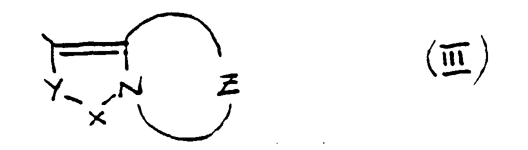 darstellt, wobei X-Y und Z die in Anspruch 1 angegebene Bedeutung haben
mit einer Verbindung der allgemeinen Formel XI 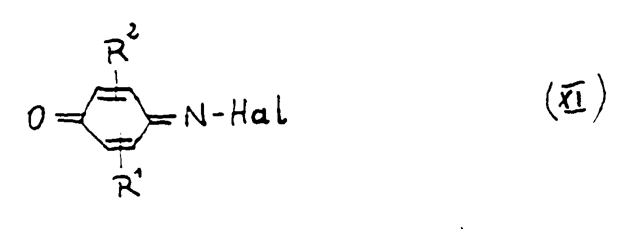 in der
R¹ und R² die in Anspruch 1 angegebene Bedeutung haben und
Hal einen Halogenrest bedeutet,
umgesetzt wird, oder
d) eine Verbindung der allgemeinen Formel XXV
L-E (XXV)
in der
E eine Nitro- oder Nitrosogruppe bedeutet und
L für einen Pyrazoloheterocyclus-Rest der allgemeinen Formel III 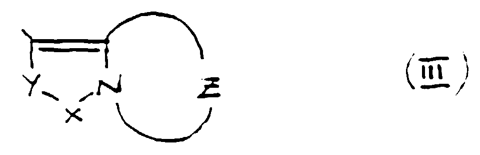 steht, wobei X-Y und Z die in Anspruch 1 angegebene Bedeutung haben
mit einer metallorganischen Verbindung der allgemeinen Formel XXVI 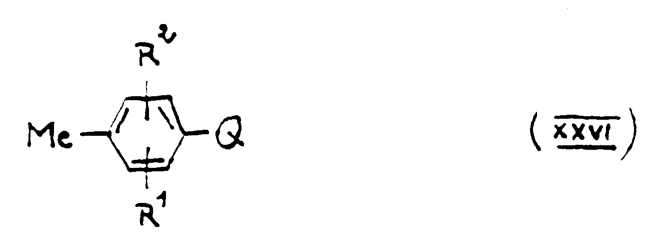 in der
R¹ und R² die in Anspruch 9 angegebene Bedeutung haben,
Q eine Hydroxy- oder Dialkylaminogruppe und
Me Lithium oder durch Halogen substituiertes Magnesium
bedeuten, umgesetzt und anschließend wässrig aufgearbeitet wird.

11. Verwendung einer Verbindung der allgemeinen Formel I 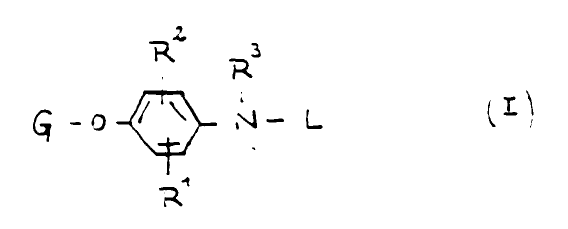 in der
G einen organischen oder anorganischen Säurerest oder einen Glycosidrest darstellt,
R¹ und R², die gleich oder verschieden sind, Wasserstoff,
Halogen, SO₃H, PO₃H₂ oder ein Salz dieser Säuregruppe, eine Hydroxy-, Nitro-, Carboxy-, Carboxamido- oder Cyanogruppe oder eine gegebenenfalls durch einen oder mehrere Hydroxy-, Carboxy-, Halogen-, Cyano-, SO₃H- oder PO₃H₂- Reste oder ein Salz eines dieser Säurereste substituierte Alkyl-, Alkenyl-, Alkoxy-, Alkyl- sulfinyl-, Alkylsulfonyl-, Alkoxycarbonyl-, Alkylcarbonyl-, Aryl- oder Aralkylgruppe bedeuten oder
wenn sich beide Reste an benachbarten Kohlenstoffatomen befinden gemeinsam eine 1,4-Butadiendiylgruppe darstellen, die gegebenenfalls ein- oder mehrmals durch SO₃H, PO3 H2 oder ein Salz dieser Säuregruppen, eine Alkyl- oder/und eine Carboxygruppe substituiert ist,
R³ Wasserstoff, CO-COOH, SO₃H, PO₃H₂ oder ein Salz dieser Säuregruppen, eine gegebenenfalls ein- oder mehrfach durch Halogen, CO₂H, SO₃H oder/und PO₃H₂ oder ein Salz dieser Säuregruppen substituierte Alkylcarbonylgruppe oder eine gegebenenfalls ein- oder mehrfach durch SO₃H, PO₃H₂ oder ein Salz dieser Säurereste substituierte Arylcarbonylgruppe ist und
L einen Pyrazoloheterocyclus-Rest der allgemeinen Formel III 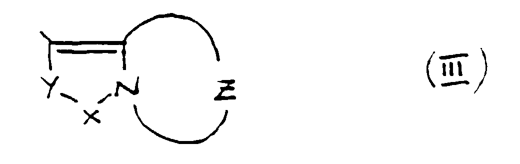 darstellt,
in der
X-Y NR⁸-CO oder N=CR⁹ bedeutet,
wobei
R⁸ Wasserstoff oder Alkyl und
R⁹ Alkyl, Alkenyl, Alkoxy, Alkylthio, Aryl, Aralkyl, gegebenenfalls jeweils substituiert durch Hydroxy, Dialkylphosphinyl, Carboxy, SO₃H, PO₃ H₂, ein Salz eines dieser Säurereste oder/und Alkoxycarbonyl;
Amino, das gegebenenfalls durch ein oder zwei gegebenenfalls einen oder mehrere Hydroxy-, Carboxy- oder/und Alkoxycarbonylreste tragenden Alkylrest substituiert ist wobei, wenn Amino durch 2 Alkylreste substituiert ist, diese Reste auch zu einem Ring geschlossen sein können, der außer durch das N-Atom der Aminogruppe gegebenenfalls auch durch Sauerstoff, Schwefel oder ein weiteres Stickstoffatom unterbrochen sein kann, oder Amino gegebenenfalls durch ein oder zwei Acylgruppen, Alkoxy- oder/und Aralkoxycarbonylgruppen, H₂N-CO, Alkyl-, Aralkyl- oder Arylcarbamoylgruppen substituiert ist; oder
Wasserstoff, Carboxy, Alkoxycarbonyl, Carboxamido oder Halogen ist, und
Z NR¹⁰-N=N bedeutet
wobei R¹⁰ Wasserstoff, Alkyl oder Aralkyl ist oder
Z eine ungesättigte Kette mit 2 bis 5 Gliedern aus Stickstoffatomen oder aus Kohlenstoffatomen und gegebenenfalls einem oder mehreren Stickstoff- oder Schwefelatomen darstellt, wobei Kohlenstoffatome gegebenenfalls durch Alkyl, Alkoxy, Hydroxyalkyl, Alkylthio, Hydroxy, Aralkyl, Aryl, Carboxy, Carboxamido, Alkoxycarbonyl, Cyano, Amino, das gegebenenfalls durch ein oder zwei gegebenenfalls einen oder mehrere Hydroxy-, Carboxy- oder/und Alkoxycarbonylreste tragenden Alkylrest substituiert ist, oder/und Halogen sowie Stickstoffatome, die nicht über eine Doppelbindung gebunden sind, gegebenenfalls durch Alkyl oder Aralkyl substituiert sind oder zwei benachbarte Kettensubstituenten gegebenenfalls eine Alkylengruppe bilden, die ihrerseits gegebenenfalls mit Aryl substituiert oder anelliert ist oder ein entsprechender tautomerer Rest als Hydrolasesubstrat.

12. Verwendung gemäß Anspruch 11 einer Verbindung der allgemeinen Formel I wie in Anspruch 11 definiert, wobei G einen N-Acetyl-β-D-glucosaminidylrest darstellt, als Substrat für das Enzym N-Acetyl-β-D-glucosaminidase.

13. Verwendung von Verbindungen gemäß Ansprüchen 7 oder 9 zur Herstellung von Verbindungen der allgemeinen Formel I wie sie in Ansprüchen 1 und 11 definiert sind.

14. Verfahren zur kolorimetrischen Bestimmung einer Hydrolase durch Umsetzung eines chromogenen Enzymsubstrats mit dem Enzym, Oxidation eines durch das Enzym aus dem Substrat freigesetzten Leukofarbstoffs und Bestimmung des resultierenden Farbstoffs als Maß für die Menge an Enzym, dadurch gekennzeichnet, daß als Enzymsubstrat eine Verbindung gemäß Anspruch 11 eingesetzt wird.

15. Verfahren gemäß Anspruch 14 zur Bestimmung des Enzyms N-Acetyl-β-D-glucosaminidase, dadurch gekennzeichnet, daß als Enzymsubstrat eine Verbindung gemäß Anspruch 12 eingesetzt wird.

16. Diagnostisches Mittel zur Bestimmung einer Hydrolase enthaltend ein chromogenes Enzymsubstrat und eine geeignete Puffersubstanz, dadurch gekennzeichnet, daß als chromogens Enzymsubstrat eine Verbindung gemäß Anspruch 11 eingesetzt wird.

17. Diagnostisches Mittel gemäß Anspruch 16, dadurch gekennzeichnet, daß es ein Oxidationsmittel enthält.

18. Diagnostisches Mittel gemäß einem der Ansprüche 16 oder 17, dadurch gekennzeichnet, daß es zusätzlich ein 1-Arylsemicarbazid der allgemeinen Formel XII
Ar-NH-NH-CONH₂ (XII)
in der
Ar einen gegebenenfalls durch Alkyl, Alkoxy oder Halogen substituierten Arylrest bedeutet,
enthält.

19. Diagnostisches Mittel gemäß einem der Ansprüche 16-18, dadurch gekennzeichnet, daß es die Komponenten in trägergebundener Form enthält.

20. Verwendung einer Verbindung der allgemeinen Formel I wie sie in Anspruch 1 definiert ist zur Herstellung eines diagnostischen Mittels zur Bestimmung einer Hydrolase.

## Claims

1. N- and O-substituted aminophenol derivatives of the general formula I 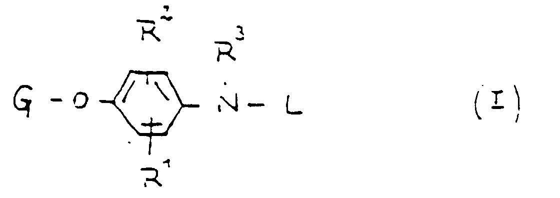 in which
G represents an organic or inorganic acid residue or a glycoside residue,
R¹ and R² which are the same or different, denote hydrogen,
halogen, SO₃H, PO₃H₂ or a salt of these acid residues, a hydroxy, nitro, carboxy, carboxamido or cyano group or an alkyl, alkenyl, alkoxy, alkylsulfinyl, alkylsulfonyl, alkoxycarbonyl, alkylcarbonyl, aryl or aralkyl group optionally substituted by one or several hydroxy, carboxy, halogen, cyano, SO₃H or PO₃H₂ residues or a salt of one of these acid residues or if both residues are present on neighbouring carbon atoms they together represent a 1,4 butadienediyl group which is optionally substituted once or several times by SO₃H, PO₃H₂ or a salt of these acid groups, an alkyl or/and a carboxy group,
R³ is hydrogen, CO-COOH, SO₃H, PO₃H₂ or a salt of these acid groups, an alkylcarbonyl group optionally substituted once or several times by halogen, CO₂H, SO₃H or/and PO₃H₂ or a salt of these acid groups, or an arylcarbonyl group optionally substituted once or several times by SO₃H, PO₃H₂ or a salt of these acid residues and
L represents a pyrazolo-heterocyclic residue of the general formula III 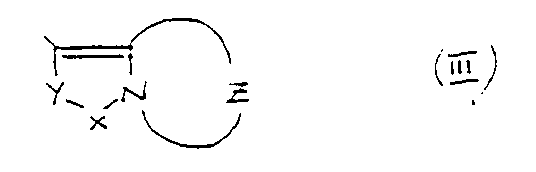 in which
X-Y denotes NR⁸-CO or N=CR⁹
in which
R⁸ is hydrogen or alkyl and
R⁹ is alkyl, alkenyl, alkoxy, alkylthio, aryl, aralkyl, each optionally substituted by hydroxy, dialkylphosphinyl, carboxy, SO₃H, PO₃H₂, a salt of one of these acid residues or/and alkoxycarbonyl;
amino which is optionally substituted by one or two alkyl residues which are optionally substituted by one or several hydroxy, carboxy or/and alkoxycarbonyl residues whereby if amino is substituted by two alkyl residues, these residues can also be joined to form a ring which, apart from the N atom of the amino group, can optionally also be interrupted by oxygen, sulphur or a further nitrogen atom, or amino is optionally substituted by one or two acyl groups, alkoxy or/and aralkoxycarbonyl groups, H₂N-CO, alkyl, aralkyl, or arylcarbamoyl groups; or
is hydrogen, carboxy, alkoxycarbonyl, carboxamido or halogen and
Z denotes NR¹⁰-N=N
in which R¹⁰ is hydrogen, alkyl or aralkyl or
Z represents an unsaturated chain containing 3 to 5 members composed of nitrogen atoms or of carbon atoms and optionally one or several nitrogen or sulphur atoms, in which the carbon atoms are optionally substituted by alkyl, alkoxy, hydroxyalkyl, alkylthio, hydroxy, aralkyl, aryl, carboxy, carboxamido, alkoxycarbonyl, cyano, amino which is optionally substituted by one or two alkyl residues which are optionally substituted by one or several hydroxy, carboxy or/and alkoxycarbonyl residues, or/and halogen as well as nitrogen atoms which are not linked by a double bond and are optionally substituted by alkyl or aralkyl or two neighbouring chain substituents optionally form an alkylene group which in turn is optionally substituted or anellated with aryl or a corresponding tautomeric residue
provided that if
G is an alkane-carboxylic acid residue,
R³ is hydrogen, an arylcarbonyl group or an alkylcarbonyl group optionally substituted once or several times by halogen and
L is a pyrazolo-heterocyclic residue of the general formula III in which X-Y denotes N=CR⁹ and R⁹ has the previously stated meaning,
Z does not form a 1,2,4-triazole ring in which a nitrogen that is not bound via a double bond is substituted with hydrogen.

2. Compound as claimed in claim 1, wherein
G denotes a galactoside, glycoside, mannoside, N-acetyl glucosaminide or an oligosaccharide residue containing 2 - 10 monosaccharide units.

3. Compound as claimed in claim 1, wherein
G denotes an N-acetyl-β-D-glucosamininidyl residue.

4. Compound as claimed in claim 1, wherein
G denotes PO₃MM', SO₃M, a carboxyl-bound alkane carboxylic acid, amino acid or oligopeptide residue and M and M' denote hydrogen, an alkali, alkaline earth or ammonium ion.

5. Process for the production of a compound as claimed in claim 1, wherein
a compound of the general formula IV
G-D (IV)
in which
G denotes an organic or inorganic acid residue or a glycoside residue and
D denotes a reactive group,
and functional groups in G are optionally protected with protective groups that are conventionally used in peptide and carbohydrate chemistry, is reacted with a compound of the general formula V 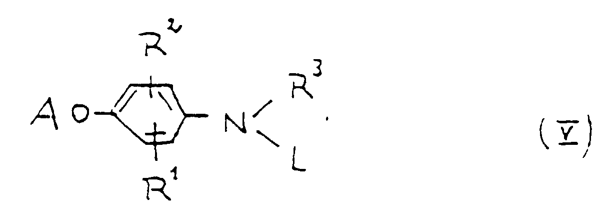 in which
R¹-R³ and L have the meaning given in claim 1 and
A represents hydrogen, an optionally substituted ammonium ion or an alkali metal,
and optionally protective groups are subsequently cleaved off.

6. Process as claimed in claim 5, wherein in order to produce a compound as claimed in claim 3 as a compound of the general formula IV,
a compound of the general formula VI 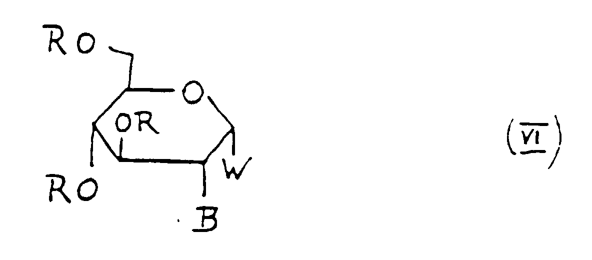 in which
W represents a halogen residue,
R represents a standard hydroxy protective group in carbohydrate chemistry and
B represents an azide residue, a protected amino group or NH-COCH₃ or
B and W together denote the group 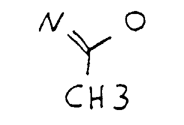 is reacted with a compound of the general formula V 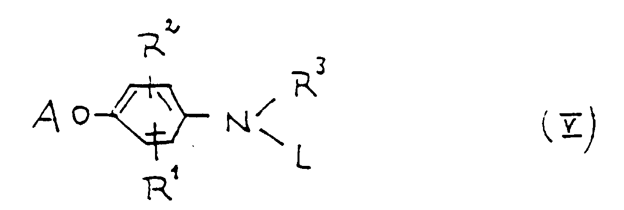 in which
R¹-R³ and L have the meaning given in claim 1 and A represents hydrogen, an optionally substituted ammonium ion or an alkali metal,
if B is a protected amino group, the amino-protecting group is removed or
if B is an azide residue, it is converted into an NH₂ group by reduction and the amino group is converted by acetylation into an NHCOCH₃ residue and subsequently the hydroxy-protecting groups are cleaved off.

7. Compound of the general formula V' 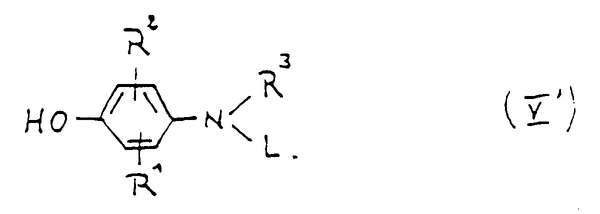 in which
R¹-R³ have the meaning given in claim 1 and
L represents a pyrazolo-heterocyclic residue of the general formula III 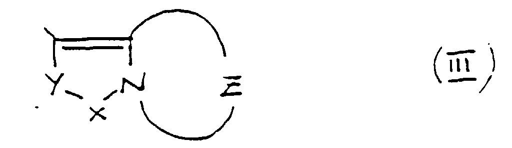 in which X-Y and Z have the meaning given in claim 1, provided that if
R³ represents an arylcarbonyl group or an alkylcarbonyl group optionally substituted once or several times by halogen, and X-Y is C=NR⁹,
Z does not form a 1,2,4-triazole ring in which a nitrogen that is not bound via a double bond is substituted with hydrogen.

8. Process for the production of a compound as claimed in claim 7, wherein
a) a substance of the general formula VII 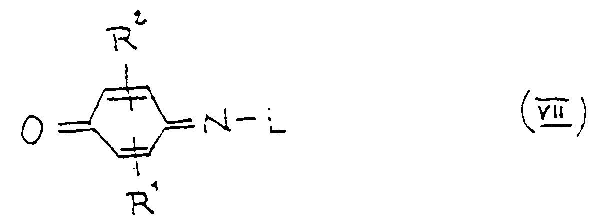 in which
R¹, R² and L have the meaning given in claim 7, is reduced and, if R³ is not hydrogen, the anilino group is acylated and subsequently an ester bond that may be present is cleaved or
b) a compound as claimed in claim 1 in which L represents a pyrazolo-heterocyclic residue of the general formula III 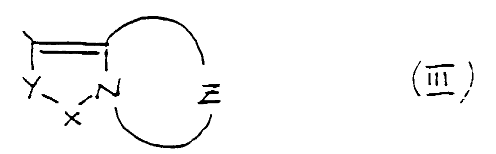 in which X-Y, G and Z have the meaning given in claim 1 is reacted with a suitable hydrolase.

9. Compound of the general formula VII 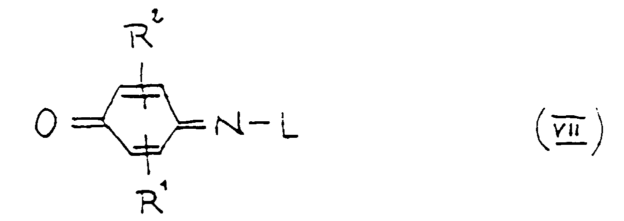 in which
R¹ and R² have the meaning given in claim 1 and
L represents a pyrazolo-heterocyclic residue of the general formula III 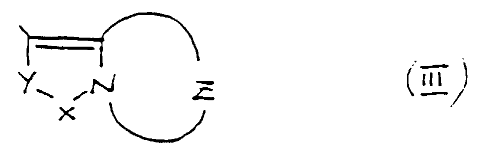 in which X-Y and Z have the meaning given in claim 1.

10. Process for the production of a compound as claimed in claim 9, wherein
a) a compound as claimed in claim 1 in which L represents a pyrazolo-heterocyclic residue of the general formula III 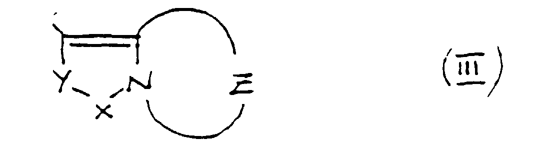 in which X-Y and Z have the meaning given in claim 1
is reacted with a hydrolase and the reaction product is oxidized or
b) a compound of the general formula VIII
NH₂-L (VIII)
in which
L represents a pyrazolo-heterocyclic residue of the general formula III 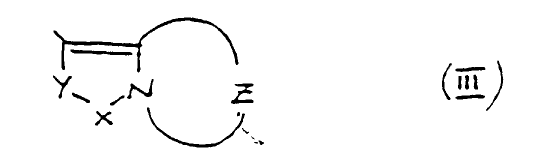 in which X-Y and Z have the meaning given in claim 1
is reacted in the presence of an oxidizing agent with a phenol of the general formula IX 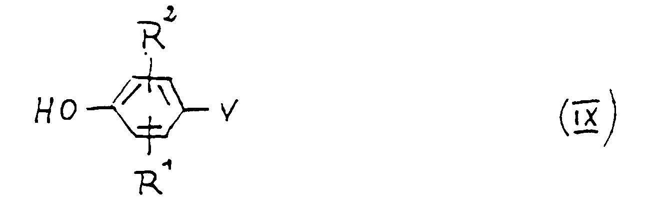 in which
R¹ and R² have the meaning given in claim 1 and
V represents hydrogen, halogen, CO₂H or SO₃H or
c) a compound of the general formula X
L-H (X)
in which L represents a pyrazolo-heterocyclic residue of the general formula III 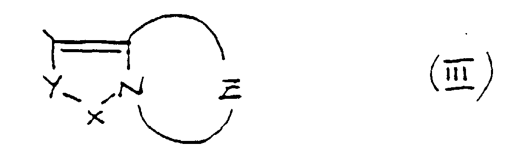 in which X-Y and Z have the meaning given in claim 1 is reacted with a compound of the general formula XI 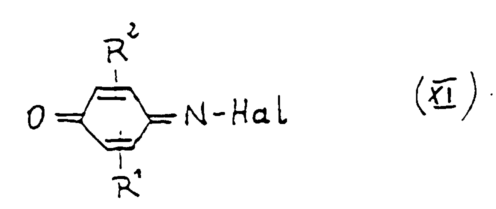 in which
R¹ and R² have the meaning given in claim 1 and
Hal represents a halogen residue or
d) a compound of the general formula XXV
L-E (XXV)
in which
E denotes a nitro or nitroso group and L represents a pyrazolo-heterocyclic residue of the general formula III 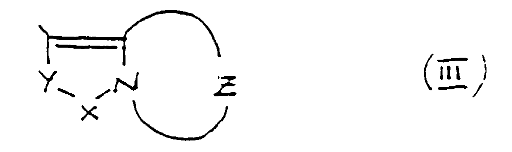 in which X-Y and Z have the meaning given in claim 1
is reacted with an organo-metallic compound of the general formula XXVI 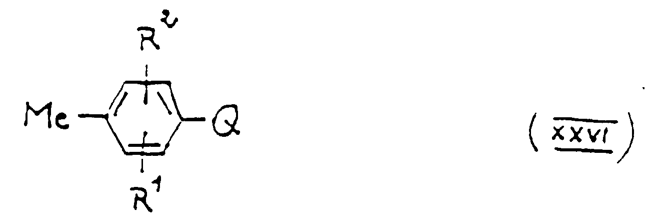 in which
R¹ and R² have the meaning given in claim 9
Q denotes a hydroxy or dialkylamino group and
Me denotes lithium or magnesium substituted by halogen
and is subsequently processed in an aqueous medium.

11. Use of a compound of the general formula I 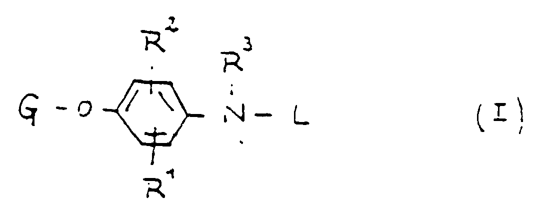 in which
G represents an organic or inorganic acid residue or a glycoside residue,
R¹ and R² which are the same or different, denote hydrogen,
halogen, SO₃H, PO₃H₂ or a salt of these acid groups, a hydroxy, nitro, carboxy, carboxamido or cyano group or an alkyl, alkenyl, alkoxy, alkylsulfinyl, alkylsulfonyl, alkoxycarbonyl, alkylcarbonyl, aryl or aralkyl group optionally substituted by one or several hydroxy, carboxy, halogen, cyano, SO₃H or PO₃H₂ residues or a salt of one of these acid residues or
if both residues are present on neighbouring carbon atoms, they together represent a 1,4 butadienediyl group which is optionally substituted once or several times by SO₃H, PO₃H₂ or a salt of these acid groups, an alkyl or/and a carboxyl group,
R³ is hydrogen, CO-COOH, SO₃H, PO₃H₂ or a salt of these acid groups, an alkylcarbonyl group optionally substituted once or several times by halogen, CO₂H, SO₃H or/and PO₃H₂ or a salt of these acid groups, or an arylcarbonyl group optionally substituted once or several times by SO₃H, PO₃H₂ or a salt of these acid residues and
L represents a pyrazolo-heterocyclic residue of the general formula III 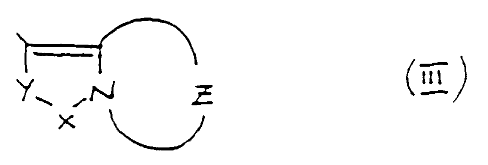 in which
X-Y denotes NR⁸-CO or N=CR⁹
in which
R⁸ is hydrogen or alkyl and
R⁹ is alkyl, alkenyl, alkoxy, alkylthio, aryl, aralkyl, each optionally substituted by hydroxy, dialkylphosphinyl, carboxy, SO₃H, PO₃H₂, a salt of one of these acid residues or/and alkoxycarbonyl;
amino which is optionally substituted by one or two alkyl residues which are optionally substituted by one or several hydroxy, carboxy or/and alkoxycarbonyl residues whereby if amino is substituted by two alkyl residues, these residues can also be joined to form a ring which, apart from the N atom of the amino group, can optionally also be interrupted by oxygen, sulphur or a further nitrogen atom, or amino is optionally substituted by one or two acyl groups, alkoxy or/and aralkoxycarbonyl groups, H₂N-CO, alkyl, aralkyl, or arylcarbamoyl groups; or
hydrogen, carboxy, alkoxycarbonyl, carboxamido or halogen and
Z denotes NR¹⁰-N=N
in which R¹⁰ is hydrogen, alkyl or aralkyl or
Z represents an unsaturated chain containing 3 to 5 members composed of nitrogen atoms or of carbon atoms and optionally one or several nitrogen or sulphur atoms, in which the carbon atoms are optionally substituted by alkyl, alkoxy, hydroxyalkyl, alkylthio, hydroxy, aralkyl, aryl, carboxy, carboxamido, alkoxycarbonyl, cyano, amino which is optionally substituted by one or two alkyl residues which are optionally substituted by one or several hydroxy, carboxy or/and alkoxycarbonyl residues, or/and halogen as well as nitrogen atoms which are not linked by a double bond and are optionally substituted by alkyl or aralkyl or two neighbouring chain substituents optionally form an alkylene group which in turn is optionally substituted or anellated with aryl or a corresponding tautomeric residue, as a hydrolase substrate.

12. Use as claimed in claim 11 of a compound of the general formula I as defined in claim 11 in which G represents an N-acetyl-β-D-glucosaminidyl residue as a substrate for the enzyme N-acetyl-β-D-glucosaminidase.

13. Use of compounds as claimed in claims 7 or 9 to produce compounds of the general formula I as defined in claims 1 and 11.

14. Method for the colorimetric determination of a hydrolase by reacting a chromogenic enzyme substrate with the enzyme, oxidizing a leuco dye released from the substrate by the enzyme and determining the resulting dye as a measure for the amount of enzyme, wherein a compound as claimed in claim 11 is used as the enzyme substrate.

15. Method as claimed in claim 14 for the determination of the enzyme N-acetyl-β-D-glucosaminidase, wherein a compound as claimed in claim 12 is used as the enzyme substrate.

16. Diagnostic agent for the determination of a hydrolase containing a chromogenic enzyme substrate and a suitable buffer substance, wherein a compound as claimed in claim 11 is used as the chromogenic enzyme substrate.

17. Diagnostic agent as claimed in claim 16, wherein it contains an oxidizing agent.

18. Diagnostic agent as claimed in one of the claims 16 or 17, wherein it additionally contains a 1-arylsemicarbazide of the general formula XII
Ar-NH-NH-CONH₂ (XII)
in which
Ar denotes an aryl residue optionally substituted by alkyl, alkoxy or halogen.

19. Diagnostic agent as claimed in one of the claims 16 - 18, wherein it contains the components in a carrier-bound form.

20. Use of a compound of the general formula I as defined in claim 1 for the production of a diagnostic agent for the determination of a hydrolase.

## Revendications

1. Dérivé aminophénol substitué sur l'atome de N et l'atome de O, de formule générale I 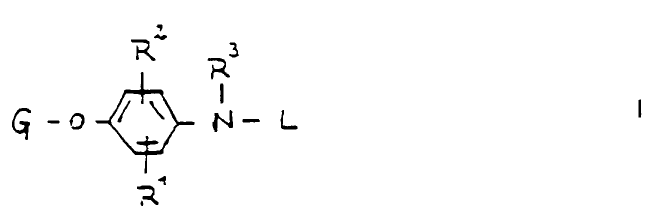 dans laquelle
G représente un résidu acide organique ou inorganique ou un résidu glycoside,
R¹ et R², qui sont identiques ou différents, représentent un atome d'hydrogène,
un atome d'halogène, un groupe SO₃H, un groupe PO₃H₂ ou un sel de ce groupe acide, un groupe hydroxy, un groupe nitro, un groupe carboxy, un groupe carboxamido ou un groupe cyano ou un groupe alkyle, un groupe alcényle, un groupe alcoxy, un groupe alkylsulfinyle, un groupe alkylsulfonyle, un groupe alcoxycarbonyle, un groupe alkylcarbonyle, un groupe aryle ou un groupe aralkyle, le cas échéant substitué par un ou plusieurs résidus hydroxy, un ou plusieurs résidus carboxy, un ou plusieurs résidus halogène, un ou plusieurs résidus cyano, un ou plusieurs résidus SO₃H ou un ou plusieurs résidus PO₃H₂ ou un sel d'un de ces résidus acides ou,
lorsque les deux résidus se trouvent sur des atomes de carbone voisins, représentent ensemble un groupe 1,4-butadiènediyle, qui est, le cas échéant, substitué une ou plusieurs fois par un groupe SO₃H, un groupe PO₃H₂ ou un sel de ces groupes acides, un groupe alkyle et/ou un groupe carboxy,
R³ représente un atome d'hydrogène, un groupe CO-COOH, un groupe SO₃H, un groupe PO₃H₂ ou un sel de ces groupes acides, un groupe alkylcarbonyle, le cas échéant substitué une ou plusieurs fois par un atome d'halogène, un groupe CO₂H, un groupe SO₃H et/ou un groupe PO₃H₂ ou un sel de ces groupes acides ou un groupe arylcarbonyle, le cas échéant substitué une ou plusieurs fois par un groupe SO₃H, un groupe PO₃H₂ ou un sel de ces résidus acides et
L représente un résidu d'un hétérocycle pyrazolo de formule générale III 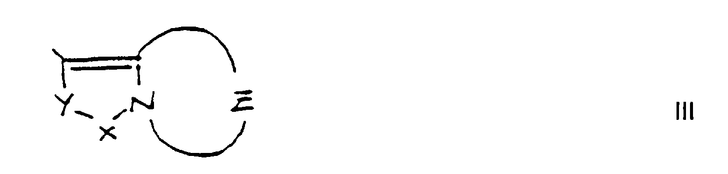 dans laquelle
X-Y signifie un groupe NR⁸-CO ou N=CR⁹, où
R⁸ représente un atome d'hydrogène ou un groupe alkyle et
R⁹ représente un groupe alkyle, un groupe alcényle, un groupe alcoxy, un groupe alkylthio, un groupe aryle, un groupe aralkyle, le cas échéant substitué respectivement par un groupe hydroxy, un groupe dialkylphosphinyle, un groupe carboxy, un groupe SO₃H, un groupe PO₃H₂, un sel d'un de ces résidus acides et/ou un groupe alcoxycarbonyle ;
un groupe amino, qui est le cas échéant substitué par un ou deux résidus alkyle, portant le cas échéant un ou plusieurs résidus hydroxy, un ou plusieurs résidus carboxy et/ou un ou plusieurs résidus alcoxycarbonyle, où, lorsque le groupe amino est substitué par deux résidus alkyle, ces résidus peuvent également être fermés en un cycle, qui peut être interrompu, outre par l'atome de N du groupe amino, le cas échéant également par un atome d'oxygène, un atome de soufre ou un autre atome d'azote, ou un groupe amino, le cas échéant substitué par un ou deux groupes acyle, un ou deux groupes alcoxycarbonyle et/ou un ou deux groupes aralcoxycarbonyle, un ou deux groupes H₂N-CO, un ou deux groupes alkylcarbamoyle, un ou deux groupes aralkylcarbamoyle ou un ou deux groupes arylcarbamoyle ; ou
un atome d'hydrogène, un groupe carboxy, un groupe alcoxycarbonyle, un groupe carboxamido ou un atome d'halogène et
Z signifie NR¹⁰-N=N, où R¹⁰ est de l'hydrogène, un groupe alkyle ou un groupe aralkyle ou
Z représente une chaîne insaturée comprenant 3 à 5 membres constitués par des atomes d'azote ou des atomes de carbone et, le cas échéant, un ou plusieurs atomes d'azote ou de soufre, les atomes de carbone étant le cas échéant substitués par un groupe alkyle, un groupe alcoxy, un groupe hydroxyalkyle, un groupe alkylthio, un groupe hydroxy, un groupe aralkyle, un groupe aryle, un groupe carboxy, un groupe carboxamido, un groupe alcoxycarbonyle, un groupe cyano, un groupe amino, qui est le cas échéant substitué par un ou deux résidus alkyle, le cas échéant substitué par un ou plusieurs groupes hydroxy, un ou plusieurs groupes carboxy et/ou un ou plusieurs groupes alcoxycarbonyle, et/ou un atome d'halogène ainsi que des atomes d'azote, qui ne sont pas liés via une double liaison, le cas échéant substitués par un groupe alkyle ou un groupe aralkyle ou deux substituants de chaîne voisins forment le cas échéant un groupe alkylène, qui est à son tour, le cas échéant, substitué ou condensé par un groupe aryle ou un résidu tautomère correspondant,
sous réserve que, lorsque
G est un résidu d'acide alcanecarboxylique,
R³ est un atome d'hydrogène, un groupe arylcarbonyle ou un groupe alkylcarbonyle, le cas échéant substitué une ou plusieurs fois par un atome d'halogène et
L est un résidu d'hétérocycle pyrazolo de formule générale III avec X-Y ayant la signification N=CR⁹, où R⁹ a la signification susmentionnée,
Z ne forme pas de cycle 1,2,4-triazole, dans lequel un atome d'azote qui n'est pas lié via une double liaison est substitué par un atome d'hydrogène.

2. Composé selon la revendication 1, caractérisé en ce que G est un résidu galactoside, un résidu glycoside, un résidu mannoside, un résidu N-acétyl-glucosaminide ou un résidu oligosaccharide comprenant 2 à 10 unités de monosaccharide.

3. Composé selon la revendication 1, caractérisé en ce que G représente un résidu N-acétyl-β-D-glucosaminidyle.

4. Composé selon la revendication 1, caractérisé en ce que G représente un groupe PO₃MM', un groupe SO₃M, un résidu d'acide alcanecarboxylique lié par le groupe carboxyle, un résidu d'aminoacide lié par le groupe carboxyle ou un résidu d'oligopeptide lié par le groupe carboxyle et M et M' représentent un atome d'hydrogène, un ion alcalin, un ion alcalino-terreux ou un ion ammonium.

5. Procédé pour la préparation d'un composé selon la revendication 1, caractérisé en ce qu'on fait réagir un composé de formule générale IV
G-D (IV)
dans laquelle
G est un résidu d'acide organique ou inorganique ou un résidu glycoside et
D est un groupe réactif,
les groupes fonctionnels dans G étant le cas échéant protégés par des groupes protecteurs, tels que ceux usuels dans la chimie des peptides et des hydrates de carbone,
avec un composé de formule générale V 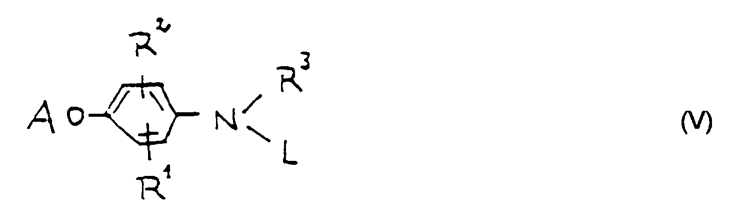 dans laquelle R¹-R³ et L ont la signification indiquée dans la revendication 1 et
A représente un atome d'hydrogène, un ion ammonium, le cas échéant substitué ou un métal alcalin,
et en ce que les groupes protecteurs sont, le cas échéant, finalement éliminés.

6. Procédé selon la revendication 5, caractérisé en ce qu'on fait réagir pour la préparation d'un composé selon la revendication 3, comme composé de formule générale IV, un composé de formule générale VI 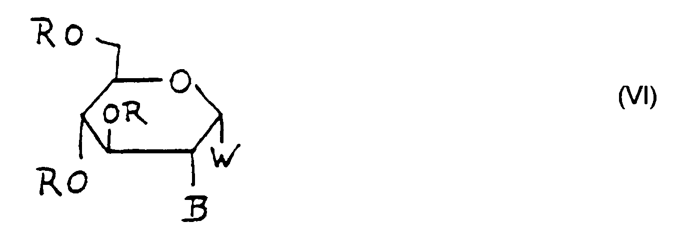 dans laquelle
W est un résidu halogène,
R est un groupe de protection du groupe hydroxy usuel dans la chimie des hydrates de carbone et
B est un résidu azide, un groupe amino protégé ou un groupe NH-COCH₃ ou
B et W représentent ensemble le groupe 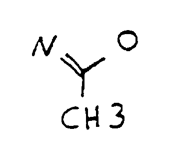 avec un composé de formule générale V 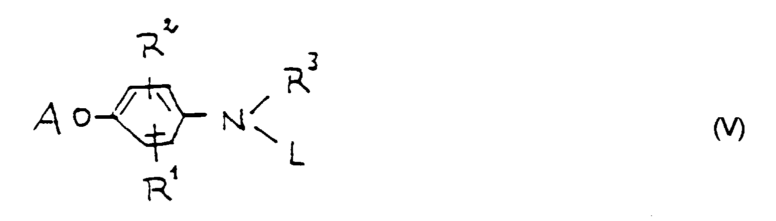 dans laquelle R¹-R³ et L ont la signification indiquée dans la revendication 1 et
A représente un atome d'hydrogène, un ion d'ammonium, le cas échéant substitué ou un métal alcalin,
lorsque B représente un groupe amino protégé, on élimine le groupe de protection du groupe amino ou,
lorsque B représente un résidu azide, on transforme celui-ci par réduction en un groupe NH₂ et on convertit le groupe amino par acétylation en résidu NHCOCH₃, et on élimine ensuite les groupes de protection des groupes hydroxy.

7. Composé de formule générale V' 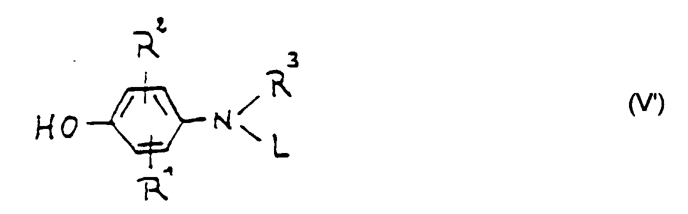 dans laquelle
R¹-R³ ont la signification indiquée dans la revendication 1 et
L représente un résidu d'hétérocycle pyrazolo de formule générale III 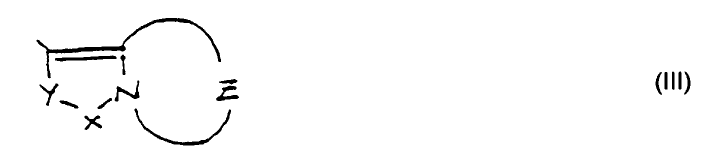 dans laquelle X-Y et Z ont la signification indiquée dans la revendication 1, sous réserve que,
lorsque R³ est un groupe arylcarbonyle un ou groupe alkylcarbonyle, le cas échéant substitué une ou plusieurs fois par un atome d'halogène, et
lorsque X-Y est égal à C=NR⁹,
Z ne forme pas de cycle 1,2,4-triazole dans lequel un atome d'azote qui n'est pas lié par une double liaison est substitué par un atome d'hydrogène.

8. Procédé pour la préparation d'un composé selon la revendication 7, caractérisé
a) en ce qu'on réduit une substance de formule générale VII 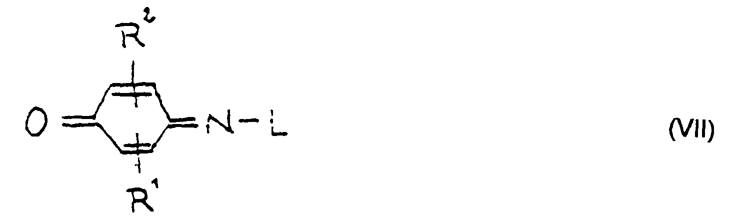 dans laquelle R¹, R² et L ont la signification indiquée dans la revendication 7, et, lorsque R³ n'est pas un atome d'hydrogène, le groupe anilino est acylé puis en ce qu'une liaison ester éventuellement présente est coupée ou
b) en ce qu'on fait réagir un composé selon la revendication 1, où L représente un résidu d'hétérocycle pyrazolo de formule générale III 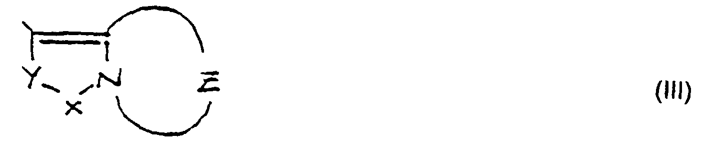 dans laquelle X-Y, G et Z ont la signification indiquée dans la revendication 1, avec une hydrolase correspondante.

9. Composé selon la formule générale VII 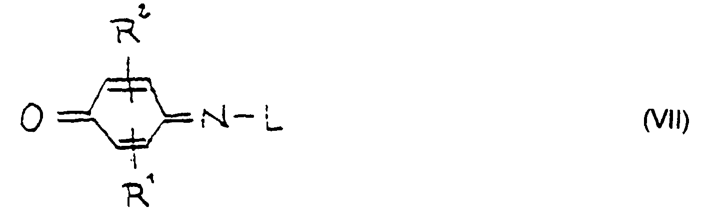 dans laquelle R¹ et R² ont la signification indiquée dans la revendication 1 et L représente un résidu d'hétérocycle pyrazolo de formule générale III 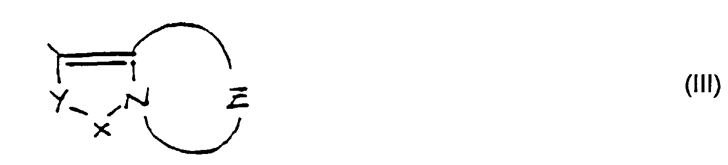 dans laquelle X-Y et Z ont la signification indiquée dans la revendication 1.

10. Procédé pour la préparation d'un composé selon la revendication 9, caractérisé
a) en ce qu'on fait réagir un composé selon la revendication 1, dans laquelle L représente un résidu d'hétérocycle pyrazolo de formule générale III 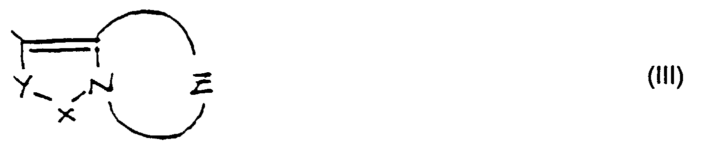 dans laquelle X-Y et Z ont la signification indiquée dans la revendication 1, avec une hydrolase et en ce que le produit de réaction est oxydé ou
b) en ce qu'on fait réagir un composé de formule générale VIII
NH₂-L (VIII)
dans laquelle L représente un résidu d'hétérocycle pyrazolo de formule générale III 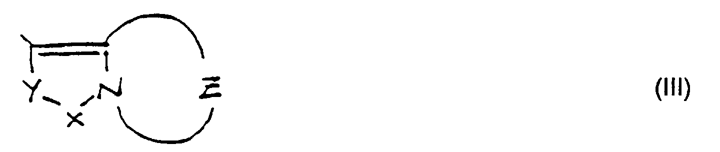 dans laquelle X-Y et Z ont la signification indiquée dans la revendication 1, avec un phénol de formule générale IX 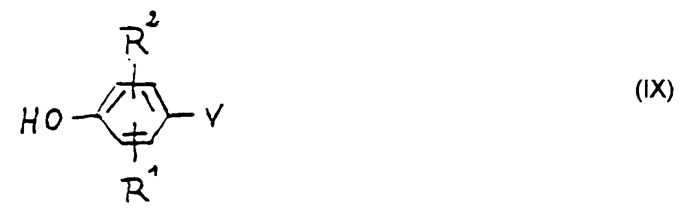 dans laquelle R¹ et R² ont la signification indiquée dans la revendication 1, V représente un atome d'hydrogène, un atome d'halogène, un groupe CO₂H ou un groupe SO₃H, en présence d'un agent d'oxydation ou
c) en ce qu'on fait réagir un composé de formule générale (X)
L-H (X)
dans laquelle L représente un résidu d'hétérocycle pyrazolo de formule générale III 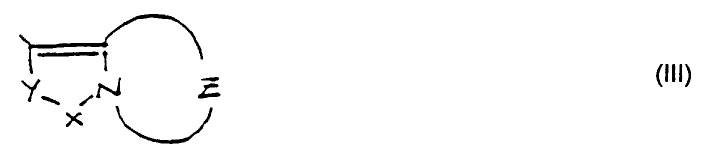 dans laquelle X-Y et Z ont la signification indiquée dans la revendication 1, avec un composé de formule générale XI 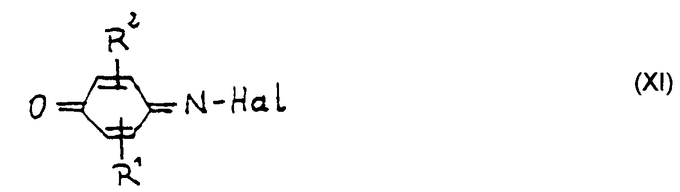 dans laquelle R¹ et R² ont la signification indiquée dans la revendication 1 et
Hal signifie un résidu halogène ou
d) en ce qu'on fait réagir un composé de formule générale XXV
L-E (XXV)
dans laquelle E est un groupe nitro ou nitroso et
L représente un résidu d'hétérocycle pyrazolo de formule générale III 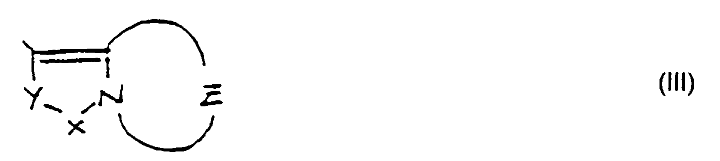 dans laquelle X-Y et Z ont la signification indiquée dans la revendication 1, avec un composé métallo-organique de formule générale XXVI 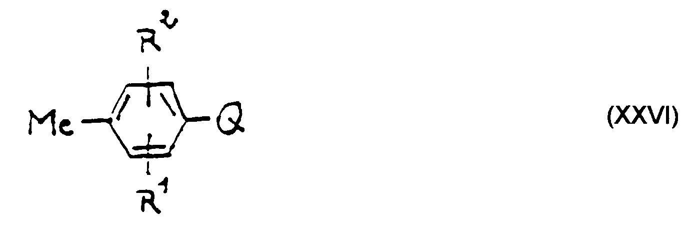 dans laquelle R¹ et R² ont la signification indiquée dans la revendication 9,
Q représente un groupe hydroxy ou un groupe dialkylamino et Me représente un atome de lithium ou un atome de magnésium substitué par un atome d'halogène
et en ce qu'on traite ensuite en phase aqueuse.

11. Utilisation d'un composé de formule générale I 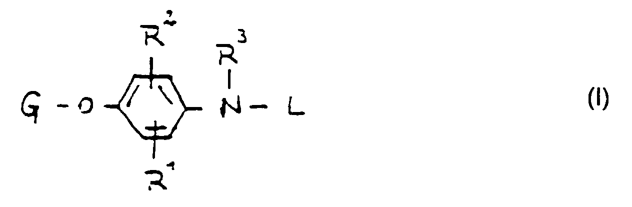 dans laquelle
G représente un résidu acide organique ou inorganique ou un résidu glycoside,
R¹ et R², qui sont identiques ou différents, représentent
un atome d'hydrogène,
un atome d'halogène, un groupe SO₃H, un groupe PO₃H₂ ou un sel de ce groupe acide, un groupe hydroxy, un groupe nitro, un groupe carboxy, un groupe carboxamido ou un groupe cyano ou un groupe alkyle, un groupe alcényle, un groupe alcoxy, un groupe alkylsulfinyle, un groupe alkylsulfonyle, un groupe alcoxycarbonyle, un groupe alkylcarbonyle, un groupe aryle ou un groupe aralkyle, le cas échéant substitué par un ou plusieurs résidus hydroxy, un ou plusieurs résidus carboxy, un ou plusieurs résidus halogène, un ou plusieurs résidus cyano, un ou plusieurs résidus SO₃H ou un ou plusieurs résidus PO₃H₂ ou un sel d'un de ces résidus d'acide ou,
lorsque les deux résidus se trouvent sur des atomes de carbone voisins, représentent ensemble un groupe 1,4-butadiènediyle, qui est, le cas échéant, substitué une ou plusieurs fois par un groupe SO₃H, un groupe PO₃H₂ ou un sel de ces groupes acides, un groupe alkyle et/ou un groupe carboxy,
R³ représente un atome d'hydrogène, un groupe CO-COOH, un groupe SO₃H, un groupe PO₃H₂ ou un sel de ces groupes acides, un groupe alkylcarbonyle, le cas échéant substitué une ou plusieurs fois par un atome d'halogène, une ou plusieurs fois par un groupe CO₂H, une ou plusieurs fois par un groupe SO₃H et/ou une ou plusieurs fois par un groupe PO₃H₂ ou un sel de ces groupes acides ou un groupe arylcarbonyle, le cas échéant substitué une ou plusieurs fois par un groupe SO₃H, une ou plusieurs fois par un groupe PO₃H₂ ou un sel de ces résidus acides et
L représente un résidu d'un hétérocycle pyrazolo de formule générale III 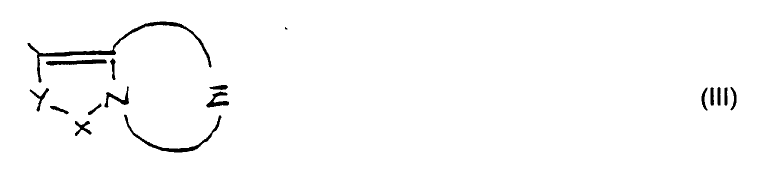 dans laquelle
X-Y signifie un groupe NR⁸-CO ou N=CR⁹, où
R⁸ représente un atome d'hydrogène ou un groupe alkyle et
R⁹ représente un groupe alkyle, un groupe alcényle, un groupe alcoxy, un groupe alkylthio, un groupe aryle, un groupe aralkyle, le cas échéant substitué respectivement par un groupe hydroxy, un groupe dialkylphosphinyle, un groupe carboxy, un groupe SO₃H, un groupe PO₃H₂, un sel d'un de ces résidus acides et/ou un groupe alcoxycarbonyle ;
un groupe amino, qui est le cas échéant substitué par un ou deux résidus alkyle, portant le cas échéant un ou plusieurs résidus hydroxy, un ou plusieurs résidus carboxy et/ou un ou plusieurs résidus alcoxycarbonyle, où, lorsque le groupe amino est substitué par deux résidus alkyle, ces résidus peuvent également être fermés en un cycle, qui peut être interrompu, outre par l'atome de N du groupe amino, le cas échéant également par un atome d'oxygène, un atome de soufre ou un autre atome d'azote, ou un groupe amino, le cas échéant substitué par un ou deux groupes acyle, un ou deux groupes alcoxycarbonyle et/ou un ou deux groupes aralcoxycarbonyle, un ou deux groupes H₂N-CO, un ou deux groupes alkylcarbamoyle, un ou deux groupes aralkylcarbamoyle ou un ou deux groupes arylcarbamoyle ; ou un atome d'hydrogène, un groupe carboxy, un groupe alcoxycarbonyle, un groupe carboxamido ou un atome d'halogène et
Z signifie NR¹⁰-N=N, où R¹⁰ est de l'hydrogène, un groupe alkyle ou un groupe aralkyle ou
Z représente une chaîne insaturée comprenant 3 à 5 membres constitués par des atomes d'azote ou des atomes de carbone et, le cas échéant, un ou plusieurs atomes d'azote ou de soufre, les atomes de carbone étant le cas échéant substitués par un groupe alkyle, un groupe alcoxy, un groupe hydroxyalkyle, un groupe alkylthio, un groupe hydroxy, un groupe aralkyle, un groupe aryle, un groupe carboxy, un groupe carboxamido, un groupe alcoxycarbonyle, un groupe cyano, un groupe amino, qui est le cas échéant substitué par un ou deux résidus alkyle, le cas échéant substitué par un ou plusieurs groupes hydroxy, un ou plusieurs groupes carboxy et/ou un ou plusieurs groupes alcoxycarbonyle, et/ou un atome d'halogène ainsi que des atomes d'azote, qui ne sont pas liés via une double liaison, le cas échéant substitués par un groupe alkyle ou un groupe aralkyle ou deux substituants de chaîne voisins forment le cas échéant un groupe alkylène, qui est à son tour, le cas échéant, substitué ou condensé par un groupe aryle ou un résidu tautomère correspondant comme substrat d'hydrolase.

12. Utilisation selon la revendication 11 d'un composé de formule générale I, tel que définie dans la revendication 11, G représentant un résidu N-acétyl-β-D-glucosaminidyle comme substrat pour l'enzyme N-acétyl-β-D-glucosaminidase.

13. Utilisation de composés selon les revendications 7 ou 9 pour la préparation de composés de formule générale I, tels qu'ils sont définis dans les revendications 1 et 11.

14. Procédé pour la détermination par colorimétrie d'une hydrolase par réaction d'un substrat d'enzyme chromogène avec l'enzyme, l'oxydation d'un leucodérivé libéré du substrat par l'enzyme et la détermination du colorant résultant comme mesure de la quantité d'enzyme, caractérisé en ce qu'on utilise comme substrat d'enzyme un composé selon la revendication 11.

15. Procédé selon la revendication 14 pour la détermination de l'enzyme N-acétyl-β-D-glucosaminidase, caractérisé en ce qu'on utilise comme substrat d'enzyme un composé selon la revendication 12.

16. Agent diagnostique pour la détermination d'une hydrolase contenant un substrat d'enzyme chromogène et une substance tampon appropriée, caractérisé en ce qu'on utilise comme substrat d'enzyme chromogène un composé selon la revendication 11.

17. Agent diagnostique selon la revendication 16, caractérisé en ce qu'il contient un agent d'oxydation.

18. Agent diagnostique selon l'une quelconque des revendications 16 ou 17, caractérisé en ce qu'il contient en outre un 1-arylsemi-carbazide de formule générale XII
Ar-NH-NH-CONH₂ (XII)
dans laquelle Ar signifie un résidu aryle, le cas échéant substitué par un groupe alkyle, un groupe alcoxy ou un atome d'halogène.

19. Agent diagnostique selon l'une quelconque des revendications 16 à 18, caractérisé en ce qu'il contient les composants sous une forme liée à un support.

20. Utilisation d'un composé de formule générale I, tel que défini dans la revendication 1, pour la préparation d'un agent diagnostique pour la détermination d'une hydrolase.
